# EUROPEAN PATENT APPLICATION

(11) **EP 4 834 906 A1**
(43) Date of publication of application: **04.11.2026**
(21) Application number: 24911621.1
(22) Date of filing: 30.12.2024
(51) Int. Cl.: C07D 487/04, A61K 31/519, A61P 1/04, A61P 1/16, A61P 3/00, A61P 9/00, A61P 9/10, A61P 11/00, A61P 17/00, A61P 21/00, A61P 25/00, A61P 35/00

(54) **PYRAZOLOPYRIMIDINE COMPOUND AND PHARMACEUTICAL USE THEREOF**

(30) Priority: 31.12.2023 CN 202311861173; 09.12.2024 CN 202411801131
(71) Applicant: Zhejiang Pantheon Innovation Pharmaceutical Co., Ltd., Jinhua, Zhejiang 321000 (CN)
(72) Inventor: ZHANG, Fengmin, Hangzhou, Zhejiang 310051 (CN); ZHANG, Sicheng, Hangzhou, Zhejiang 310051 (CN)
(74) Representative: karo IP
(86) International application number: PCT/CN2024/143829
(87) International publication number: WO 2025/140708

(57) **Abstract**

An NLRP3 inhibitor, a preparation method therefor and a pharmaceutical use thereof. Provided are an NLRP3 inhibitor of formula (I), a composition thereof and a use thereof. The compound can be used for treating or preventing diseases or conditions related to abnormal expression of an NLRP3 signaling pathway.

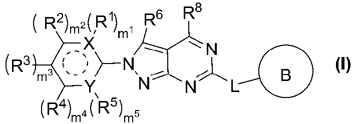

## Description

### TECHNICAL FIELD

The present disclosure belongs to the field of medicinal chemistry and relates to a pyrazolopyrimidine compound, specifically to an isomer of the compound or a pharmaceutically acceptable salt thereof. Further disclosed are a preparation method of the pyrazolopyrimidine compound and a pharmaceutical use thereof as an NLRP3 inhibitor for the treatment of diseases associated with abnormal expression of an NLRP3 signaling pathway.

### BACKGROUND

NLRP3 belongs to the NOD-like receptor protein family and is one of the most extensively studied intracellular pattern recognition receptors in recent years. Upon recognition of pathogen-associated molecular patterns (PAMPs) or host-derived danger-associated molecular patterns (DAMPs), NLRP3 recruits apoptosis-associated speck-like protein (ASC) and cysteine-aspartic protease-1 (caspase-1) to assemble into an inflammasome, while simultaneously releasing the activated inflammatory cytokines IL-1β and IL-18, thereby triggering an inflammatory response.

Inflammation is a defensive response of the body to various stimuli, including both infectious and sterile inflammation. Inflammation is primarily characterized by redness, swelling, heat, pain, and dysfunction, typically caused by increased permeability of vascular endothelial cells and extravasation of immune cells from the plasma. The inflammatory response resolves rapidly following tissue repair, but excessive cytokine production may lead to a cytokine storm, causing damage to the body. Inflammatory dysregulation is an important pathogenesis of a variety of human diseases.

Abnormal activation of the NLRP3 inflammasome has been observed in numerous inflammations and inflammatory diseases, and the excessive production of IL-1β and IL-18 has also been implicated in and contributes to the onset and progression of various diseases, including cryopyrin-associated periodic syndrome; sickle cell disease; autoimmune diseases such as systemic lupus erythematosus and psoriasis; liver diseases such as chronic liver disease, viral hepatitis, non-alcoholic steatohepatitis, alcoholic steatohepatitis, and alcoholic liver disease; inflammatory arthritis-related diseases such as gout, chondrocalcinosis, osteoarthritis, and rheumatoid arthritis; kidney diseases such as hyperoxaluria, lupus nephritis, hypertensive nephropathy, hemodialysis-associated inflammation, and diabetic nephropathy; neuroinflammatory diseases such as brain infection, acute injury, and neurodegenerative disorders including multiple sclerosis, Alzheimer's disease, and Parkinson's disease; cardiovascular and metabolic diseases such as atherosclerosis, type I and type II diabetes and related complications (*e.g*., nephropathy and retinopathy), peripheral arterial disease, acute heart failure, and hypertension; wound healing and scar formation; inflammatory skin diseases such as acne and hidradenitis suppurativa; asthma; sarcoidosis; age-related macular degeneration; and cancer-related diseases such as myeloproliferative neoplasm, leukemia, myelodysplastic syndrome, myelofibrosis, lung cancer, and colon cancer.

To date, no NLRP3 inhibitor has been approved for marketing. MCC950, the earliest compound to advance into clinical development, had its trials terminated due to hepatotoxicity. Nevertheless, MCC950 has been widely used as a tool molecule in a large number of literatures for investigation of various indications or mechanisms. Other compounds, including OLT-1177, Emlenoflast, RG6418, and DFV890, are currently undergoing clinical development for the treatment of diseases such as acute gout flare, knee osteoarthritis, Schnitzler syndrome, cryopyrin-associated periodic syndrome, ulcerative colitis, and Parkinson's disease. Diseases that are essentially inflammatory or immune-mediated are often difficult to diagnose or treat efficiently. Most therapeutic approaches involve symptom management, slowing disease progression, lifestyle modification, and surgery as a last resort. There is an urgent need for more efficient and effective therapeutic strategies for the treatment of such diseases.

Accordingly, the development of NLRP3 inflammasome inhibitors has significant therapeutic potential for the treatment of diseases associated with pathological features of inflammation.

### SUMMARY

The technical problem addressed by the present disclosure is to provide a small molecule inhibitor targeting NLRP3 with a novel structure, which exhibits significant NLRP3 inhibitory activity and is useful for the prevention or treatment of diseases associated with abnormal NLRP3 expression.

To address the above technical problem, the present disclosure provides the following technical solutions:

In one aspect, the present disclosure provides a pyrazolopyrimidine compound, which is a compound represented by the following general formula **(I),** an isomer thereof, or a pharmaceutically acceptable salt thereof: 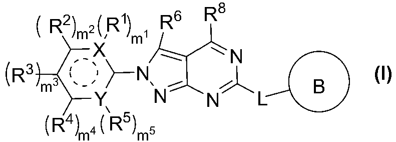
wherein -̅ -̅ -̅ is selected from the group consisting of a single bond and a double bond;
m¹, m², m³, m⁴, and m⁵ are each independently selected from the group consisting of 1 and 2;
X and Y are each independently selected from the group consisting of C and N;
R¹ and R⁵ are each independently selected from the group consisting of absent, hydrogen, halogen, hydroxyl, cyano, amino, C₁₋₆ alkyl, C₁₋₆ alkylamino, C₁₋₆ alkoxy, C₂₋₆ alkenyl, C₂₋₆ alkynyl, C₃₋₈ cycloalkyl, 3- to 6-membered heterocyclyl, and 5- to 10-membered heteroaryl; the C₁₋₆ alkyl, C₁₋₆ alkylamino, C₁₋₆ alkoxy, C₂₋₆ alkenyl, C₂₋₆ alkynyl, C₃₋₈ cycloalkyl, 3- to 6-membered heterocyclyl, or 5- to 10-membered heteroaryl may be optionally further substituted by one or more substituents selected from the group consisting of C₁₋₆ alkyl and halogen;
R², R³, and R⁴ are each independently selected from the group consisting of hydrogen, halogen, hydroxyl, cyano, amino, C₁₋₆ alkyl, C₁₋₆ alkylamino, C₁₋₆ alkoxy, C₂₋₆ alkenyl, C₂₋₆ alkynyl, C₃₋₈ cycloalkyl, 3- to 6-membered heterocyclyl, and 5- to 10-membered heteroaryl; the C₁₋₆ alkyl, C₁₋₆ alkylamino, C₁₋₆ alkoxy, C₂₋₆ alkenyl, C₂₋₆ alkynyl, C₃₋₈ cycloalkyl, 3- to 6-membered heterocyclyl, or 5- to 10-membered heteroaryl may be optionally further substituted by one or more substituents selected from the group consisting of C₁₋₆ alkyl and halogen;
alternatively, R³ and R⁴, together with the carbon atom to which they are attached, form a C₃₋₈ cycloalkyl group, a 3- to 6-membered heterocyclyl group, or a 5- to 10-membered heteroaryl group;
alternatively, R³ and R², together with the carbon atom to which they are attached, form a C₃₋₈ cycloalkyl group, a 3- to 6-membered heterocyclyl group, or a 5- to 10-membered heteroaryl group;
at least one of R¹, R², R³, R⁴, or R⁵ is hydroxyl;
R⁶ is selected from the group consisting of hydrogen, halogen, and C₁₋₆ alkyl; the C₁₋₆ alkyl may be optionally further substituted by one or more substituents selected from the group consisting of C₁₋₆ alkyl and halogen;
L is a chemical bond, NR⁷, OR⁷, or C(R⁷ )₂; R⁷ is selected from the group consisting of hydrogen, C₁₋₆ alkyl, and absent;
R⁸ is selected from the group consisting of hydrogen, hydroxyl, cyano, amino, C₁₋₆ alkyl, C₁₋₆ alkoxy, C₂₋₆ alkynyl, C₃₋₈ cycloalkyl, and 5- to 10-membered heteroaryl; the C₁₋₆ alkyl, C₁₋₆ alkoxy, C₂₋₆ alkynyl, C₃₋₈ cycloalkyl, or 5- to 10-membered heteroaryl may be optionally further substituted by one or more substituents selected from the group consisting of C₁₋₆ alkyl and halogen;
ring B is selected from the group consisting of C₃₋₈ cycloalkyl, 3- to 9-membered heterocyclyl, C₆₋₁₀ aryl, and 5- to 10-membered heteroaryl; the C₃₋₈ cycloalkyl, 3- to 9-membered heterocyclyl, C₆₋₁₀ aryl, or 5- to 10-membered heteroaryl may be optionally further substituted by one or more substituents selected from the group consisting of hydroxyl, halogen, C₁₋₆ alkyl, C₁₋₆ haloalkyl, C₁₋₆ hydroxyalkyl, -COOR⁹, -CH₂COOR⁹, and C₁₋₆ alkylaminoalkyl;
R⁹ is selected from the group consisting of hydrogen and C₁₋₆ alkyl;
wherein the 3- to 6-membered heterocyclyl, 3- to 9-membered heterocyclyl, or 5- to 10-membered heteroaryl contains at least one heteroatom selected from the group consisting of N, O, and S.

In some embodiments, the compound is a compound represented by the following formula (IA), an isomer thereof, or a pharmaceutically acceptable salt thereof: 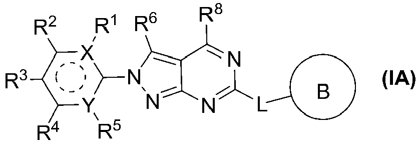
wherein X and Y are each independently selected from the group consisting of C and N;
R¹, R², R³, R⁴, and R⁵ are each independently selected from the group consisting of hydrogen, halogen, substituted or unsubstituted C₁₋₆ alkyl, substituted or unsubstituted C₁₋₆ haloalkyl, substituted or unsubstituted C₁₋₆ alkylamino, substituted or unsubstituted C₁₋₆ alkoxy, substituted or unsubstituted C₁₋₆ haloalkoxy, substituted or unsubstituted C₂₋₆ alkenyl, substituted or unsubstituted C₂₋₆ haloalkenyl, substituted or unsubstituted C₂₋₆ alkynyl, substituted or unsubstituted C₃₋₈ cycloalkyl, substituted or unsubstituted 3- to 6-membered heterocyclyl, substituted or unsubstituted 5- to 10-membered heteroaryl, hydroxyl, cyano, and amino, and at least one of R¹, R², R³, R⁴, or R⁵ is hydroxyl; when the C₁₋₆ alkyl, C₁₋₆ haloalkyl, C₁₋₆ alkylamino, C₁₋₆ alkoxy, C₁₋₆ haloalkoxy, C₂₋₆ alkenyl, C₂₋₆ haloalkenyl, C₂₋₆ alkynyl, C₃₋₈ cycloalkyl, 3- to 6-membered heterocyclyl, or 5- to 10-membered heteroaryl contains at least one substituent, the substituent is selected from the group consisting of C₁₋₆ alkyl and halogen, or any combination thereof; or
R³ and an adjacent R⁴, together with the carbon atom to which they are attached, form a C₃₋₈ cycloalkyl group, a 3- to 6-membered heterocyclyl group, or a 5- to 10-membered heteroaryl group; R³ and an adjacent R², together with the carbon atom to which they are attached, form a C₃₋₈ cycloalkyl group, a 3- to 6-membered heterocyclyl group, or a 5- to 10-membered heteroaryl group;
R⁶ is selected from the group consisting of hydrogen, substituted or unsubstituted C₁₋₆ alkyl, and halogen; when the C₁₋₆ alkyl contains at least one substituent, the substituent is selected from the group consisting of C₁₋₆ alkyl and halogen, or any combination thereof;
L is a direct bond, NR⁷, OR⁷, or CR⁷; R⁷ is selected from the group consisting of H, C₁₋₆ alkyl, and absent;
R⁸ is selected from the group consisting of hydrogen, substituted or unsubstituted C₁₋₆ alkyl, substituted or unsubstituted C₁₋₆ haloalkyl, substituted or unsubstituted C₁₋₆ alkoxy, substituted or unsubstituted C₁₋₆ haloalkoxy, substituted or unsubstituted C₂₋₆ alkynyl, substituted or unsubstituted C₃₋₈ cycloalkyl, substituted or unsubstituted 5- to 10-membered heteroaryl, hydroxyl, cyano, and amino; when the C₁₋₆ alkyl, C₁₋₆ haloalkyl, C₁₋₆ alkoxy, C₁₋₆ haloalkoxy, C₂₋₆ alkynyl, C₃₋₈ cycloalkyl, or 5- to 10-membered heteroaryl contains at least one substituent, the substituent is selected from the group consisting of C₁₋₆ alkyl and halogen, or any combination thereof;
ring B is selected from the group consisting of substituted or unsubstituted C₃₋₈ cycloalkyl, substituted or unsubstituted 3- to 9-membered heterocyclyl, substituted or unsubstituted C₆₋₁₀ aryl, and substituted or unsubstituted 5- to 10-membered heteroaryl; when the C₃₋₈ cycloalkyl, 3- to 9-membered heterocyclyl, C₆₋₁₀ aryl, or 5- to 10-membered heteroaryl contains at least one substituent, the substituent is selected from the group consisting of C₁₋₆ alkyl, hydroxyl, halogen, C₁₋₆ haloalkyl, C₁₋₆ hydroxyalkyl, -COOR⁹, and C₁₋₆ alkylaminoalkyl, or any combination thereof;
R⁹ is selected from the group consisting of hydrogen and C₁₋₆ alkyl;
the heterocyclyl or heteroaryl contains at least one heteroatom selected from the group consisting of N, O, and S.

In some embodiments, the compound is a compound represented by the following formula **(IB),** an isomer thereof, or a pharmaceutically acceptable salt thereof: 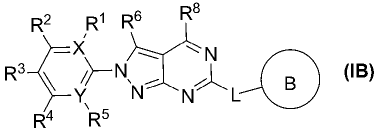
wherein ring B is selected from the group consisting of C₃₋₈ cycloalkyl, 3- to 9-membered heterocyclyl, C₆₋₁₀ aryl, and 5- to 10-membered heteroaryl; the C₃₋₈ cycloalkyl, 3-to 9-membered heterocyclyl, C₆₋₁₀ aryl, or 5- to 10-membered heteroaryl may be optionally further substituted by one or more substituents selected from the group consisting of hydroxyl, halogen, C₁₋₆ alkyl, C₁₋₆ haloalkyl, C₁₋₆ hydroxyalkyl, CH₂COOR⁹, and C₁₋₆ alkylaminoalkyl;
X, Y, L, R¹, R², R³, R⁴, R⁵, R⁶, R⁸, and R⁹ are as defined in the general formula (**I**).

In some embodiments, the compound is a compound represented by the following formula (**I-1**), an isomer thereof, or a pharmaceutically acceptable salt thereof: 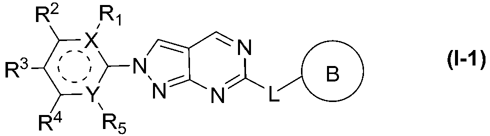
wherein X and Y are each independently selected from the group consisting of C and N;
R¹, R², R³, R⁴, and R⁵ are each independently selected from the group consisting of hydrogen, halogen, substituted or unsubstituted C₁₋₆ alkyl, substituted or unsubstituted C₁₋₆ haloalkyl, substituted or unsubstituted C₁₋₆ alkylamino, substituted or unsubstituted C₁₋₆ alkoxy, substituted or unsubstituted C₁₋₆ haloalkoxy, substituted or unsubstituted C₂₋₆ alkynyl, substituted or unsubstituted C₃₋₈ cycloalkyl, substituted or unsubstituted 3- to 6-membered heterocyclyl, substituted or unsubstituted 5- to 10-membered heteroaryl, hydroxyl, and cyano, and at least one of R¹, R², R³, R⁴, or R⁵ is hydroxyl; when the C₁₋₆ alkyl, C₁₋₆ alkylamino, C₁₋₆ haloalkyl, C₁₋₆ alkoxy, C₁₋₆ haloalkoxy, C₂₋₆ alkynyl, C₃₋₈ cycloalkyl, 3- to 6-membered heterocyclyl, or 5- to 10-membered heteroaryl contains at least one substituent, the substituent is selected from the group consisting of C₁₋₆ alkyl and halogen, or any combination thereof; or
R³ and an adjacent R⁴, together with the carbon atom to which they are attached, form a C₃₋₈ cycloalkyl group, a 3- to 6-membered heterocyclyl group, or a 5- to 10-membered heteroaryl group; R³ and an adjacent R², together with the carbon atom to which they are attached, form a C₃₋₈ cycloalkyl group, a 3- to 6-membered heterocyclyl group, or a 5- to 10-membered heteroaryl group;
R⁶ is selected from the group consisting of hydrogen, substituted or unsubstituted C₁₋₆ alkyl, and halogen; when the C₁₋₆ alkyl contains at least one substituent, the substituent is selected from the group consisting of C₁₋₆ alkyl and halogen, or any combination thereof;
L is selected from the group consisting of NR⁷, OR⁷, and CR⁷; R⁷ is selected from the group consisting of H, methyl, and absent;
ring B is selected from the group consisting of substituted or unsubstituted C₃₋₈ cycloalkyl, substituted or unsubstituted 3- to 9-membered heterocyclyl, and substituted or unsubstituted 5- to 10-membered heteroaryl; when the C₃₋₈ cycloalkyl, 3- to 9-membered heterocyclyl, or 5- to 10-membered heteroaryl contains at least one substituent, the substituent is selected from the group consisting of C₁₋₆ alkyl, hydroxyl, halogen, C₁₋₆ haloalkyl, C₁₋₆ hydroxyalkyl, -CH₂COOEt, and C₁₋₆ alkylaminoalkyl, or any combination thereof;
the heterocyclyl or heteroaryl contains at least one heteroatom selected from the group consisting of N, O, and S.

In some embodiments, the compound is a compound represented by the following formula **(II),** an isomer thereof, or a pharmaceutically acceptable salt thereof: 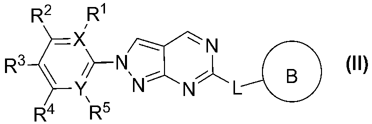
wherein X and Y are each independently selected from the group consisting of C and N;
R¹ and R⁵ are each independently selected from the group consisting of absent, hydrogen, halogen, hydroxyl, cyano, C₁₋₆ alkyl, C₁₋₆ alkylamino, C₁₋₆ alkoxy, C₂₋₆ alkynyl, C₃₋₈ cycloalkyl, 3- to 6-membered heterocyclyl, and 5- to 10-membered heteroaryl; the C₁₋₆ alkyl, C₁₋₆ alkylamino, C₁₋₆ alkoxy, C₂₋₆ alkynyl, C₃₋₈ cycloalkyl, 3- to 6-membered heterocyclyl, or 5- to 10-membered heteroaryl may be optionally further substituted by one or more substituents selected from the group consisting of C₁₋₆ alkyl and halogen;
R², R³, and R⁴ are each independently selected from the group consisting of hydrogen, halogen, hydroxyl, cyano, C₁₋₆ alkyl, C₁₋₆ alkylamino, C₁₋₆ alkoxy, C₂₋₆ alkynyl, C₃₋₈ cycloalkyl, 3- to 6-membered heterocyclyl, and 5- to 10-membered heteroaryl; the C₁₋₆ alkyl, C₁₋₆ alkylamino, C₁₋₆ alkoxy, C₂₋₆ alkynyl, C₃₋₈ cycloalkyl, 3- to 6-membered heterocyclyl, or 5- to 10-membered heteroaryl may be optionally further substituted by one or more substituents selected from the group consisting of C₁₋₆ alkyl and halogen;
alternatively, R³ and R⁴, together with the carbon atom to which they are attached, form a C₃₋₈ cycloalkyl group, a 3- to 6-membered heterocyclyl group, or a 5- to 10-membered heteroaryl group;
alternatively, R³ and R², together with the carbon atom to which they are attached, form a C₃₋₈ cycloalkyl group, a 3- to 6-membered heterocyclyl group, or a 5- to 10-membered heteroaryl group;
at least one of R¹, R², R³, R⁴, or R⁵ is hydroxyl;
L is selected from the group consisting of NR⁷, OR⁷, and C(R⁷)₂; R⁷ is selected from the group consisting of hydrogen, methyl, and absent;
ring B is selected from the group consisting of C₃₋₈ cycloalkyl, 3- to 9-membered heterocyclyl, C₆₋₁₀ aryl, and 5- to 10-membered heteroaryl; the C₃₋₈ cycloalkyl, 3- to 9-membered heterocyclyl, C₆₋₁₀ aryl, or 5- to 10-membered heteroaryl may be optionally further substituted by one or more substituents selected from the group consisting of hydroxyl, halogen, C₁₋₆ alkyl, C₁₋₆ haloalkyl, C₁₋₆ hydroxyalkyl, -CH₂COOEt, and C₁₋₆ alkylaminoalkyl;
wherein the 3- to 6-membered heterocyclyl, 3- to 9-membered heterocyclyl, or 5- to 10-membered heteroaryl contains at least one heteroatom selected from the group consisting of N, O, and S.

In some embodiments, the compound is a compound represented by the following formula **(IIA),** an isomer thereof, or a pharmaceutically acceptable salt thereof: 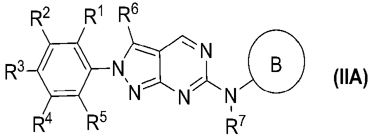
wherein R¹, R², R³, R⁴, and R⁵ are each independently selected from the group consisting of hydrogen, halogen, substituted or unsubstituted C₁₋₆ alkyl, substituted or unsubstituted C₁₋₆ haloalkyl, substituted or unsubstituted C₁₋₆ alkylamino, substituted or unsubstituted C₁₋₆ alkoxy, substituted or unsubstituted C₁₋₆ haloalkoxy, substituted or unsubstituted C₂₋₆ alkynyl, substituted or unsubstituted C₃₋₈ cycloalkyl, substituted or unsubstituted 3- to 6-membered heterocyclyl, substituted or unsubstituted 5- to 10-membered heteroaryl, hydroxyl, and cyano, and at least one of R¹, R², R³, R⁴, or R⁵ is hydroxyl; when the C₁₋₆ alkyl, C₁₋₆ haloalkyl, C₁₋₆ alkoxy, C₁₋₆ haloalkoxy, C₂₋₆ alkynyl, C₃₋₈ cycloalkyl, 3- to 6-membered heterocyclyl, or 5- to 10-membered heteroaryl contains at least one substituent, the substituent is selected from the group consisting of C₁₋₆ alkyl and halogen, or any combination thereof; or
R³ and an adjacent R⁴, together with the carbon atom to which they are attached, form a C₃₋₈ cycloalkyl group, a 3- to 6-membered heterocyclyl group, or a 5- to 10-membered heteroaryl group; R³ and an adjacent R², together with the carbon atom to which they are attached, form a C₃₋₈ cycloalkyl group, a 3- to 6-membered heterocyclyl group, or a 5- to 10-membered heteroaryl group;
R⁶ is selected from the group consisting of hydrogen, C₁₋₆ alkyl, and halogen;
R⁷ is selected from the group consisting of hydrogen and C₁₋₆ alkyl;
ring B is selected from the group consisting of substituted or unsubstituted C₃₋₈ cycloalkyl, substituted or unsubstituted 3- to 8-membered heterocyclyl, and substituted or unsubstituted 5- to 10-membered heteroaryl; when the C₃₋₈ cycloalkyl, 3- to 8-membered heterocyclyl, or 5- to 10-membered heteroaryl contains at least one substituent, the substituent is selected from the group consisting of C₁₋₆ alkyl, hydroxyl, halogen, C₁₋₆ haloalkyl, C₁₋₆ hydroxyalkyl, -CH₂COOEt, and C₁₋₆ alkylaminoalkyl, or any combination thereof;
the heterocyclyl or heteroaryl contains at least one heteroatom selected from the group consisting of N, O, and S.

In some embodiments, the compound is a compound represented by the following formula (**II-1**), an isomer thereof, or a pharmaceutically acceptable salt thereof: 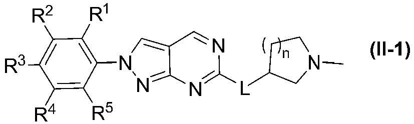
wherein n is selected from the group consisting of 0, 1, 2, and 3;
R¹, R², R³, R⁴, and R⁵ are each independently selected from the group consisting of hydrogen, halogen, hydroxyl, cyano, C₁₋₆ alkyl, C₁₋₆ alkylamino, C₁₋₆ alkoxy, C₂₋₆ alkynyl, C₃₋₈ cycloalkyl, 3- to 6-membered heterocyclyl, and 5- to 10-membered heteroaryl; the C₁₋₆ alkyl, C₁₋₆ alkylamino, C₁₋₆ alkoxy, C₂₋₆ alkynyl, C₃₋₈ cycloalkyl, 3- to 6-membered heterocyclyl, or 5- to 10-membered heteroaryl may be optionally further substituted by one or more substituents selected from the group consisting of C₁₋₆ alkyl and halogen;
alternatively, R³ and R⁴, together with the carbon atom to which they are attached, form a C₃₋₈ cycloalkyl group, a 3- to 6-membered heterocyclyl group, or a 5- to 10-membered heteroaryl group;
alternatively, R³ and R², together with the carbon atom to which they are attached, form a C₃₋₈ cycloalkyl group, a 3- to 6-membered heterocyclyl group, or a 5- to 10-membered heteroaryl group;
at least one of R¹, R², R³, R⁴, or R⁵ is hydroxyl;
L is as defined in the general formula (**II**).

In some embodiments, the compound is a compound represented by the following formula **(IIA-1),** an isomer thereof, or a pharmaceutically acceptable salt thereof: 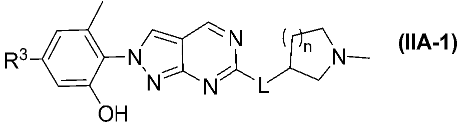
wherein n is selected from the group consisting of 1 and 2;
L is selected from the group consisting of O, NH, and N(CH₃);
R³ is selected from the group consisting of hydrogen, halogen, hydroxyl, cyano, C₁₋₆ alkyl, C₁₋₆ alkylamino, C₁₋₆ alkoxy, C₂₋₆ alkynyl, C₃₋₈ cycloalkyl, 3- to 6-membered heterocyclyl, and 5- to 10-membered heteroaryl; the C₁₋₆ alkyl, C₁₋₆ alkylamino, C₁₋₆ alkoxy, C₂₋₆ alkynyl, C₃₋₈ cycloalkyl, 3- to 6-membered heterocyclyl, or 5- to 10-membered heteroaryl may be optionally further substituted by one or more substituents selected from the group consisting of halogen and C₁₋₆ alkyl;

In some embodiments, the compound is a compound represented by the following formula **(IIA-1-1),** an isomer thereof, or a pharmaceutically acceptable salt thereof: 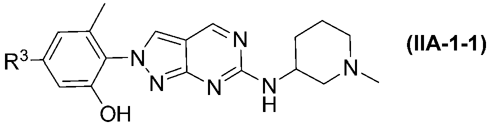 wherein R³ is as defined in the general formula (IIA-1).

In some embodiments, R³ is selected from the group consisting of hydrogen, C₁₋₆ haloalkyl, and halogen.

In some embodiments, the compound is a compound represented by the following formula **(IIA-1-2),** an isomer thereof, or a pharmaceutically acceptable salt thereof: 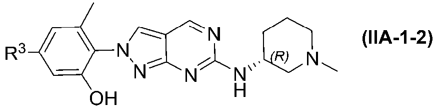 wherein R³ is as defined in the general formula **(IIA-1).**

In some embodiments, R³ is selected from the group consisting of hydrogen, halogen, cyano, methyl, cyclopropyl, trifluoromethyl, difluoromethyl, trifluoromethoxy, dimethylamino,

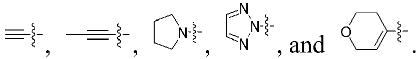

The present disclosure further provides a pyrazolopyrimidine compound, which is a compound having the following structure, an isomer thereof, or a pharmaceutically acceptable salt thereof:

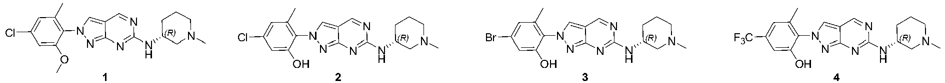

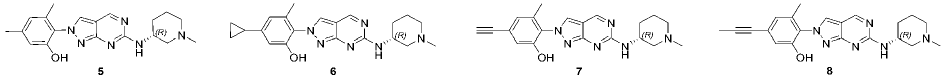

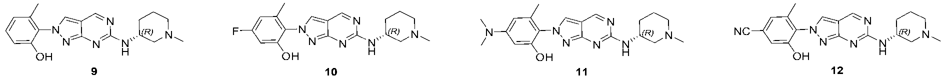

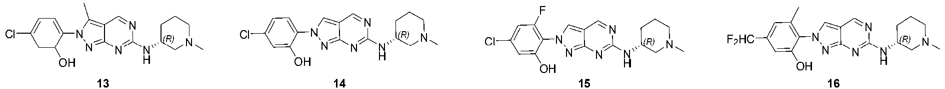

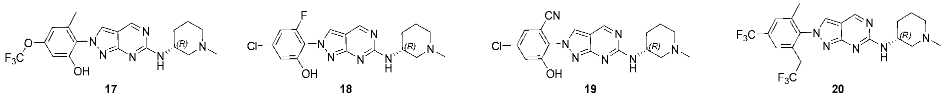

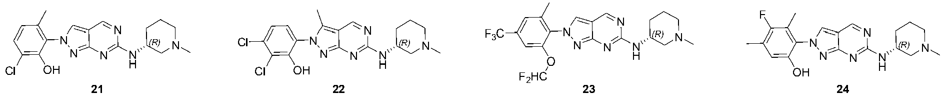

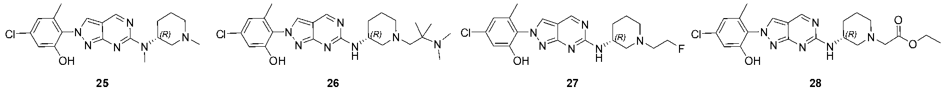

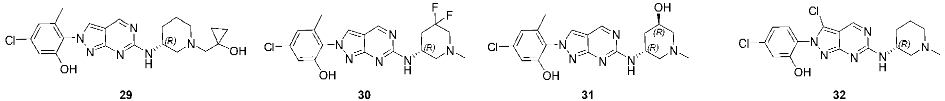

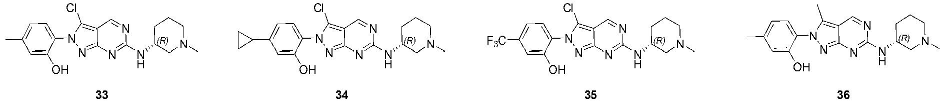

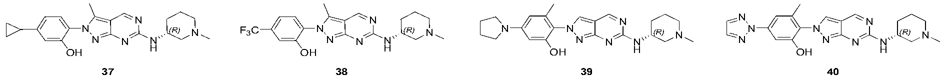

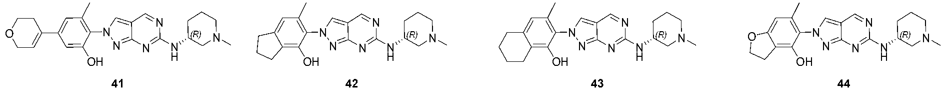

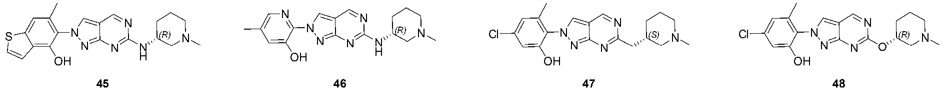

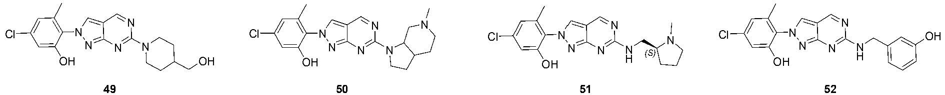

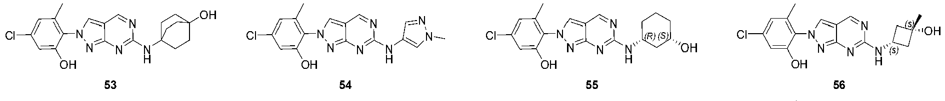

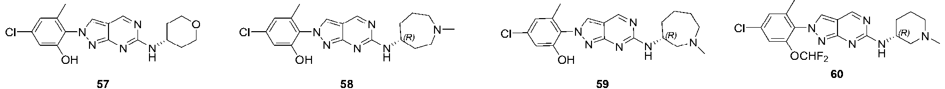

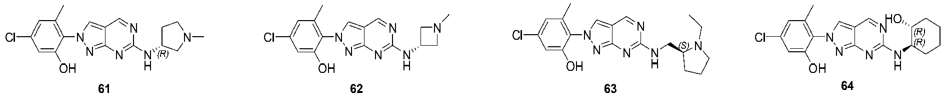

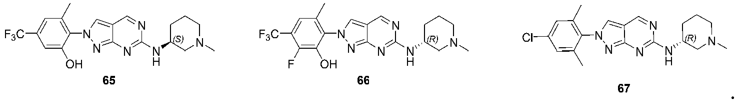

In another aspect, the present disclosure provides a pharmaceutical composition comprising at least one compound represented by formula **(I), (IA), (IB),** (**I-1**), **(II), (IIA), (II-1), (IIA-1), (IIA-1-1),** or **(IIA-1-2)** as described above, an isomer thereof, or a pharmaceutically acceptable salt thereof, and at least one pharmaceutically acceptable carrier or excipient.

In a further aspect, the present disclosure provides the use of the compound represented by formula **(I), (IA), (IB),** (**I-1**), **(II), (IIA), (II-1), (IIA-1), (IIA-1-1),** or **(IIA-1-2)** as described above, an isomer thereof, or a pharmaceutically acceptable salt thereof, or a pharmaceutical composition thereof, in the manufacture of a medicament for preventing and/or treating a disease or disorder associated with NLRP3.

In yet another aspect, the present disclosure further provides the compound represented by formula **(I), (IA), (IB),** (**I-1**), **(II), (IIA), (II-1), (IIA-1), (IIA-1-1),** or **(IIA-1-2)** as described above, an isomer thereof, or a pharmaceutically acceptable salt thereof, or a pharmaceutical composition thereof, for use in preventing and/or treating a disease or disorder associated with NLRP3.

In still another aspect, the present disclosure further provides a method of preventing and/or treating a disease, comprising administering to a patient in need thereof an effective amount of the compound represented by formula **(I), (IA), (IB),** (**I-1**), **(II),** (**IIA**), (**II-1**), **(IIA-1), (IIA-1-1),** or **(IIA-1-2)** as described above, an isomer thereof, or a pharmaceutically acceptable salt thereof, or a pharmaceutical composition thereof; wherein the disease to be prevented and/or treated is a disease or disorder associated with NLRP3.

In some embodiments, the disease or disorder associated with NLRP3 is selected from the group consisting of cancer, an inflammatory disease, or a disease accompanied by an inflammatory response;
the cancer includes, but is not limited to, myeloproliferative neoplasm, myeloid leukemia, lung cancer, nasopharyngeal carcinoma, laryngeal cancer, esophageal cancer, cholangiocarcinoma, oral cancer, head and neck cancer, mesothelioma, adrenocortical carcinoma, kidney cancer, liver cancer, gastric cancer, colon cancer, rectal cancer, bone cancer, brain cancer, breast cancer, melanoma, pancreatic cancer, skin cancer, lymphoma, bladder cancer, small intestine cancer, soft tissue sarcoma, endometrial cancer, cervical cancer, osteosarcoma, prostate cancer, and testicular cancer;
the inflammatory disease or disease accompanied by an inflammatory response includes, but is not limited to:
   1) autoinflammatory diseases, such as cryopyrin-associated periodic syndrome (CAPS), familial Mediterranean fever, Schnitzler syndrome, and mevalonate kinase deficiency (MKD);
   2) chronic pain, including neuropathic pain and non-neuropathic pain;
   3) dermatological disorders, such as contact hypersensitivity, bullous pemphigoid, sunburn, contact dermatitis, seborrheic dermatitis, hidradenitis suppurativa (HS), diabetic (foot) ulcer, lichen planus, scleroderma, pemphigus, epidermolysis bullosa, urticaria, acne, and alopecia;
   4) respiratory disorders, such as chronic obstructive pulmonary disease (COPD), asthma, bronchitis, rhinitis, sinusitis, idiopathic pulmonary fibrosis (IPF), cystic fibrosis, sarcoidosis, adult respiratory distress syndrome, and pneumonia;
   5) joint disorders, such as arthritis;
   6) muscular disorders, such as polymyositis and myasthenia gravis;
   7) cardiovascular disorders, such as hypertension, ischemia, reperfusion injury, vasculitis, and pericarditis;
   8) hematological disorders, such as sickle cell disease;
   9) central nervous system disorders, such as Parkinson's disease, Alzheimer's disease, Huntington's disease, brain injury, multiple sclerosis, and amyotrophic lateral sclerosis;
   10) metabolic diseases, such as type 2 diabetes (T2D), atherosclerosis, obesity, and gout;
   11) liver diseases, such as non-alcoholic fatty liver disease (NAFLD), non-alcoholic steatohepatitis (NASH), alcoholic fatty liver disease (AFLD), and alcoholic steatohepatitis (ASH);
   12) kidney diseases, such as acute kidney disease, hyperoxaluria, chronic kidney disease, nephrocalcinosis, glomerulonephritis, and diabetic nephropathy;
   13) gastrointestinal disorders, such as inflammatory bowel disease and pancreatitis;
   14) ocular disorders, such as uveitis and allergic conjunctivitis;
   15) graft-versus-host disease;
   16) burns, sunburn, and mechanical injury.

In some embodiments, the neuropathic pain includes central neuropathic pain and peripheral neuropathic pain;
the central neuropathic pain includes, but is not limited to, pain associated with spinal cord injury, post-stroke pain, pain associated with multiple sclerosis, pain associated with syringomyelia, pain associated with ischemic myelopathy, pain associated with compressive myelopathy, pain associated with radiation-induced myelopathy, pain associated with Parkinson's disease, phantom limb pain, and pain associated with myelitis;
the peripheral neuropathic pain includes, but is not limited to, postherpetic neuralgia, HIV-associated neuropathy, diabetic peripheral neuropathy, chronic post-traumatic or post-surgical pain, chemotherapy- or radiotherapy-induced neuropathy, trigeminal neuralgia, glossopharyngeal neuralgia, residual limb pain, toxin-induced neuropathy, sciatica, and radicular pain;
the non-neuropathic pain includes, but is not limited to, osteoarthritis pain, chronic low back pain, chronic visceral pain, cancer pain, and fibromyalgia.

Unless otherwise specified, the following terms and phrases used herein are intended to have the following meanings. A particular term or phrase should not be considered indefinite or unclear in the absence of a specific definition, but should be understood according to its ordinary meaning. When a trade name appears herein, it is intended to refer to the corresponding commercial product or its active ingredient.

As used herein, the term "compound" includes, but is not limited to, the following forms of a compound: free bases, stereoisomers, geometric isomers, tautomers, isotopes, pharmaceutically acceptable salts, solvates, hydrates, and prodrugs (esters).

The "compound" of the present disclosure may be asymmetric, for example, having one or more stereoisomers. Unless otherwise specified, all stereoisomers are included, such as enantiomers and diastereomers. In the present disclosure, compounds containing asymmetric carbon atoms may be isolated in optically pure or racemic forms. Optically pure forms may be obtained by resolution of racemic mixtures or by synthesis using chiral starting materials or chiral reagents.

As used herein, the term "isomer" includes, unless otherwise specified, stereoisomers and tautomers. Unless otherwise specified, the term "stereoisomer" refers to compounds having the same chemical structure but differing in the spatial arrangement of atoms or groups. Stereoisomers include, but are not limited to, enantiomers, diastereomers, conformational isomers (rotamers), geometric (cis/trans) isomers, and atropisomers. Any mixture of stereoisomers obtained may be separated into pure or substantially pure geometric isomers, enantiomers, or diastereomers based on differences in physicochemical properties of the components, for example, by chromatography and/or fractional crystallization. Unless otherwise specified, the term "tautomer" refers to structural isomers of different energies which are interconvertible via a low energy barrier. If tautomerism is possible (*e.g.,* in solution), a tautomeric equilibrium may be established. For example, proton tautomers (also known as prototropic tautomers) involve interconversions via proton transfer, such as keto-enol tautomerization and imine-enamine tautomerization. Valence tautomers involve interconversions via reorganization of some of the bonding electrons.

As used herein, the term "isotope" refers to a compound that, unless otherwise specified, may exist in isotopically labeled or enriched form, and contains one or more atoms having an atomic mass or mass number different from that of the most abundant naturally occurring atom. Isotopes may be radioactive or non-radioactive. Isotopes commonly used for isotopic labeling include: hydrogen isotopes, including but not limited to ²H and ³H; carbon isotopes, including but not limited to ¹³C and ¹⁴C; chlorine isotopes, including but not limited to ³⁵Cl and ³⁷Cl; fluorine isotopes, including but not limited to ¹⁸F; iodine isotopes, including but not limited to ¹²³I and ¹²⁵I; nitrogen isotopes, including but not limited to ¹³N and ¹⁵N; oxygen isotopes, including but not limited to ¹⁵O, ¹⁷O, and ¹⁸O; and sulfur isotopes, including but not limited to ³⁵S. These isotopically labeled compounds may be used to study the distribution of pharmaceutical molecules in tissues. In particular, ³H and ¹³C are more widely used due to their ease of labeling and convenient detection. Substitution of certain heavy isotopes, such as deuterium (²H), can enhance metabolic stability and prolong half-life, thereby reducing dosage requirements and providing therapeutic advantages. Isotopically labeled compounds are generally synthesized from labeled starting materials using known synthetic techniques analogous to those used for non-isotopically labeled compounds.

As used herein, the term "pharmaceutically acceptable" refers to compounds, materials, compositions, and/or dosage forms that, within the scope of sound medical judgment, are suitable for use in contact with the tissues of humans and animals without excessive toxicity, irritation, allergic response, or other problems or complications, commensurate with a reasonable benefit/risk ratio.

As used herein, the term "pharmaceutically acceptable salt" refers to salts of the compounds of the present disclosure, formed by contacting a compound having a specific substituent discovered in the present disclosure with one of the following acids: 2-acetoxybenzoic acid, 2-hydroxyethanesulfonic acid, acetic acid, ascorbic acid, benzenesulfonic acid, benzoic acid, bicarbonic acid, carbonic acid, citric acid, edetic acid, ethanedisulfonic acid, ethanesulfonic acid, fumaric acid, glucoheptonic acid, gluconic acid, glutamic acid, glycolic acid, hydrobromic acid, hydrochloric acid, hydroiodic acid, hydroxynaphthoic acid, isethionic acid, lactic acid, lactobionic acid, dodecylsulfonic acid, maleic acid, malic acid, mandelic acid, methanesulfonic acid, nitric acid, oxalic acid, pamoic acid, pantothenic acid, phenylacetic acid, phosphoric acid, polygalacturonic acid, propionic acid, salicylic acid, stearic acid, subacetic acid, succinic acid, sulfamic acid, sulfanilic acid, sulfuric acid, tannic acid, tartaric acid, and*p-*toluenesulfonic acid; or, when a compound contains a relatively acidic functional group, a base addition salt may be obtained by contacting the neutral form of the compound with a sufficient amount of a base in a pure solution or a suitable inert solvent. Pharmaceutically acceptable base addition salts include, but are not limited to, sodium, potassium, calcium, magnesium, ammonium, and organic amine salts. Examples include alkali metal salts, alkaline earth metal salts, other metal salts, inorganic base salts, organic base salts, inorganic acid salts, lower alkyl sulfonates, aryl sulfonates, organic acid salts, and amino acid salts.

As used herein, the term "solvate" refers to a hydrate, ethanol solvate, methanol solvate, acetone solvate, ether solvate, or isopropanol solvate.

The terms "direct bond" and "chemical bond" both refer to a single bond directly connecting two adjacent atoms.

The term "halogen" refers to fluorine (F), chlorine (Cl), bromine (Br), or iodine (I).

The term "hydroxyl" refers to -OH. The term "cyano" refers to -CN. The term "amino" refers to -NH₂.

The term "alkyl" refers to a linear or branched saturated hydrocarbon group consisting of carbon and hydrogen atoms, such as C₁₋₆ alkyl, including but not limited to methyl, ethyl, propyl (including 1-propyl or *n*-propyl and 2-propyl or isopropyl), butyl (including 1-butyl or *n*-butyl, 2-methyl-1-propyl or isobutyl, 2-methyl-1-propyl or isobutyl, 1-methylpropyl or *sec-*butyl, and 1,1-dimethylethyl or *tert-*butyl), pentyl (including 1-pentyl, 2-pentyl, 3-pentyl, 2-methyl-2-butyl, 3-methyl-1-butyl, and 2-methyl-1-butyl), hexyl (including 1-hexyl, 2-hexyl, 3-hexyl, 2-methyl-2-pentyl, 3-methyl-2-pentyl, 4-methyl-2-pentyl, 3-methyl-3-pentyl, 2-methyl-3-pentyl, 2,3-dimethyl-2-butyl, and 3,3-dimethyl-2-butyl).

The term "C₁₋₆ haloalkyl" refers to an alkyl group in which one or more hydrogen atoms are replaced by one or more halogen atoms (*e.g*., fluorine, chlorine, bromine, or iodine), including but not limited to -CF₃, -CH₂Cl, -CH₂CF₃, -CHCl₂, and -CCl₃.

The term "hydroxyalkyl" refers to an alkyl group as defined above further substituted by one or more hydroxyl groups, such as C₁₋₆ hydroxyalkyl, including but not limited to hydroxymethyl, hydroxyethyl, hydroxypropyl, and hydroxyisopropyl.

The term "alkylamino" refers to an open-chain alkyl group containing a nitrogen atom, such as C₁₋₆ alkylamino, including but not limited to methylamino, ethylamino, isopropylamino, dimethylamino, methylethylamino, and diethylamino.

The term "alkylaminoalkyl" refers to an alkyl group further substituted by an alkylamino group as defined above, such as C₁₋₈ alkylamino-C₁₋₈ alkyl or C₁₋₆ alkylamino-C₁₋₆ alkyl, including but not limited to methylaminomethyl, ethylaminomethyl, ethylaminoethyl, isopropylaminomethyl, and propylaminomethyl.

The term "alkylaminoalkyl" refers to an alkyl group further substituted by an alkylamino group as defined above, such as C₁₋₆ alkylaminoalkyl, which refers to C₁₋₆ alkylamino-C₁₋₆ alkyl.

The term "cycloalkyl" refers to a monocyclic alkyl group consisting of carbon and hydrogen atoms, such as C₃₋₈ cycloalkyl, including but not limited to cyclopropyl, cyclobutyl, cyclopentyl, and cyclohexyl.

The term "alkoxy" refers to a linear or branched chain alkyl group linked via an oxygen atom, such as C₁₋₆ alkoxy, including but not limited to methoxy, ethoxy, propoxy (including *n*-propoxy and isopropoxy), butoxy (including *n*-butoxy, isobutoxy, *sec*-butoxy, and *tert*-butoxy), pentoxy (including *n*-pentoxy, isopentoxy, and neopentoxy), and hexyloxy (including *n*-hexyloxy, 2-methylpentoxy, 3-methylpentoxy, 2,3-dimethylbutoxy, and 2,2-dimethylbutoxy).

The term "C₁₋₆ haloalkoxy" refers to an alkoxy group in which one or more hydrogen atoms are replaced by one or more halogen atoms (*e.g*., fluorine, chlorine, bromine, or iodine), including but not limited to -OCF₃, -OCH₂Cl, -OCH₂CF₃, -OCHCl₂, and -OCCl₃.

The term "alkenyl" refers to a linear or branched unsaturated aliphatic hydrocarbon group consisting of carbon and hydrogen atoms and containing at least one carbon-carbon double bond. An alkenyl group may contain 2 to 20 carbon atoms, preferably 2 to 10 carbon atoms (*i.e.,* C₂₋₁₀ alkenyl), more preferably 2 to 8 carbon atoms (*i.e.,* C₂₋₈ alkenyl), even more preferably 2 to 6 carbon atoms (*i.e.,* C₂₋₆ alkenyl), 2 to 5 carbon atoms (*i.e.,* C₂₋₅ alkenyl), 2 to 4 carbon atoms (*i.e.,* C₂₋₄ alkenyl), 2 to 3 carbon atoms (*i.e.,* C₂₋₃ alkenyl), or 2 carbon atoms (*i.e.,* C₂ alkenyl). For example, "C₂₋₆ alkenyl" refers to an alkenyl group containing 2 to 6 (*i.e.,* 2, 3, 4, 5, or 6) carbon atoms on the carbon chain. Non-limiting examples of alkenyl groups include, but are not limited to, vinyl, 1-propenyl, 2-propenyl, 1-butenyl, 2-butenyl, 1-methyl-1-propenyl, 2-methyl-1-propenyl, 1,3-butadien-1-yl, and 1,3-butadien-2-yl.

The term "alkynyl" refers to a linear or branched unsaturated aliphatic hydrocarbon group consisting of carbon and hydrogen atoms and containing at least one carbon-carbon triple bond. An alkynyl group may contain 2 to 20 carbon atoms, preferably 2 to 10 carbon atoms (*i.e.,* C₂₋₁₀ alkynyl), more preferably 2 to 8 carbon atoms (*i.e.,* C₂₋₈ alkynyl), even more preferably 2 to 6 carbon atoms (*i.e.,* C₂₋₆ alkynyl), 2 to 5 carbon atoms (*i.e.,* C₂₋₅ alkynyl), 2 to 4 carbon atoms (*i.e.,* C₂₋₄ alkynyl), 2 to 3 carbon atoms (*i.e.,* C₂₋₃ alkynyl), or 2 carbon atoms (*i.e.,* C₂ alkynyl). For example, "C₂₋₆ alkynyl" refers to an alkynyl group containing 2 to 6 (*i.e.,* 2, 3, 4, 5, or 6) carbon atoms on the carbon chain. Non-limiting examples of alkynyl groups include, but are not limited to, ethynyl, 1-propynyl, 1-butynyl, 1,3-butadiynyl, 1-pentynyl, 3-methyl-1-butynyl, 1,3-pentadiynyl, 1,4-pentadiynyl, 1-hexynyl, 3-methyl-1-pentynyl, 4-methyl-1-pentynyl, 3,3-dimethyl-1-butynyl, 3-ethyl-1-butynyl, 1,3-hexadiynyl, 1,4-hexadiynyl, 3-methyl-1,4-pentadiynyl, and 1,5-hexadiynyl.

The term "cycloalkyl" refers to a monocyclic or bicyclic alkyl group consisting of carbon and hydrogen atoms, such as C₃₋₈ cycloalkyl, including but not limited to cyclopropyl, cyclobutyl, cyclopentyl, and cyclohexyl.

The term "alicyclic ring" refers to a monocyclic aliphatic hydrocarbon ring in which a specified number of carbon atoms are connected via carbon-carbon single and double bonds, preferably containing 3 to 6 carbon atoms. Non-limiting examples of alicyclic rings include, but are not limited to, cyclopropane, cyclobutane, cyclopentane, cyclohexane, and cyclohexene.

The term "aromatic ring" refers to an all-carbon monocyclic or fused polycyclic ring containing 6 to 16 carbon atoms with a fully conjugated π-electron system, including but not limited to a benzene ring, a naphthalene ring, and an anthracene ring, preferably a benzene ring.

The term "aryl" refers to an all-carbon monocyclic or fused polycyclic group containing 6 to 16 carbon atoms with a fully conjugated π-electron system, including but not limited to phenyl, naphthyl, and anthracenyl, preferably phenyl.

The term "heterocycle" refers to a saturated or partially unsaturated monocyclic, bicyclic, or polycyclic non-aromatic ring system containing 3 to 20 ring atoms, wherein one, two, three, or more ring atoms are selected from the group consisting of N, O, and S, and the remaining ring atoms are carbon atoms. Bicyclic or polycyclic heterocycles include spiro, fused, and bridged heterocycles. Such bicyclic or polycyclic heterocycles include those in which one ring is an aromatic ring and the other rings are non-aromatic rings, including spiro, fused, and bridged heterocycles. A bicyclic heterocycle may contain one or more heteroatoms in one or both rings. In some embodiments, a heterocycle further includes a ring system formed by fusion of the heterocycle as defined above with one or more carbocyclic rings, wherein the point of attachment is located on the carbocyclic ring or heterocycle; or, in some embodiments, a ring system formed by fusion of the heterocycle as defined above with one or more aromatic/heteroaromatic rings, wherein the point of attachment is located on the aromatic/heteroaromatic ring or heterocycle; or, in some embodiments, a ring system formed by fusion of the heterocycle as defined above with one or more heterocycles as defined above, wherein the point of attachment is located on any one of the heterocycles. In such cases, the number of members of the ring system of the heterocycle is determined by the total number of ring atoms in the fused ring system. In some embodiments, heterocycles are optionally substituted, for example, unsubstituted (unsubstituted heterocycles) or substituted by one or more substituents (substituted heterocycles). Exemplary 3-membered heterocycles containing one heteroatom include, but are not limited to, aziridine, oxirane, and thiirane. Exemplary 4-membered heterocycles containing one heteroatom include, but are not limited to, azetidine, oxetane, and thietane. Exemplary 5-membered heterocycles containing one heteroatom include, but are not limited to, tetrahydrofuran, dihydrofuran, tetrahydrothiophene, dihydrothiophene, pyrrolidine, dihydropyrrole, and 2,5-pyrrolidinedione. Exemplary 5-membered heterocycles containing two heteroatoms include, but are not limited to, dioxolane, oxathiolane, dithiolane, and 2-oxooxazolidine. Exemplary 5-membered heterocycles containing three heteroatoms include, but are not limited to, triazole, oxadiazole, and thiadiazole. Exemplary 6-membered heterocycles containing one heteroatom include, but are not limited to, piperidine, tetrahydropyran, dihydropyridine, and tetrahydrothiopyran. Exemplary 6-membered heterocycles containing two heteroatoms include, but are not limited to, piperazine, morpholine, and thiomorpholine. Exemplary 6-membered heterocycles containing three heteroatoms include, but are not limited to, triazinane, oxadiazinane, thiadiazinane, oxathiazinane, and dioxazinane. Exemplary 7-membered heterocycles containing one heteroatom include, but are not limited to, azepane, oxepane, and thiepane. Exemplary 8-membered heterocycles containing one heteroatom include, but are not limited to, azocane, oxocane, and thiocane. Exemplary 5-membered heterocycles fused to a C₆ aryl ring (also referred to herein as 5,6-bicyclic heterocycles) include, but are not limited to, indoline, isoindoline, dihydrobenzofuran, dihydrobenzothiophene, and benzoxazolinone. Exemplary 6-membered heterocycles fused to a C₆ aryl ring (also referred to herein as 6,6-bicyclic heterocycles) include, but are not limited to, tetrahydroquinoline and tetrahydroisoquinoline.

The term "heterocyclyl" refers to a substituent derived from the heterocycle as defined above. Exemplary 3-membered heterocyclyl groups containing one heteroatom include, but are not limited to, aziridinyl, oxiranyl, and thiiranyl. Exemplary 4-membered heterocyclyl groups containing one heteroatom include, but are not limited to, azetidinyl, oxetanyl, and thietanyl. Exemplary 5-membered heterocyclyl groups containing one heteroatom include, but are not limited to, tetrahydrofuranyl, dihydrofuranyl, tetrahydrothiophenyl, dihydrothiophenyl, pyrrolidinyl, dihydropyrrolyl, and 2,5-dioxopyrrolidinyl. Exemplary 5-membered heterocyclyl groups containing two heteroatoms include, but are not limited to, dioxolanyl, oxathiolanyl, dithiolanyl, and 2-oxooxazolidinyl. Exemplary 5-membered heterocyclyl groups containing three heteroatoms include, but are not limited to, triazolinyl, oxadiazolinyl, and thiadiazolinyl. Exemplary 6-membered heterocyclyl groups containing one heteroatom include, but are not limited to, piperidinyl, tetrahydropyranyl, dihydropyridyl, and tetrahydrothiopyranyl. Exemplary 6-membered heterocyclyl groups containing two heteroatoms include, but are not limited to, piperazinyl, morpholinyl, dithianyl, and dioxanyl. Exemplary 6-membered heterocyclyl groups containing three heteroatoms include, but are not limited to, triazinanyl, oxadiazinanyl, thiadiazinanyl, oxathiazinanyl, and dioxazinanyl. Exemplary 7-membered heterocyclyl groups containing one heteroatom include, but are not limited to, azepanyl, oxepanyl, and thiepanyl. Exemplary 8-membered heterocyclyl groups containing one heteroatom include, but are not limited to, azocanyl, oxocanyl, and thiocanyl. Exemplary 5-membered heterocyclyl groups fused to a C₆ aryl ring (also referred to herein as 5,6-bicyclic heterocyclyl groups) include, but are not limited to, indolinyl, isoindolinyl, dihydrobenzofuranyl, dihydrobenzothiophenyl, and benzoxazolinonyl. Exemplary 6-membered heterocyclyl groups fused to a C₆ aryl ring (also referred to herein as 6,6-bicyclic heterocyclyl groups) include, but are not limited to, tetrahydroquinolinyl and tetrahydroisoquinolinyl.

The term "heteroaromatic ring" refers to an aromatic monocyclic, bicyclic, or polycyclic ring system having 5 to 16 ring members, preferably 5 to 14 ring members, 5 to 12 ring members, 5 to 10 ring members, or 5 to 8 ring members, and more preferably 5 to 6 ring members, wherein one, two, three, or more ring atoms are heteroatoms and the remaining ring atoms are carbon atoms; the heteroatoms are independently selected from the group consisting of O, N, and S, and the number of heteroatoms is preferably 1, 2, or 3. Bicyclic or polycyclic heteroaromatic rings include fused heteroaromatic rings. Examples of heteroaromatic rings include, but are not limited to, furan, thiophene, oxazole, thiazole, isoxazole, oxadiazole, thiadiazole, pyrrole, pyrazole, imidazole, triazole, tetrazole, pyridine, pyrimidine, pyrazine, pyridazine, thiodiazole, triazine, phthalazine, quinoline, isoquinoline, pteridine, purine, indole, isoindole, indazole, benzofuran, benzothiophene, benzopyridine, benzopyrimidine, benzopyrazine, benzimidazole, benzophthalazine, pyrrolo[2,3-b]pyridine, imidazo[1,2-a]pyridine, pyrazolo[1,5-a]pyridine, pyrazolo[1,5-a]pyrimidine, imidazo[1,2-b]pyridazine, [1,2,4]triazolo[4,3-b]pyridazine, [1,2,4]triazolo[1,5-a]pyrimidine, and [1,2,4]triazolo[1,5-a]pyridine.

The term "heteroaryl" refers to a substituent derived from the heteroaromatic ring as defined above. Examples of heteroaryl groups include, but are not limited to, furyl, thienyl, oxazolyl, thiazolyl, isoxazolyl, oxadiazolyl, thiadiazolyl, pyrrolyl, pyrazolyl, imidazolyl, triazolyl, tetrazolyl, pyridinyl, pyrimidinyl, pyrazinyl, pyridazinyl, thiodiazolyl, triazinyl, phthalazinyl, quinolinyl, isoquinolinyl, pteridinyl, purinyl, indolyl, isoindolyl, indazolyl, benzofuranyl, benzothienyl, benzopyridinyl, benzopyrimidinyl, benzopyrazinyl, benzimidazolyl, benzophthalazinyl, pyrrolo[2,3-b]pyridinyl, imidazo[1,2-a]pyridinyl, pyrazolo[1,5-a]pyridinyl, pyrazolo[1,5-a]pyrimidinyl, imidazo[1,2-b]pyridazinyl, [1,2,4]triazolo[4,3-b]pyridazinyl, [1,2,4]triazolo[1,5-a]pyrimidinyl, and [1,2,4]triazolo[1,5-a]pyridinyl.

The term "pharmaceutical composition" refers to a mixture comprising one or more compounds of the present disclosure or pharmaceutically acceptable salts thereof together with pharmaceutically acceptable excipients. The purpose of the pharmaceutical composition is to facilitate administration of the compounds of the present disclosure to a subject.

As used herein, the terms "a", "an", "the", "at least one", and "one or more" are interchangeable. Thus, for example, a mixture comprising "a" pharmaceutically acceptable excipient may be interpreted as meaning that the pharmaceutical composition comprises "one or more" pharmaceutically acceptable excipients.

The term "pharmaceutically acceptable excipient" refers to excipients that cause no significant irritation to a subject and do not impair the biological activity and properties of the active compound. Suitable excipients are well known to those skilled in the art, such as carbohydrates, waxes, water-soluble and/or water-swellable polymers, hydrophilic or hydrophobic materials, gelatin, oils, solvents, and water.

The pharmaceutical composition of the present disclosure may be prepared by combining the compound of the present disclosure with suitable pharmaceutically acceptable excipients, and may be, for example, formulated as solid, semi-solid, liquid, or gaseous preparations, such as tablets, pills, capsules, powders, granules, ointments, emulsions, suspensions, suppositories, injections, inhalants, gels, microspheres, and aerosols.

The compounds of the present disclosure, as well as prodrugs, isomers, solvates, or pharmaceutically acceptable salts thereof, or pharmaceutical compositions thereof, may be administered via routes including, but not limited to, oral, rectal, transmucosal, enteral, topical transdermal, inhalation, parenteral, sublingual, intravaginal, intranasal, intraocular, intraperitoneal, intramuscular, subcutaneous, and intravenous administration.

The term "treatment" generally refers to obtaining a desired pharmacological and/or physiological effect. The effect may be therapeutic in terms of partial or complete stabilization or cure of a disease and/or side effects caused by the disease. As used herein, "treatment" encompasses any treatment of a disease in a patient, including: (a) inhibiting the symptoms of the disease, *i.e.,* arresting its development; or (b) alleviating the symptoms of the disease, *i.e.,* causing regression of the disease or symptoms.

The term "effective amount" refers to an amount of the compound of the present disclosure that is sufficient to: (i) treat or prevent a particular disease, condition, or disorder; (ii) alleviate, ameliorate, or eliminate one or more symptoms of a particular disease, condition, or disorder; or (iii) prevent or delay the onset of one or more symptoms of a particular disease, condition, or disorder described herein. The amount of the compound of the present disclosure constituting a "therapeutically effective amount" will vary depending on the compound, the disease state and its severity, the mode of administration, and the age of the mammal to be treated, but can be routinely determined by those skilled in the art based on their own knowledge and the present disclosure.

Compared with the prior art, the present disclosure provides the following advantages:

Based on the target design of NLRP3 inhibitors, the present disclosure has developed pyrazolopyrimidine compounds with completely novel structures. Relevant biological studies have demonstrated that the compounds of the present disclosure exhibit significant inhibitory activity against IL-1β expression in both THP-1 cells and PBMCs, high selectivity for the NLRP3 inflammasome, low hERG toxicity, favorable metabolic stability, and desirable pharmacokinetic properties. In addition, the compounds of the present disclosure exhibit no significant inhibitory effects on various cytochrome P450 (CYP450) enzymes and demonstrate a favorable safety profile in long-term toxicity studies in rats. Furthermore, the compounds of the present disclosure can significantly reduce serum levels of the cytokines IL-1 β, TNF-α, and IL-6 in mouse inflammation models, indicating great clinical application prospects. Moreover, the synthetic route for the compounds provided by the present disclosure is novel, safe, environmentally friendly, and highly feasible for industrial production.

### BRIEF DESCRIPTION OF THE DRAWINGS

FIG. 1 shows the effect of Compound **2** on LPS-induced serum levels of cytokine IL-1β in mice in Example 26.
FIG. 2 shows the effect of Compound **2** on LPS-induced serum levels of cytokine TNF-α in mice in Example 26.
FIG. 3 shows the effect of Compound **2** on LPS-induced serum levels of cytokine IL-6 in mice in Example 26.
FIG. 4 shows the effect of Compound **4** hydrochloride on LPS-induced serum levels of cytokine IL-1β in mice in Example 27.
FIG. 5 shows the effect of Compound **4** hydrochloride on LPS-induced serum levels of cytokine TNF-α in mice in Example 27.
FIG. 6 shows the effect of Compound **4** hydrochloride on LPS-induced serum levels of cytokine IL-6 in mice in Example 27.
FIG. 7 shows the effect of intragastric administration of Compound **4** hydrochloride for 28 consecutive days on the body weight of SD male rats in Example 28.
FIG. 8 shows the effect of intragastric administration of Compound **4** hydrochloride for 28 consecutive days on the body weight of SD female rats in Example 28.

### DETAILED DESCRIPTION OF THE PREFERRED EMBODIMENT

The following are specific examples of the present disclosure, which provide a further description of the technical solutions of the present disclosure. However, the protection scope of the present disclosure is not limited to these examples. Any modifications or equivalent substitutions that do not depart from the concept of the present disclosure shall fall within the scope of protection of the present disclosure.

In addition, all operations involving starting materials that are susceptible to oxidation or hydrolysis are conducted under a nitrogen atmosphere. Unless otherwise specified, all starting materials used in the present disclosure are commercially available and may be used directly without further purification.

The starting materials and common intermediates involved in the examples of the present disclosure may be obtained commercially or prepared in-house. The preparation procedures for self-prepared starting materials and common intermediates are described in detail below.

**Compound 1-8:** (*R*)-1-Methylpiperidin-3-amine was purchased from Shanghai Bide Pharmatech Co., Ltd. According to the certificate of analysis provided by the API supplier, the purity (NMR) was 97% and the specific optical rotation was +9.8417° (c = 1.0 g/100 mL, CHCl₃). (*S*)-1-Methylpiperidin-3-amine was purchased from Leyan Reagent (Shanghai Haohong Biomedical Technology Co., Ltd.). According to the certificate of analysis provided by the API supplier, the purity (NMR) was 95.0% or more and the specific optical rotation was -6.7° (c = 0.45 g/100 mL, CHCl₃).

The following abbreviations are used in the examples:
TsOH·H₂O: p-toluenesulfonic acid monohydrate; MeOH (CH₃OH): methanol; MeB(OH)₂: methylboronic acid; Pd(dppf)Cl₂: [1,1'-bis(diphenylphosphino)ferrocene]dichloropalladium(II); K₂CO₃: potassium carbonate; Dioxane: 1,4-dioxane; H₂O: water; NaNO₂: sodium nitrite; SnCl₂·2H₂O: Tin(II) chloride dihydrate; HCl: hydrochloric acid; TEA: triethylamine; THF: tetrahydrofuran; ACN: acetonitrile; DIPEA: N,N-diisopropylethylamine; n-BuOH: n-butanol; BBr₃: boron tribromide; DCM: dichloromethane; NBS: N-bromosuccinimide; CuI: copper(I) iodide; CsF: cesium fluoride; DMF: *N,N*-dimethylformamide; LiAlH₄: lithium aluminum hydride; NaH: sodium hydride; Na₂CO₃: sodium carbonate; Fe: iron powder; NH₄Cl: ammonium chloride; EtOH: ethanol; IPA (*i*-PrOH): isopropanol; NaBH₃CN: sodium cyanoborohydride; (HCHO)ₙ: paraformaldehyde; EA: ethyl acetate; NaCl: sodium chloride; NaHCO₃: sodium bicarbonate; Na₂SO₄: sodium sulfate; KF: potassium fluoride; PE: petroleum ether; LC-MS: liquid chromatography-mass spectrometry; NADPH: reduced nicotinamide adenine dinucleotide phosphate; UDPGA: uridine diphosphate glucuronic acid; PB: phosphate buffer; CYP3A4: cytochrome P450 3A4; UGT: uridine diphosphate glucuronosyltransferase. In the examples of the present disclosure, x mL × y indicates that an operation is repeated y times using x mL each time. For example, extraction with EA (80 mL × 3) means extraction with 80 mL of EA each time, repeated three times.

### Preparation of Intermediate 1-2

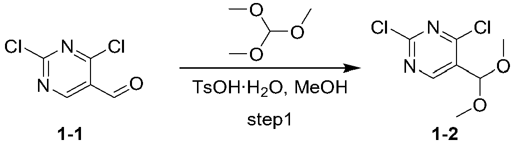

### Step 1: Synthesis of 2,4-dichloro-5-(dimethoxymethyl)pyrimidine (1-2)

Compound **1-1** (500.0 mg, 2.82 mmol), TsOH·H₂O (32.3 mg, 0.17 mmol), and trimethyl orthoformate (1.08 g, 10.18 mmol) were sequentially added to a flask, followed by addition of MeOH (6 mL), and the mixture was stirred at 65°C for 16 hours. After completion of the reaction, the reaction mixture was concentrated under reduced pressure. The residue was purified by column chromatography (EA: PE = 1:5) to obtain Intermediate **1-2** (439.0 mg) as a colorless liquid with a yield of 73.5%.

LC-MS (m/z): 223.0 [M+H]⁺.

### Example 1: Synthesis of (R)-2-(4-chloro-2-methoxy-6-methylphenyl)-N-(1-methylpiperidin-3-yl)-2H-pyrazolo[3,4-d]pyrimidin-6-amine (Target Compound 1)

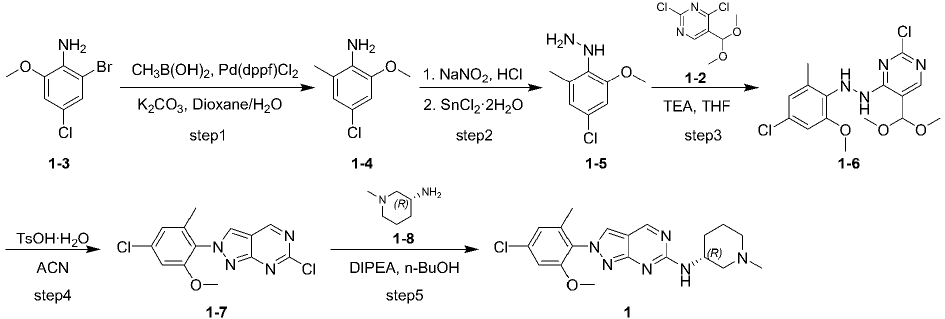

### Step 1: Synthesis of 4-chloro-2-methoxy-6-methylaniline (1-4)

Compound **1-3** (5.00 g, 21.14 mmol), methylboronic acid (2.54 g, 42.43 mmol), K₂CO₃ (8.77 g, 63.45 mmol), and Pd(dppf)Cl₂ (774.0 mg, 1.06 mmol) were sequentially added to a flask, followed by addition of dioxane (60 mL) and water (12 mL), and the mixture was stirred at 100°C for 16 hours under a nitrogen atmosphere. After completion of the reaction, the reaction mixture was diluted with water (50 mL) and extracted with EA (80 mL × 3). The combined organic phases were washed with saturated NaCl solution (80 mL), dried over anhydrous Na₂SO₄, filtered, and the filtrate was concentrated under reduced pressure. The residue was purified by column chromatography (EA: PE = 1:15) to obtain Compound **1-4** (3.48 g) as a yellow solid with a yield of 95.7%.

LC-MS (m/z): 172.0 [M+H]⁺.

### Step 2: Synthesis of (4-chloro-2-methoxy-6-methylphenyl)hydrazine (1-5)

Compound **1-4** (1.87 g, 10.91 mmol) was added to a flask, followed by addition of concentrated hydrochloric acid (13 mL). NaNO₂ (1.13 g, 16.38 mmol) dissolved in distilled water (13 mL) was added at 0°C, and the mixture was stirred for 0.5 hours. A solution of SnCl₂·2H₂O (4.93 g, 21.85 mmol) in concentrated hydrochloric acid (13 mL) was then added at 0°C, and the mixture was stirred for 1 hour. After completion of the reaction, the reaction mixture was filtered. The filter cake was added with NaOH solution (3 N) to adjust the pH to 8 to 9, diluted with water (30 mL), and extracted with DCM (60 mL × 3). The combined organic phases were washed with saturated NaCl solution (50 mL), dried over anhydrous Na₂SO₄, filtered, and the filtrate was concentrated under reduced pressure to obtain Compound **1-5** (1.58 g) as a yellow solid with a yield of 77.6%.

LC-MS (m/z): 187.0 [M+H]⁺.

### Step 3: Synthesis of 2-chloro-4-(2-(4-chloro-2-methoxy-6-methylphenyl)hydrazinyl)-5-(dimethoxymethyl)pyrimidine (1-6)

Compound **1-5** (387.3 mg, 2.08 mmol), Compound **1-2** (463.0 mg, 2.08 mmol), and TEA (631.5 mg, 6.24 mmol) were sequentially added to a flask, followed by addition of THF (8 mL), and the mixture was stirred at room temperature for 16 hours under a nitrogen atmosphere. After completion of the reaction, the reaction mixture was concentrated under reduced pressure. The residue was purified by column chromatography (EA: PE = 1:5) to obtain Compound **1-6** (439.0 mg) as a yellow solid with a yield of 56.7%.

LC-MS (m/z): 373.0 [M+H]⁺.

### Step 4: Synthesis of 6-chloro-2-(4-chloro-2-methoxy-6-methylphenyl)-2H-pyrazolo[3,4-d]pyrimidine (1-7)

Compound **1-6** (339.0 mg, 0.91 mmol) and TsOH·H₂O (172.8 mg, 0.91 mmol) were sequentially added to a flask, followed by addition of ACN (6 mL), and the mixture was stirred at 70°C for 2 hours. After completion of the reaction, the reaction mixture was concentrated under reduced pressure. The residue was purified by column chromatography (EA: PE = 1:2) to obtain Compound **1-7** (235.0 mg) as a yellow solid with a yield of 73.0%.

LC-MS (m/z): 309.0 [M+H]⁺.

### Step 5: Synthesis of (R)-2-(4-chloro-2-methoxy-6-methylphenyl)-N-(1-methylpiperidin-3-yl)-2H-pyrazolo[3,4-d]pyrimidin-6-amine (Target Compound 1)

Compound **1-7** (225.0 mg, 0.73 mmol), Compound **1-8** (99.8 mg, 0.87 mmol), and DIPEA (470.3 mg, 3.64 mmol) were sequentially added to a flask, followed by addition of*n-*BuOH (9 mL), and the mixture was stirred at 100°C for 1 hour under microwave irradiation. After completion of the reaction, the reaction mixture was concentrated under reduced pressure. The residue was purified by column chromatography (DCM: MeOH = 10:1) and lyophilized to obtain **Target Compound 1** (250.0 mg) as a yellow solid with a yield of 89.0%.

LC-MS (m/z): 387.0 [M+H]⁺.

¹H NMR (400 MHz, DMSO-*d*₆) δ 9.08 (s, 1H), 8.42 (s, 1H), 8.16 (s, 1H), 7.21 (d,*J* = 1.6 Hz, 1H), 7.13 (d, *J* = 1.6 Hz, 1H), 4.08-3.93 (m, 1H), 3.74 (s, 3H), 3.06-2.92 (m, 2H), 2.78-2.64 (m, 1H), 2.28 (s, 3H), 2.15-2.01 (m, 1H), 1.99 (s, 3H), 1.90-1.80 (m, 1H), 1.79-1.67 (m, 1H), 1.64-1.49 (m, 1H), 1.43-1.30 (m, 1H).

### Example 2: Synthesis of (R)-5-chloro-3-methyl-2-(6-((1-methylpiperidin-3-yl)amino)-2H-pyrazolo[3,4-d]pyrimidin-2-yl)phenol (Target Compound 2)

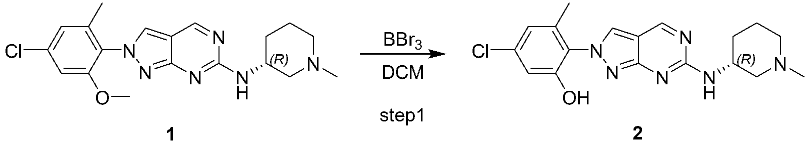

Compound **1** (120.0 mg, 0.31 mmol) was added to a flask, followed by addition of ultra-dry DCM (5 mL). Under a nitrogen atmosphere, a solution of BBr₃ in DCM (1.6 mL, 3.10 mmol, 2 M) was added at 0°C, and the mixture was stirred at 0°C for 1 hour. After completion of the reaction, the reaction mixture was quenched with an appropriate amount of MeOH in an ice-water bath, and the filtrate was concentrated under reduced pressure. The residue was purified by reverse-phase chromatography (ACN: 0.1% aqueous formic acid = 85%) to obtain **Target Compound 2** (69.1 mg) as a yellow solid with a yield of 60.1%.

LC-MS (m/z): 373.0 [M+H]⁺.

¹H NMR (400 MHz, DMSO-*d*₆) δ 9.08 (s, 1H), 8.41 (s, 1H), 8.16 (s, 1H), 7.22-7.05 (m, 1H), 7.00-6.86 (m, 2H), 4.13-3.95 (m, 1H), 3.13-2.95 (m, 1H), 2.86-2.71 (m, 1H), 2.33 (s, 3H), 2.21-2.03 (m, 2H), 1.97 (s, 3H), 1.91-1.82 (m, 1H), 1.79-1.69 (m, 1H), 1.65-1.51 (m, 1H), 1.45-1.31 (m, 1H).

### Example 3: Synthesis of (R)-5-bromo-3-methyl-2-(6-((1-methylpiperidin-3-yl)amino)-2H-pyrazolo[3,4-d]pyrimidin-2-yl)phenol (Target Compound 3)

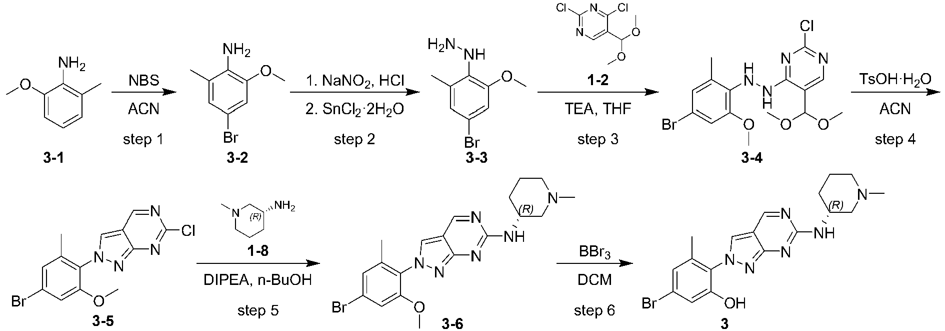

### Step 1: Synthesis of 4-bromo-2-methoxy-6-methylaniline (3-2)

Compound **3-1** (22.00 g, 160.30 mmol) was dissolved in ACN (220 mL), and NBS (34.25 g, 192.40 mmol) was slowly added in an ice-water bath. After completion of the addition, the mixture was stirred at the same temperature for 10 minutes. The reaction mixture was concentrated under reduced pressure to remove most of the solvent, diluted with water (100 mL), and extracted twice with DCM/MeOH (10:1, 100 mL). The combined organic phases were washed with saturated NaCl solution (100 mL), dried over anhydrous Na₂SO₄, filtered, and concentrated. The residue was purified by column chromatography (EA: PE = 4:96) to obtain Compound **3-2** (22.34 g) as a dark brown solid with a yield of 64.5%.

LC-MS (m/z): 216.0/218.0 [M+H]⁺.

### Step 2: Synthesis of 4-bromo-2-methoxy-6-methylphenylhydrazine (3-3)

Compound **3-2** (5.00 g, 23.15 mmol) was dissolved in concentrated hydrochloric acid (25 mL) in an ice-salt bath. A solution of NaNO₂ (2.40 g, 34.72 mmol) in water (25 mL) was slowly added dropwise while maintaining the internal temperature below 0°C, and the mixture was stirred at the same temperature for 20 minutes. A solution of SnCl₂·2H₂O (10.40 g, 46.30 mmol) in concentrated hydrochloric acid (25 mL) was then slowly added dropwise while maintaining the internal temperature below 5°C, and the mixture was stirred at the same temperature for 1 hour. The reaction mixture was filtered. The filter cake was washed with a small amount of water, then diluted with DCM (50 mL), and added with saturated Na₂CO₃ aqueous solution to adjust the pH to alkaline. The phases were separated, and the aqueous phase was extracted with DCM (50 mL). The combined organic phases were washed with saturated NaCl solution (50 mL), dried over anhydrous Na₂SO₄, filtered, and concentrated under reduced pressure to obtain crude Compound **3-3** (3.98 g) as a dark brown oily liquid.

### Step 3: Synthesis of 4-(2-(4-bromo-2-methoxy-6-methylphenyl)hydrazinyl)-2-chloro-5-(dimethoxymethyl)pyrimidine (3-4)

Crude Compound **3-3** (3.98 g) and Compound **1-2** (3.84 g, 17.23 mmol) were dissolved in THF (40 mL), followed by addition of TEA (7.16 mL, 51.67 mmol), and the mixture was stirred at room temperature for 16 hours. The reaction mixture was directly concentrated. The residue was purified by column chromatography (EA: PE = 35:65) to obtain Compound **3-4** (3.05 g) as a brown solid with a two-step yield of 31.6%.

LC-MS (m/z): 419.0 [M+H]⁺.

### Step 4: Synthesis of 2-(4-bromo-2-methoxy-6-methylphenyl)-6-chloro-2H-pyrazolo[3,4-d]pyrimidine (3-5)

Compound **3-4** (3.05 g, 7.30 mmol) was dissolved in ACN (30 mL), followed by addition of TsOH·H₂O (1.39 g, 7.30 mmol), and the reaction mixture was stirred at 70°C for 1 hour. The reaction mixture was cooled to room temperature and concentrated. The residue was purified by column chromatography (EA: PE = 1:1) to obtain Compound **3-5** (2.82 g) as a brown solid with a yield of 99.9%.

LC-MS (m/z): 355.0 [M+H]⁺.

### Step 5: Synthesis of (R)-2-(4-bromo-2-methoxy-6-methylphenyl)-N-(1-methylpiperidin-3-yl)-2H-pyrazolo[3,4-d]pyrimidin-6-amine (3-6)

Compound **3-5** (2.82 g, 7.30 mmol) and Compound **1-8** (1.00 g, 8.76 mmol) were dissolved in *n*-BuOH (20 mL), followed by addition of DIPEA (3.82 mL, 21.90 mmol), and the mixture was stirred at 100°C for 1 hour. The reaction mixture was cooled to room temperature and concentrated under reduced pressure. The residue was purified by column chromatography (MeOH: DCM = 5:95) to obtain Compound **3-6** (2.66 g) as an off-white solid with a yield of 84.4%.

LC-MS (m/z): 431.0/433.0 [M+H]⁺.

### Step 6: Synthesis of (R)-5-bromo-3-methyl-2-(6-((1-methylpiperidin-3-yl)amino)-2H-pyrazolo[3,4-d]pyrimidin-2-yl)phenol (Target Compound 3)

Compound **3-6** (0.20 g, 0.46 mmol) was dissolved in DCM (10 mL), followed by addition of a solution of BBr₃ in DCM (2.0 M, 1.2 mL) in an ice-water bath, and the mixture was stirred at room temperature for 1 hour. The reaction mixture was quenched with MeOH, diluted with DCM (20 mL), washed with saturated NaHCO₃ aqueous solution (20 mL) and saturated NaCl solution (20 mL), dried over anhydrous Na₂SO₄, filtered, and concentrated under reduced pressure. The residue was purified by column chromatography (MeOH: DCM = 15:85), and further purified by reverse-phase chromatography (ACN: 0.1% aqueous formic acid = 85%) to obtain **Target Compound 3** (25.8 mg) as a white solid with a yield of 13.3%.

LC-MS (m/z): 417.0/419.0 [M+H]⁺.

¹H NMR (400 MHz, DMSO-*d*₆) δ 9.06 (s, 1H), 8.40 (s, 1H), 7.17-6.99 (m, 3H), 4.04-3.92 (m, 1H), 3.01-2.90 (m, 1H), 2.69-2.63 (m, 1H), 2.22 (s, 3H), 2.05-1.88 (m, 5H), 1.88-1.80 (m, 1H), 1.75-1.67 (m, 1H), 1.62-1.48 (m, 1H), 1.40-1.27 (m, 1H).

### Example 4: Synthesis of (R)-3-methyl-2-(6-((1-methylpiperidin-3-yl)amino)-2H-pyrazolo[3,4-d]pyrimidin-2-yl)-5-(trifluoromethyl)phenol (Target Compound 4)

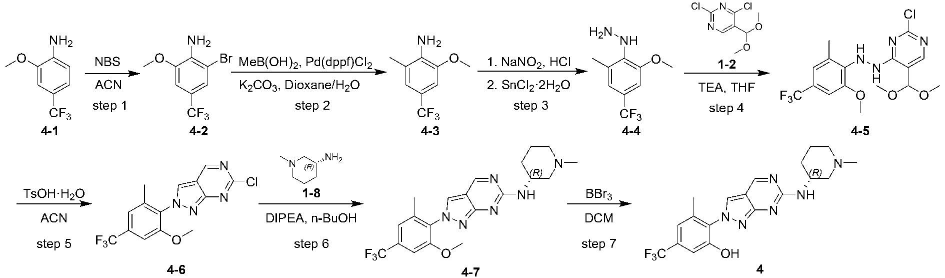

### Step 1: Synthesis of 2-bromo-6-methoxy-4-(trifluoromethyl)aniline (4-2)

Compound **4-1** (1.00 g, 5.23 mmol) was dissolved in ACN (20 mL). NBS (977.5 mg, 5.49 mmol) was slowly added in an ice-water bath while maintaining the internal temperature below 10°C, and the mixture was stirred at room temperature for 1 hour. The reaction mixture was diluted with EA (50 mL), washed with NaHCO₃ aqueous solution (30 mL), water (20 mL), and saturated NaCl solution (20 mL), dried over anhydrous Na₂SO₄, filtered, and concentrated under reduced pressure. The residue was purified by column chromatography (EA: PE = 7:93) to obtain Compound **4-2** (1.15 g) as a brown solid with a yield of 81.6%.

LC-MS (m/z): 270.0/272.0 [M+H]⁺.

### Step 2: 2-methoxy-6-methyl-4-(trifluoromethyl)aniline (4-3)

Compound **4-2** (1.14 g, 4.25 mmol), MeB(OH)₂ (0.51 g, 8.50 mmol), K₂CO₃ (1.47 g, 10.62 mmol), and Pd(dppf)Cl₂ (115.5 mg, 0.21 mmol) were sequentially added to a flask, followed by addition of dioxane (20 mL) and water (2 mL), and the mixture was stirred at 100°C for 16 hours under a nitrogen atmosphere. The reaction mixture was cooled to room temperature, diluted with water (50 mL), and extracted with EA (50 mL × 2). The combined organic phases were washed with saturated NaCl solution (50 mL), dried over anhydrous Na₂SO₄, filtered, and concentrated under reduced pressure. The residue was purified by column chromatography (EA: PE = 1:9) to obtain Compound **4-3** (568.0 mg) as a green oily liquid with a yield of 65.1%.

LC-MS (m/z): 206.0 [M+H]⁺.

### Step 3: 2-methoxy-6-methyl-4-(trifluoromethyl)phenylhydrazine (4-4)

Compound **4-3** (568.0 mg, 2.77 mmol) was dissolved in concentrated hydrochloric acid (5 mL) in an ice-salt bath. A solution of NaNO₂ (286.5 mg, 4.15 mmol) in water (5 mL) was slowly added dropwise while maintaining the internal temperature below 0°C, and the mixture was stirred at the same temperature for 40 minutes. A solution of SnCl₂·2H₂O (1.25 g, 5.34 mmol) in concentrated hydrochloric acid (3 mL) was then slowly added dropwise while maintaining the internal temperature below 0°C, and the mixture was stirred at the same temperature for 1 hour. The reaction mixture was filtered. The filter cake was washed with a small amount of water, then diluted with EA (20 mL), and added with saturated Na₂CO₃ aqueous solution to adjust the pH to alkaline. The phases were separated, and the aqueous phase was extracted with EA (20 mL). The combined organic phases were washed with saturated NaCl solution (20 mL), dried over anhydrous Na₂SO₄, filtered, and concentrated under reduced pressure to obtain Compound **4-4** (135.0 mg) as an off-white solid with a yield of 22.1%.

### Step 4: Synthesis of 2-chloro-5-(dimethoxymethyl)-4-(2-(2-methoxy-6-methyl-4-(trifluoromethyl)phenyl)hydrazinyl)pyrimidine (4-5)

Compound **4-4** (135.0 mg, 0.61 mmol) and Compound **1-2** (136.8 mg, 0.61 mmol) were dissolved in THF (50 mL), followed by addition of TEA (0.25 mL, 1.84 mmol), and the mixture was stirred at room temperature for 16 hours. The reaction mixture was directly concentrated. The residue was purified by column chromatography (EA: PE = 35:65) to obtain Compound **4-5** (88.0 mg) as a yellow solid with a yield of 35.3%.

LC-MS (m/z): 407.0 [M+H]⁺.

### Step 5: Synthesis of 6-chloro-2-(2-methoxy-6-methyl-4-(trifluoromethyl)phenyl)-2H-pyrazolo[3,4-d]pyrimidine (4-6)

Compound **4-5** (88.0 mg, 0.22 mmol) was dissolved in ACN (5 mL), followed by addition of TsOH·H₂O (41.1 mg, 0.22 mmol), and the reaction mixture was stirred at 70°C for 1 hour. The reaction mixture was cooled to room temperature and concentrated. The residue was purified by column chromatography (EA: PE = 30:70) to obtain Compound **4-6** (59.0 mg) as a yellow oily liquid with a yield of 79.7%.

LC-MS (m/z): 343.0 [M+H]⁺.

### Step 6: Synthesis of (R)-2-(2-methoxy-6-methyl-4-(trifluoromethyl)phenyl)-N-(1-methylpiperidin-3-yl)-2H-pyrazolo[3,4-d]pyrimidin-6-amine (4-7)

Compound **4-6** (59.0 mg, 0.17 mmol) and Compound **1-8** (23.6 mg, 0.21 mmol) were dissolved in *n*-BuOH (3 mL), followed by addition of DIPEA (0.09 mL, 0.52 mmol), and the mixture was stirred at 100°C for 1 hour. The reaction mixture was cooled to room temperature and concentrated under reduced pressure. The residue was purified by column chromatography (MeOH: DCM = 5:95) to obtain Compound **4-7** (128.0 mg) as an off-white solid.

LC-MS (m/z): 421.0 [M+H]⁺.

### Step 7: Synthesis of (R)-3-methyl-2-(6-((1-methylpiperidin-3-yl)amino)-2H-pyrazolo[3,4-d]pyrimidin-2-yl)-5-(trifluoromethyl)phenol (Target Compound 4)

Compound **4-7** (128.0 mg) was dissolved in DCM (5 mL), followed by addition of a solution of BBr₃ in DCM (2.0 M, 0.5 mL) in an ice-water bath, and the mixture was stirred at room temperature for 1 hour. The reaction mixture was quenched with MeOH and concentrated under reduced pressure. The residue was purified by reverse-phase chromatography (ACN: 0.1% aqueous formic acid = 85%) to obtain **Target Compound 4** (41.3 mg) as a white solid with a two-step yield of 47.0% and e.e.% > 99.9%.

LC-MS (m/z): 407.0 [M+H]⁺.

¹H NMR (400 MHz, DMSO-*d*₆) δ 9.10 (s, 1H), 8.48 (s, 1H), 7.23 (s, 1H), 7.20-7.13 (m, 2H), 4.08-3.94 (m, 1H), 3.06-2.98 (m, 1H), 2.80-2.70 (m, 1H), 2.30 (s, 3H), 2.21-1.99 (m, 5H), 1.91-1.82 (m, 1H), 1.79-1.69 (m, 1H), 1.65-1.50 (m, 1H), 1.45-1.29 (m, 1H).

### Example 5: Synthesis of hydrochloride salt of (R)-3-methyl-2-(6-((1-methylpiperidin-3-yl)amino)-2H-pyrazolo[3,4-d]pyrimidin-2-yl)-5-(trifluoromethyl)phenol (Target Compound 4)

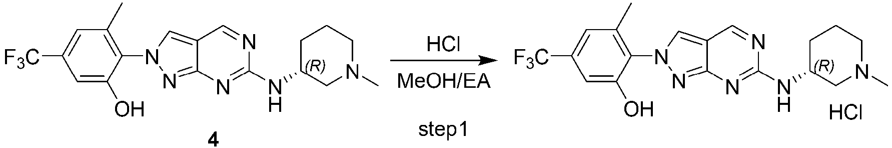

Compound **4** (3.22 g) was dissolved in MeOH (40 mL), followed by addition of a solution of HCl in EA (4.0 M, 10 mL), and the mixture was stirred at room temperature for 1 hour. The reaction mixture was concentrated and dried to obtain the hydrochloride salt of Compound 4 (3.47 g) as a yellow solid with a yield of 98.9%.

LC-MS (m/z): 407.0 [M+H]⁺.

¹H NMR (600 MHz, DMSO-*d*₆) δ 11.09 (brs, 1H), 10.55-10.35 (m, 1H), 9.30-9.15 (m, 1H), 8.72-8.56 (m, 1H), 8.10-7.70 (m, 1H), 7.31 (d, *J* = 2.0 Hz, 1H), 7.25 (d, *J =* 2.0 Hz, 1H), 4.32-4.24 (m, 1H), 3.65-3.54 (m, 1H), 3.41-3.33 (m, 1H), 2.92-2.74 (m, 5H), 2.08 (s, 3H), 2.07-2.01 (m, 1H), 1.95-1.76 (m, 2H), 1.50 (m, 1H).

### Example 6: Synthesis of (R)-3,5-dimethyl-2-(6-((1-methylpiperidin-3-yl)amino)-2H-pyrazolo[3,4-d]pyrimidin-2-yl)phenol (Target Compound 5)

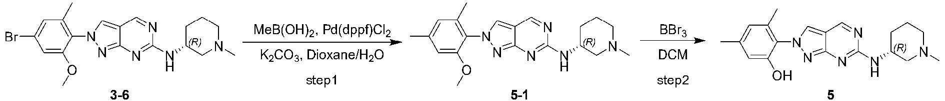

### Step 1: Synthesis of (R)-2-(2-methoxy-4,6-dimethylphenyl)-N-(1-methylpiperidin-3-yl)-2H-pyrazolo[3,4-d]pyrimidin-6-amine (5-1)

Compound **3-6** (100.0 mg, 0.23 mmol) was added to a flask, followed by addition of dioxane (2 mL) and water (0.4 mL). MeB(OH)₂ (27.8 mg, 0.46 mmol), K₂CO₃ (96.2 mg, 0.70 mmol), and Pd(dppf)Cl₂ (17.0 mg, 0.02 mmol) were then sequentially added, and the mixture was stirred at 100°C for 16 hours under a nitrogen atmosphere. After completion of the reaction, the reaction mixture was diluted with water (10 mL) and extracted with EA (30 mL × 3). The combined organic phases were washed with saturated NaCl solution (20 mL), dried over anhydrous Na₂SO₄, filtered, and the filtrate was concentrated under reduced pressure. The residue was purified by column chromatography (DCM: MeOH = 10:1) to obtain Compound **5-1** (77.0 mg) as a yellow solid with a yield of 90.6%.

LC-MS (m/z): 367.0 [M+H]⁺.

### Step 2: Synthesis of (R)-3,5-dimethyl-2-(6-((1-methylpiperidin-3-yl)amino)-2H-pyrazolo[3,4-d]pyrimidin-2-yl)phenol (Target Compound 5)

Compound **5-1** (77.0 mg, 0.21 mmol) was added to a flask, followed by addition of ultra-dry DCM (3 mL). Under a nitrogen atmosphere, BBr₃ (1.1 mL, 2 M in DCM) was added at 0°C, and the mixture was stirred at 0°C for 1 hour. After completion of the reaction, the reaction mixture was quenched with an appropriate amount of MeOH in an ice-water bath, and the filtrate was concentrated under reduced pressure. The residue was purified by reverse-phase chromatography (ACN: 0.1% aqueous formic acid = 85%) to obtain **Target Compound 5** (10.2 mg) as a white solid with a yield of 13.8%.

LC-MS (m/z): 353.0 [M+H]⁺.

¹H NMR (400 MHz, DMSO-*d*₆) δ 10.20-9.65 (m, 1H), 9.05 (s, 1H), 8.34 (s, 1H), 7.11-6.93 (m, 1H), 6.69 (s, 1H), 6.65 (s, 1H), 4.09-3.92 (m, 1H), 3.04-2.87 (m, 1H), 2.79-2.62 (m, 1H), 2.27 (s, 3H), 2.24 (s, 3H), 2.06-1.96 (m, 2H), 1.93 (s, 3H), 1.88-1.80 (m, 1H), 1.76-1.67 (m, 1H), 1.62-1.48 (m, 1H), 1.41-1.27 (m, 1H).

### Example 7: Synthesis of (R)-5-cyclopropyl-3-methyl-2-(6-((1-methylpiperidin-3-yl)amino)-2H-pyrazolo[3,4-d]pyrimidin-2-yl)phenol (Target Compound 6)

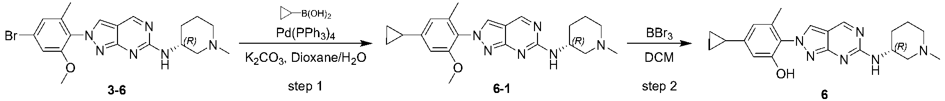

### Step 1: Synthesis of (R)-2-(4-cyclopropyl-2-methoxy-6-methylphenyl)-N-(1-methylpiperidin-3-yl)-2H-pyrazolo[3,4-d]pyrimidin-6-amine (6-1)

Compound **3-6** (90.0 mg, 0.21 mmol), cyclopropylboronic acid (53.7 mg, 0.63 mmol), and Pd(PPh₃)₄ (12.1 mg, 0.01 mmol) were weighed separately and added to dioxane (2.5 mL). Anhydrous K₂CO₃ (115.3 mg, 0.83 mmol) was then weighed and dissolved in water (1 mL), and the resulting solution was added to the reaction system, which was purged with nitrogen, heated to 90°C, and stirred for 16 hours. The heating was stopped, and the system was allowed to cool to room temperature. The reaction mixture was added with water (50 mL) and extracted with EA (40 mL). The organic phase was separated, dried over anhydrous Na₂SO₄, filtered, and concentrated under reduced pressure. The residue was purified by column chromatography (DCM: MeOH = 8:1) to obtain Compound **6-1** (70.0 mg) as a brown oil with a yield of 85.4%.

LC-MS (m/z): 393.0 [M+H]⁺.

### Step 2: Synthesis of (R)-5-cyclopropyl-3-methyl-2-(6-((1-methylpiperidin-3-yl)amino)-2H-pyrazolo[3,4-d]pyrimidin-2-yl)phenol (Target Compound 6)

Compound **6-1** (70.0 mg, 0.178 mmol) was dissolved in DCM (3 mL), followed by dropwise addition of a solution of BBr₃ in DCM (2 M, 0.5 mL), and the mixture was stirred at room temperature for 1 hour. The reaction mixture was quenched by dropwise addition of MeOH (2 mL), then subjected to rotary evaporation to remove the solvent, added with saturated NaHCO₃ aqueous solution (30 mL), and extracted with EA (70 mL). The organic phase was separated, dried over anhydrous Na₂SO₄, filtered, and concentrated under reduced pressure. The resulting crude brownish-yellow solid was purified by preparative HPLC and lyophilized to obtain **Target Compound 6** (10.2 mg) as a white solid with a yield of 15.1%.

LC-MS (m/z): 379.0 [M+H]⁺.

¹H NMR (400 MHz, DMSO-*d*₆) δ 9.88 (s, 1H), 9.04 (s, 1H), 8.33 (s, 1H), 7.02 (s, 1H), 6.58 (s, 1H), 6.54 (s, 1H), 4.09-3.89 (m, 1H), 2.96-2.89 (m, 1H), 2.73-2.62 (m, 1H), 2.23 (s, 3H), 1.93 (s, 3H), 1.92-1.81 (m, 4H), 1.78-1.66 (m, 1H), 1.63-1.47 (m, 1H), 1.43-1.28 (m, 1H), 1.06-0.89 (m, 2H), 0.77-0.58 (m, 2H).

### Example 8: Synthesis of (R)-5-ethynyl-3-methyl-2-(6-((1-methylpiperidin-3-yl)amino)-2H-pyrazolo[3,4-d]pyrimidin-2-yl)phenol (Target Compound 7)

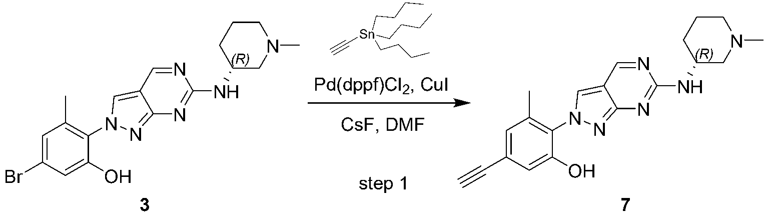

**Compound 3** (60.0 mg, 0.15 mmol), tributyl(ethynyl)stannane (181.2 mg, 0.57 mmol), CsF (43.7 mg, 0.29 mmol), CuI (5.5 mg, 0.03 mmol), and Pd(dppf)Cl₂ (10.5 mg, 0.01 mmol) were weighed separately and added to anhydrous DMF (2.5 mL). The reaction system was purged with nitrogen, sealed, heated to 120°C, and stirred for 16 hours. The heating was stopped, and the system was allowed to cool to room temperature. KF (300.0 mg) was dissolved in water (4 mL), and the resulting solution was added to the reaction system, which was then added with EA (15 mL) and stirred at room temperature for 2 hours. The reaction mixture was filtered to remove insoluble materials, and the filtrate was collected. The organic phase was separated, and the aqueous phase was extracted once with EA (30 mL). The combined organic phases were dried over anhydrous Na₂SO₄, filtered, and concentrated under reduced pressure. The resulting crude solid was purified by preparative HPLC and lyophilized to obtain **Target Compound 7** (5.5 mg) as a brownish-yellow solid with a yield of 10.5%.

LC-MS (m/z): 363.0 [M+H]⁺.

¹H NMR (400 MHz, DMSO-*d*₆) δ 10.36 (s, 1H), 9.06 (s, 1H), 8.41 (s, 1H), 7.15-7.01 (m, 1H), 6.97 (s, 2H), 4.27 (s, 1H), 4.02-3.90 (m, 1H), 2.96-2.84 (m, 1H), 2.65-2.58 (m, 1H), 2.18 (s, 3H), 1.98 (s, 3H), 1.95-1.76 (m, 3H), 1.73-1.64 (m, 1H), 1.61-1.46 (m, 1H), 1.39-1.26 (m, 1H).

### Example 9: Synthesis of (R)-3-methyl-2-(6-((1-methylpiperidin-3-yl)amino)-2H-pyrazolo[3,4-d]pyrimidin-2-yl)-5-(prop-1-yn-1-yl)phenol (Target Compound 8)

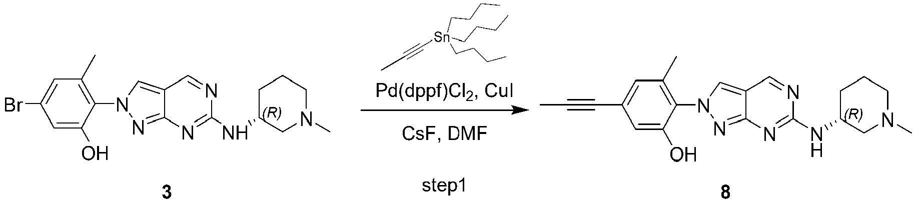

**Compound 3** (60.0 mg, 0.15 mmol) was dissolved in ultra-dry DMF (4 mL), followed by sequential addition of tributyl(prop-1-yn-1-yl)stannane (189.3 mg, 0.57 mmol), CsF (43.8 mg, 0.29 mmol), CuI (5.5 mg, 0.03 mmol), and Pd(dppf)Cl₂ (10.7 mg, 0.01 mmol), and the mixture was stirred at 120°C for 16 hours under a nitrogen atmosphere. After completion of the reaction, the mixture was added with an aqueous KF solution and stirred at room temperature for 2 hours. The reaction mixture was diluted with water (10 mL) and extracted with EA (30 mL × 3). The combined organic phases were washed with saturated NaCl solution (20 mL), dried over anhydrous Na₂SO₄, filtered, and the filtrate was concentrated under reduced pressure. The residue was purified by reverse-phase chromatography (ACN: 0.1% aqueous formic acid = 85%) to obtain **Target Compound 8** (19.9 mg) as a white solid with a yield of 36.9%.

LC-MS (m/z): 377.0 [M+H]⁺.

¹H NMR (400 MHz, DMSO-*d*₆) δ 10.60-9.99 (m, 1H), 9.07 (s, 1H), 8.41 (s, 1H), 7.26-7.07 (m, 1H), 6.90-6.85 (m, 2H), 4.10-3.98 (m, 1H), 3.13-2.96 (m, 1H), 2.86-2.75 (m, 1H), 2.33 (s, 3H), 2.24-2.09 (m, 2H), 2.06 (s, 3H), 1.95 (s, 3H), 1.90-1.81 (m, 1H), 1.80-1.70 (m, 1H), 1.69-1.49 (m, 1H), 1.46-1.31 (m, 1H).

### Example 10: Synthesis of (R)-3-methyl-2-(6-((1-methylpiperidin-3-yl)amino)-2H-pyrazolo[3,4-d]pyrimidin-2-yl)phenol (Target Compound 9)

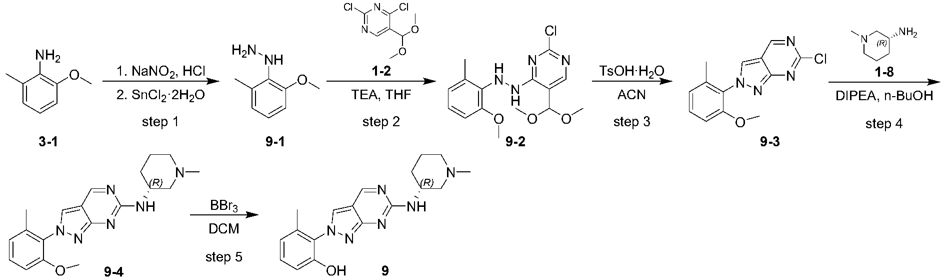

### Step 1: Synthesis of (2-methoxy-6-methylphenyl)hydrazine (9-1)

Compound **3-1** (1.37 g, 9.98 mmol) was weighed and dissolved in concentrated hydrochloric acid (6 mL) in an ice-water bath. NaNO₂ (1.03 g, 14.97 mmol) was then weighed and dissolved in water (6 mL), and the resulting solution was added dropwise to the reaction system, which was stirred in an ice-water bath for 25 minutes. SnCl₂·2H₂O (4.50 g, 19.96 mmol) was then weighed and dissolved in concentrated hydrochloric acid (5 mL), and the resulting solution was added dropwise to the reaction system, which was stirred in an ice-water bath for 2 hours. The reaction mixture was added with water (50 mL), followed by dropwise addition of sodium hydroxide aqueous solution (4 M, 35 mL) to adjust the pH to 9. The mixture was extracted with DCM (80 mL). The organic phase was separated, washed once with water (70 mL), dried over anhydrous Na₂SO₄, filtered, and concentrated under reduced pressure to obtain crude Compound **9-1** (1.16 g) as a red oil.

LC-MS (m/z): 153.0 [M+H]⁺.

### Step 2: Synthesis of 2-chloro-5-(dimethoxymethyl)-4-(2-(2-methoxy-6-methylphenyl)hydrazinyl)pyrimidine (9-2)

Crude Compound **9-1** (300.0 mg, 1.97 mmol), Compound **1-2** (439.6 mg, 1.97 mmol), and TEA (598.3 mg, 5.91 mmol) were weighed separately and dissolved in anhydrous THF (6 mL), and the mixture was stirred at room temperature overnight. The reaction mixture was added with water (70 mL) and extracted with EA (80 mL). The organic phase was separated, washed once with water (50 mL), dried over anhydrous Na₂SO₄, filtered, and concentrated under reduced pressure. The residue was purified by column chromatography (PE: EA = 5:1) to obtain Compound **9-2** (420.0 mg) as a red oil with a yield of 62.9%.

LC-MS (m/z): 339.0 [M+H]⁺.

### Step 3: Synthesis of 6-chloro-2-(2-methoxy-6-methylphenyl)-2H-pyrazolo[3,4-d]pyrimidine (9-3)

Compound **9-2** (420.0 mg, 1.24 mmol) and TsOH·H₂O (259.4 mg, 1.36 mmol) were weighed separately and added to ACN (6 mL), and the mixture was heated to 70°C and stirred for 2 hours. The reaction mixture was added with water (50 mL) and extracted with EA (50 mL). The organic phase was separated, dried over anhydrous Na₂SO₄, filtered, and concentrated under reduced pressure. The residue was purified by column chromatography (PE: EA = 1:1) to obtain Compound **9-3** (175.0 mg) as a white solid with a yield of 51.4%.

LC-MS (m/z): 275.0 [M+H]⁺.

¹H-NMR (400 MHz, DMSO-*d*₆) δ 9.55 (s, 1H), 9.05 (s, 1H), 7.51 (t, *J =* 8.1 Hz, 1H), 7.17 (d, *J =* 8.4 Hz, 1H), 7.07 (d, *J =* 7.7 Hz, 1H), 3.73 (s, 3H), 1.97 (s, 3H).

### Step 4: Synthesis of (R)-2-(2-methoxy-6-methylphenyl)-N-(1-methylpiperidin-3-yl)-2H-pyrazolo[3,4-d]pyrimidin-6-amine (9-4)

Compound **9-3** (171.0 mg, 0.62 mmol), Compound **1-8** (23.6 mg, 0.21 mmol) (85.3 mg, 0.75 mmol), and DIPEA (321.8 mg, 2.49 mmol) were weighed separately and added to *n-*BuOH (3.5 mL). The reaction system was purged with nitrogen, heated to 100°C, and stirred for 2 hours. The heating was stopped, and the system was allowed to cool to room temperature. The reaction mixture was concentrated under reduced pressure to obtain a crude product, which was purified by column chromatography (DCM: CH₃OH = 8:1) to obtain Compound **9-4** (270.0 mg) as a white solid.

LC-MS (m/z): 353.0 [M+H]⁺.

### Step 5: Synthesis of (R)-3-methyl-2-(6-((1-methylpiperidin-3-yl)amino)-2H-pyrazolo[3,4-d]pyrimidin-2-yl)phenol (Target Compound 9)

Crude Compound **9-4** (270.0 mg) was dissolved in DCM (6 mL), followed by dropwise addition of a solution of BBr₃ in DCM (2 M, 1.5 mL) at room temperature, and the mixture was stirred at room temperature for 2 hours. The reaction mixture was quenched by dropwise addition of MeOH (5 mL) in an ice-water bath, and concentrated under reduced pressure. The mixture was added with saturated NaHCO₃ aqueous solution (30 mL) and extracted with DCM/CH₃OH (8:1, 50 mL). The organic phase was separated, dried over anhydrous Na₂SO₄, filtered, and concentrated under reduced pressure. The residue was purified by column chromatography (DCM: CH₃OH = 8:1) to obtain **Target Compound 9** (116.0 mg) as a brownish-yellow solid with a two-step yield of 55.3%.

LC-MS (m/z): 339.0 [M+H]⁺.

¹H-NMR (400 MHz, DMSO-*d*₆) δ 10.00 (s, 1H), 9.06 (s, 1H), 8.39 (s, 1H), 7.23 (t, *J* = 8.0 Hz, 1H), 7.06 (s, 1H), 6.90 (d, *J =* 8.0, 1H), 6.82 (d, *J =* 8.0 Hz, 1H), 4.07-3.93 (m, 1H), 3.04-2.93 (m, 1H), 2.78-2.63 (m, 1H), 2.26 (s, 3H), 2.09-1.99 (m, 5H), 1.90-1.79 (m, 1H), 1.77-1.67 (m, 1H), 1.64-1.47 (m, 1H), 1.43-1.28 (m, 1H).

### Example 11: Synthesis of (R)-5-fluoro-3-methyl-2-(6-((1-methylpiperidin-3-yl)amino)-2H-pyrazolo[3,4-d]pyrimidin-2-yl)phenol (Target Compound 10)

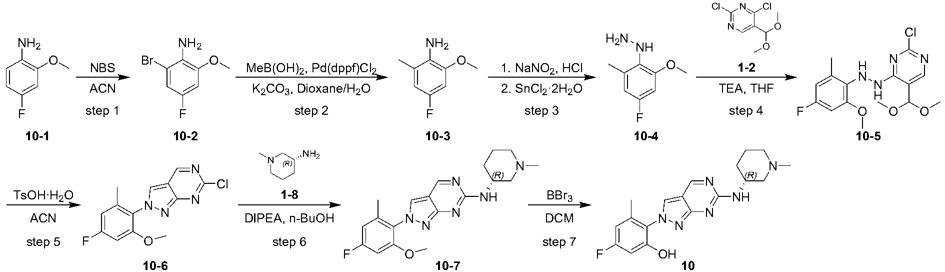

### Step 1: Synthesis of 2-bromo-4-fluoro-6-methoxyaniline (10-2)

Compound **10-1** (1.20 g, 8.50 mmol) was weighed and dissolved in ACN (10 mL). The reaction system was purged with nitrogen and cooled in an ice-water bath. NBS (1.58 g, 8.93 mmol) was weighed and added portionwise to the reaction system, which was stirred in an ice-water bath for 1 hour. The reaction mixture was added with saturated NaHCO₃ aqueous solution (20 mL), followed by addition of water (50 mL), and stirred thoroughly. The mixture was extracted with EA (60 mL). The organic phase was separated, dried over anhydrous Na₂SO₄, filtered, and concentrated under reduced pressure. The residue was purified by column chromatography (PE: EA = 5:1) to obtain Compound **10-2** (930.0 mg) as a yellow oil with a yield of 49.7%.

LC-MS (m/z): 220.0/222.0 [M+H]⁺.

### Step 2: Synthesis of 4-fluoro-2-methoxy-6-methylaniline (10-3)

Compound **10-2** (930.0 mg, 4.23 mmol), MeB(OH)₂ (505.9 mg, 8.45 mmol), and Pd(dppf)Cl₂ (175.0 mg, 0.24 mmol) were weighed separately and added to anhydrous dioxane (8 mL). Anhydrous K₂CO₃ (1.75 g, 12.68 mmol) was then weighed and dissolved in water (5 mL), and the resulting solution was added to the reaction system, which was purged with nitrogen, heated to 100°C, and stirred for 16 hours. The heating was stopped, and the system was allowed to cool to room temperature. The reaction mixture was added with water (50 mL) and extracted with EA (50 mL). The organic phase was separated, dried over anhydrous Na₂SO₄, filtered, and concentrated under reduced pressure. The residue was purified by column chromatography (PE: EA = 5:1) to obtain Compound **10-3** (463.0 mg) as a pale-yellow solid with a yield of 70.6%.

LC-MS (m/z): 156.0 [M+H]⁺.

¹H-NMR (400 MHz, DMSO-*d*₆) δ 6.61 (dd, *J* = 12.0 Hz, 2.8 Hz, 1H), 6.45 (dd, *J* = 12.0 Hz, 2.8 Hz, 1H), 4.24 (s, 2H), 3.76 (s, 3H), 2.07 (s, 3H).

### Step 3: Synthesis of (4-fluoro-2-methoxy-6-methylphenyl)hydrazine (10-4)

Compound **10-3** (460.0 mg, 2.96 mmol) was weighed and dissolved in concentrated hydrochloric acid (3 mL) in an ice-water bath. NaNO₂ (306.8 mg, 4.45 mmol) was then weighed and dissolved in water (2 mL), and the resulting solution was added dropwise to the reaction system, which was stirred in an ice-water bath for 10 minutes. SnCl₂·2H₂O (1.34 g, 5.93 mmol) was then weighed and dissolved in concentrated hydrochloric acid (5 mL), and the resulting solution was added dropwise to the reaction system, which was stirred in an ice-water bath for 2 hours. The reaction mixture was added with water (20 mL), followed by dropwise addition of sodium hydroxide aqueous solution (0.5 M, 10 mL) to adjust the pH to 9. The mixture was extracted with DCM (50 mL). The organic phase was separated, washed once with water (30 mL), dried over anhydrous Na₂SO₄, filtered, and concentrated under reduced pressure to obtain crude Compound **10-4** (300 mg) as a red oil.

LC-MS (m/z): 171.0 [M+H]⁺.

### Step 4: Synthesis of 2-chloro-5-(dimethoxymethyl)-4-(2-(4-fluoro-2-methoxy-6-methylphenyl)hydrazinyl)pyrimidine (10-5)

Compound **10-4** (300 mg), Compound **1-2** (393.2 mg, 1.76 mmol), and TEA (535.1 mg, 5.29 mmol) were weighed and dissolved in anhydrous tetrahydrofuran (6 mL), and the mixture was stirred at room temperature for 16 hours. The reaction mixture was added with water (50 mL) and extracted with EA (50 mL). The organic phase was separated, washed once with water (30 mL), dried over anhydrous Na₂SO₄, filtered, and concentrated under reduced pressure. The residue was purified by column chromatography (PE: EA = 5:1) to obtain Compound **10-5** (360.0 mg) as a red solid with a yield of 57.2%.

LC-MS (m/z): 357.0 [M+H]⁺.

### Step 5: Synthesis of 6-chloro-2-(4-fluoro-2-methoxy-6-methylphenyl)-2H-pyrazolo[3,4-d]pyrimidine (10-6)

Compound **10-5** (360.0 mg, 1.01 mmol) and TsOH·H₂O (211.1 mg, 1.11 mmol) were weighed separately and added to ACN (6 mL), and the mixture was heated to 70°C and stirred for 2 hours. The heating was stopped, and the system was allowed to cool to room temperature. The reaction mixture was added with water (70 mL) and extracted with EA (60 mL). The organic phase was separated, dried over anhydrous Na₂SO₄, filtered, and concentrated under reduced pressure. The residue was purified by column chromatography (PE: EA = 1:1) to obtain Compound **10-6** (134.0 mg) as a white solid with a yield of 45.3%.

LC-MS (m/z): 293.0 [M+H]⁺.

¹H-NMR (400 MHz, DMSO-*d*₆) δ 9.55 (s, 1H), 9.04 (s, 1H), 7.13 (dd, *J* = 12.0, 2.4 Hz, 1H), 6.97 (dd, *J =* 8.0, 2.8 Hz, 1H), 3.75 (s, 3H), 1.97 (s, 3H).

### Step 6: Synthesis of (R)-2-(4-fluoro-2-methoxy-6-methylphenyl)-N-(1-methylpiperidin-3-yl)-2H-pyrazolo[3,4-d]pyrimidin-6-amine (10-7)

Compound **10-6** (134.0 mg, 0.46 mmol), Compound **1-8** (62.7 mg, 0.55 mmol), and DIPEA (236.7 mg, 1.83 mmol) were weighed separately and added to n-BuOH (3.5 mL). The reaction system was purged with nitrogen, heated to 100°C, and stirred for 2 hours. The heating was stopped, and the system was allowed to cool to room temperature. The reaction mixture was directly subjected to rotary evaporation to remove the solvent. The resulting crude product was purified by silica gel column chromatography (DCM: MeOH = 8:1) to obtain crude Compound **10-7** (220.0 mg).

LC-MS (m/z): 371.0 [M+H]⁺.

### Step 7: Synthesis of (R)-5-fluoro-3-methyl-2-(6-((1-methylpiperidin-3-yl)amino)-2H-pyrazolo[3,4-d]pyrimidin-2-yl)phenol (Target Compound 10)

Crude Compound **10-7** (220.0 mg) was dissolved in DCM (3 mL), followed by dropwise addition of a solution of BBr₃ in DCM (2 M, 2 mL) at room temperature, and the mixture was stirred at room temperature for 1 hour. The reaction mixture was quenched by dropwise addition of MeOH (4 mL) in an ice-water bath, then subjected to rotary evaporation to remove the solvent, added with saturated NaHCO₃ aqueous solution (30 mL), and extracted with DCM/MeOH (8:1, 50 mL). The organic phase was separated, dried over anhydrous Na₂SO₄, filtered, and concentrated under reduced pressure. The residue was purified by column chromatography (DCM: MeOH = 8:1) to obtain **Target Compound 10** (93.9 mg) as an off-white solid with a two-step yield of 44.3%.

LC-MS (m/z): 357.0 [M+H]⁺.

¹H-NMR (400 MHz, DMSO-*d*₆) δ 10.54 (s, 1H), 9.06 (s, 1H), 8.39 (s, 1H), 7.06 (s, 1H), 6.81-6.54 (m, 2H), 4.04-3.90 (m, 1H), 2.98-2.89 (m, 1H), 2.71-2.61 (m, 1H), 2.21 (s, 3H), 1.97 (s, 3H), 2.05-1.77 (m, 3H), 1.76-1.65 (m, 1H), 1.60-1.48 (m, 1H), 1.40-1.27 (m, 1H).

### Example 12: Synthesis of (R)-2-(4-chloro-2,6-dimethylphenyl)-N-(1-methylpiperidin-3-yl)-2H-pyrazolo[3,4-d]pyrimidin-6-amine (Target Compound 67)

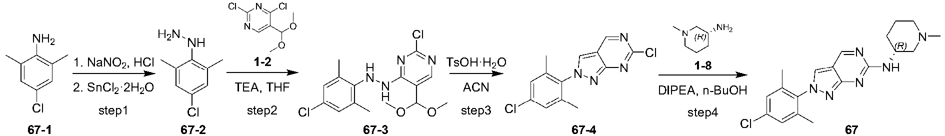

With reference to Steps 2 to 5 of Example 1, Compound **67-1** was used in place of Compound **1-4** in Step 2. The remaining intermediate materials and preparation procedures were identical to those described in Example 1. Using Compound **67-1** (2.00 g) as the starting material, Target Compound **67** (395.1 mg) was obtained as a white solid with a four-step yield of 8.5%.

LC-MS (m/z): 371.0 [M+H]⁺.

¹H NMR (400 MHz, DMSO-*d*₆) δ 9.12 (s, 1H), 8.53 (s, 1H), 7.40 (s, 2H), 7.31-7.20 (m, 1H), 4.23-3.86 (m, 1H), 3.14-2.98 (m, 1H), 2.86-2.75 (m, 1H), 2.35 (s, 3H), 2.23-2.07 (m, 2H), 1.96 (s, 6H), 1.91-1.82 (m, 1H), 1.80-1.71 (m, 1H), 1.66-1.51 (m, 1H), 1.46-1.31 (m, 1H).

### Example 13: Synthesis of (R)-5-chloro-3-methyl-2-(6-((1-methylpiperidin-3-yl)oxy)-2H-pyrazolo[3,4-d]pyrimidin-2-yl)phenol (Target Compound 48)

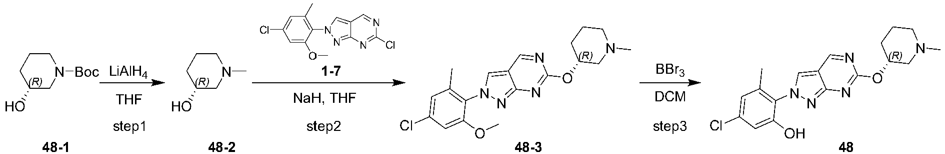

### Step 1: Synthesis of (R)-1-methylpiperidin-3-ol (48-2)

Compound **48-1** (1.01 g, 5.00 mmol) was dissolved in anhydrous THF (10 mL), followed by addition of LiAlH₄ (380.0 mg, 10.00 mmol) in an ice-water bath, and the mixture was stirred at 40°C for 3 hours. The reaction mixture was quenched with saturated Na₂CO₃ aqueous solution until gas evolution ceased. After filtration, the filter cake was washed with MeOH, and the filtrate was concentrated to obtain Compound **48-2** (357.0 mg) with a yield of 62.0%.

LC-MS (m/z): 116.0 [M+H]⁺.

### Step 2: Synthesis of (R)-2-(4-chloro-2-methoxy-6-methylphenyl)-6-((1-methylpiperidin-3-yl)oxy)-2H-pyrazolo[3,4-d]pyrimidine (48-3)

Compound **48-2** (73.7 mg, 0.64 mmol) was dissolved in anhydrous THF (3 mL), followed by addition of NaH (38.4 mg, 60% w/w, 0.96 mmol) in an ice-water bath, and the mixture was stirred at the same temperature for 0.5 hours. Compound **1-7** (100.0 mg, 0.32 mmol) was then added, and the mixture was immediately stirred at 50°C for 0.5 hours. The reaction mixture was cooled to room temperature, then quenched with saturated NH₄Cl aqueous solution, diluted with water (20 mL), and extracted with EA (20 mL × 2). The combined organic phases were washed with saturated brine (20 mL), filtered, and concentrated. The residue was purified by column chromatography (MeOH: DCM: TEA = 15:84:1) to obtain **48-3** (30.0 mg) with a yield of 24.2%.

LC-MS (m/z): 388.0 [M+H]⁺.

### Step 3: Synthesis of (R)-5-chloro-3-methyl-2-(6-((1-methylpiperidin-3-yl)oxy)-2H-pyrazolo[3,4-d]pyrimidin-2-yl)phenol (Target Compound 48)

Compound **48-3** (30.0 mg, 0.08 mmol) was dissolved in DCM (2 mL), followed by addition of a solution of BBr₃ in DCM (2.0 M, 0.5 mL) in an ice-water bath, and the mixture was stirred at room temperature for 2 hours. The reaction mixture was quenched with MeOH in an ice-water bath and concentrated. The residue was purified twice by thin-layer chromatography (MeOH: DCM = 1:10) to obtain **Target Compound 48** (4.6 mg) with a yield of 15.9%.

LC-MS (m/z): 374.0 [M+H]⁺.

¹H NMR (400 MHz, DMSO-*d*₆) δ 10.70 (s, 1H), 9.36 (s, 1H), 8.73 (s, 1H), 6.97 (d, *J* = 2.2 Hz, 1H), 6.94 (d, *J* = 2.2 Hz, 1H), 5.10 (tt, *J =* 8.4, 4.0 Hz, 1H), 3.00-2.90 (m, 1H), 2.60-2.52 (m, 1H), 2.23-2.10 (m, 4H), 2.11-1.98 (m, 2H), 1.96 (s, 3H), 1.83-1.71 (m, 1H), 1.65-1.37 (m, 2H).

### Example 14: Synthesis of (R)-2-(4-chloro-2-(difluoromethoxy)-6-methylphenyl)-N-(1-methylpiperidin-3-yl)-2H-pyrazolo[3,4-d]pyrimidin-6-amine (Target Compound 60)

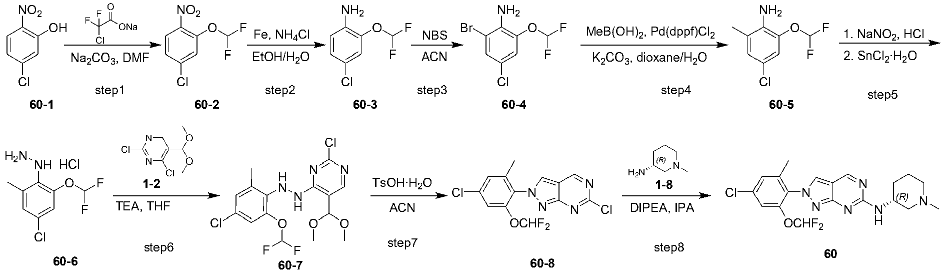

### Step 1: Synthesis of 4-chloro-2-(difluoromethoxy)-1-nitrobenzene (60-2)

Compound **60-1** (2.00 g, 11.53 mmol), anhydrous Na₂CO₃ (1.83 g, 17.29 mmol), and sodium chlorodifluoroacetate (3.52 g, 23.05 mmol) were dissolved in anhydrous DMF (20 mL), and the mixture was stirred at 100°C for 5 hours under a nitrogen atmosphere. The reaction mixture was cooled to room temperature, then added with dilute hydrochloric acid to adjust the pH to less than 5, diluted with water (30 mL), and extracted with EA (30 mL × 2). The combined organic phases were washed with water (30 mL × 3) and saturated brine (30 mL), dried over anhydrous Na₂SO₄, filtered, and concentrated. The residue was purified by column chromatography (PE: EA = 70:30) to obtain Compound **60-2** (2.18 g) with a yield of 84.6%.

### Step 2: Synthesis of 4-chloro-2-(difluoromethoxy)aniline (60-3)

Compound **60-2** (2.18 g, 9.75 mmol) was dissolved in EtOH (20 mL), followed by addition of an aqueous solution (10 mL) of NH₄Cl (4.70 g, 87.78 mmol) and iron powder (4.92 g, 87.78 mmol), and the mixture was stirred at 80°C for 1 hour. The reaction mixture was cooled to room temperature and filtered. The filtrate was diluted with EA (50 mL), washed with water (20 mL × 2) and saturated brine (20 mL), dried over anhydrous Na₂SO₄, filtered, and concentrated. The residue was purified by column chromatography (PE: EA = 65:35) to obtain crude Compound **60-3** (1.94 g).

LC-MS (m/z): 194.0 [M+H]⁺.

### Step 3: Synthesis of 2-bromo-4-chloro-6-(difluoromethoxy)aniline (60-4)

Crude Compound **60-3** (1.94 g) was dissolved in ACN (20 mL), and NBS (1.91 g, 10.72 mmol) was slowly added in an ice-water bath. After completion of the addition, the mixture was stirred at the same temperature for 0.5 hours. The reaction mixture was diluted with EA (30 mL), washed sequentially with saturated NaHCO₃ aqueous solution (20 mL), water (20 mL), and saturated brine (20 mL), dried over anhydrous Na₂SO₄, filtered, and concentrated. The residue was purified by column chromatography (PE: EA = 95:5) to obtain Compound **60-4** (2.10 g) with a two-step yield of 79.1%.

LC-MS (m/z): 274.0 [M+H]⁺.

### Step 4: Synthesis of 4-chloro-2-(difluoromethoxy)-6-methylaniline (60-5)

Compound **60-4** (2.10 g, 7.71 mmol), methylboronic acid (924.8 mg, 15.41 mmol), Pd(dppf)Cl₂ (281.9 mg, 0.39 mmol), and anhydrous K₂CO₃ (2.66 g, 19.27 mmol) were added to a mixed solvent of dioxane (30 mL) and water (3 mL), and the mixture was stirred at 100°C for 16 hours under a nitrogen atmosphere. The reaction mixture was cooled to room temperature, filtered, and concentrated. The residue was purified by column chromatography (PE: EA = 90:10) to obtain Compound **60-5** (1.23 g) with a yield of 76.8%.

LC-MS (m/z): 208.0 [M+H]⁺.

### Step 5: Synthesis of (4-chloro-2-(difluoromethoxy)-6-methylphenyl)hydrazine hydrochloride (60-6)

Compound **60-5** (1.23 g, 5.95 mmol) was dissolved in concentrated hydrochloric acid (6 mL), followed by dropwise addition of an aqueous solution (6 mL) of NaNO₂ (616.2 mg, 8.93 mmol) at 0°C, and the mixture was stirred at the same temperature for 1 hour. A mixture of SnCl₂·2H₂O (2.69 g, 11.91 mmol) and concentrated hydrochloric acid (6 mL) was then added dropwise at 5°C. After completion of the addition, the mixture was gradually warmed to room temperature and stirred for 1 hour. After filtration, the filter cake was washed with a small amount of water and EA, and dried to obtain crude Compound **60-6** (704.0 mg), which was directly used in the next step.

### Step 6: Synthesis of 2-chloro-4-(2-(4-chloro-2-(difluoromethoxy)-6-methylphenyl)hydrazinyl)-5-(dimethoxymethyl)pyrimidine (60-7)

Crude Compound **60-6** (704.0 mg) and 2,4-dichloro-5-(dimethoxymethyl)pyrimidine (606.2 mg, 2.72 mmol) were added to THF (10 mL), followed by addition of TEA (1.88 mL, 13.59 mmol), and the mixture was stirred at room temperature for 16 hours. The reaction mixture was filtered. The filter cake was washed with EA, and the filtrate was concentrated. The residue was purified by column chromatography (PE: EA = 65:35) to obtain Compound **60-7** (497.0 mg) with a two-step yield of 24.6%.

LC-MS (m/z): 409.0 [M+H]⁺.

### Step 7: Synthesis of 6-chloro-2-(4-chloro-2-(difluoromethoxy)-6-methylphenyl)-2H-pyrazolo[3,4-d]pyrimidine (60-8)

Compound **60-7** (497.0 mg, 1.21 mmol) and TsOH·H₂O (231.0 mg, 1.21 mmol) were dissolved in ACN (5 mL), and the mixture was stirred at 70°C for 0.5 hours. The reaction mixture was cooled to room temperature and concentrated. The residue was purified by column chromatography (PE: EA: TEA = 65:34:1) to obtain Compound **60-8** (302.0 mg) with a yield of 72.3%.

LC-MS (m/z): 345.0 [M+H]⁺.

### Step 8: Synthesis of (R)-2-(4-chloro-2-(difluoromethoxy)-6-methylphenyl)-N-(1-methylpiperidin-3-yl)-2H-pyrazolo[3,4-d]pyrimidin-6-amine (Target Compound 60)

Compound **60-8** (118.0 mg, 0.34 mmol), Compound **1-8** (46.9 mg, 0.41 mmol), and DIPEA (0.12 mL, 0.68 mmol) were added to IPA (2 mL), and the mixture was stirred at 80°C for 0.5 hours. The reaction mixture was cooled to room temperature and concentrated. The residue was purified by column chromatography (MeOH: DCM = 3:97), and further purified by reverse-phase preparative chromatography (ACN: 0.1% aqueous formic acid = 85%) to obtain **Target Compound 60** (98.4 mg) with a yield of 68.0%.

LC-MS (m/z): 423.0 [M+H]⁺.

¹H NMR (400 MHz, DMSO-*d*₆) δ 9.14 (s, 1H), 8.53 (s, 1H), 7.54-7.48 (m, 1H), 7.48-7.44 (m, 1H), 7.31-7.03 (m, 2H), 4.08-3.96 (m, 1H), 3.09-2.97 (m, 1H), 2.81-2.66 (m, 1H), 2.30 (s, 3H), 2.16-1.97 (m, 5H), 1.91-1.80 (m, 1H), 1.80-1.70 (m, 1H), 1.65-1.50 (m, 1H), 1.45-1.26 (m, 1H).

### Example 15: Synthesis of (R)-5-chloro-3-methyl-2-(6-((1-methylpyrrolidin-3-yl)amino)-2H-pyrazolo[3,4-d]pyrimidin-2-yl)phenol (Target Compound 61)

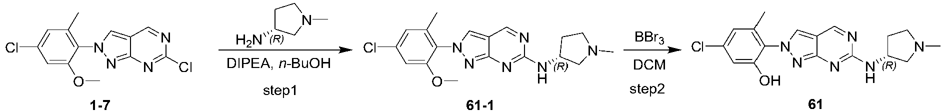

### Step 1: Synthesis of (R)-2-(4-chloro-2-methoxy-6-methylphenyl)-N-(1-methylpyrrolidin-3-yl)-2H-pyrazolo[3,4-d]pyrimidin-6-amine (61-1)

Compound **1-7** (221.0 mg, 0.72 mmol) was dissolved in n-BuOH (2 mL), followed by sequential addition of (*R*)-1-methylpyrrolidin-3-amine (65.0 mg, 0.65 mmol) and DIPEA (420.0 mg, 3.75 mmol), and the mixture was stirred at 100°C for 1 hour under microwave irradiation. The reaction mixture was cooled to room temperature and concentrated under reduced pressure. The residue was purified by column chromatography (DCM: MeOH = 10:1) to obtain Compound **61-1** (181.0 mg) with a yield of 75.1%.

LC-MS (m/z): 373.0 [M+H]⁺.

### Step 2: Synthesis of (R)-5-chloro-3-methyl-2-(6-((1-methylpyrrolidin-3-yl)amino)-2H-pyrazolo[3,4-d]pyrimidin-2-yl)phenol (Target Compound 61)

Compound **61-1** (181.0 mg, 0.49 mmol) was added to a flask, followed by addition of ultra-dry DCM (5 mL). A solution of BBr₃ in DCM (2.5 mL, 2.0 M) was added in an ice-water bath and under a nitrogen atmosphere, and the mixture was stirred at the same temperature for 1 hour. The reaction mixture was quenched with an appropriate amount of MeOH and concentrated under reduced pressure. The residue was purified by reverse-phase preparative chromatography (ACN: 10 mmol/L NH₄HCO₃ aqueous solution = 95%) to obtain **Target Compound 61** (75.9 mg) with a yield of 43.6%.

LC-MS (m/z): 359.0 [M+H]⁺.

¹H NMR (400 MHz, DMSO-*d*₆) δ 9.08 (s, 1H), 8.41 (s, 1H), 7.54-7.36 (m, 1H), 6.98-6.88 (m, 2H), 4.49-4.35 (m, 1H), 3.07-2.94 (m, 1H), 2.84-2.71 (m, 1H), 2.71-2.59 (m, 2H), 2.40 (s, 3H), 2.30-2.15 (m, 1H), 1.97 (s, 3H), 1.92-1.75 (m, 1H).

### Example 16: Synthesis of 5-chloro-3-methyl-2-(6-((1-methylazetidin-3-yl)amino)-2H-pyrazolo[3,4-d]pyrimidin-2-yl)phenol (Target Compound 62)

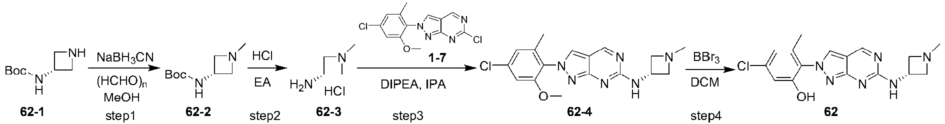

### Step 1: Synthesis of tert-butyl (1-methylazetidin-3-yl)carbamate (62-2)

Compound **62-1** (300.0 mg, 1.74 mmol) was dissolved in MeOH (6 mL), followed by sequential addition of paraformaldehyde (261.9 mg, 8.72 mmol) and one drop of AcOH. Sodium cyanoborohydride (328.4 mg, 5.23 mmol) was added in an ice-water bath, and the mixture was stirred at 35°C for 3 hours. The reaction mixture was diluted with water (10 mL) and extracted with EA (20 mL × 3). The combined organic phases were washed with saturated NaHCO₃ solution (20 mL) and saturated brine (20 mL), dried over anhydrous Na₂SO₄, filtered, and concentrated under reduced pressure to obtain crude Compound 62-2 (285.0 mg), which was directly used in the next step.

LC-MS (m/z): 187.0 [M+H]⁺.

### Step 2: Synthesis of 1-methylazetidin-3-amine hydrochloride (62-3)

Crude Compound **62-2** (285.0 mg) was dissolved in EA (5 mL), followed by addition of a solution of HCl in EA (5 mL, 4.0 M), and the mixture was stirred at room temperature for 2 hours. After completion of the reaction, the reaction mixture was concentrated under reduced pressure to obtain Compound **62-3** (131.0 mg) with a two-step yield of 61.3%.

### Step 3: Synthesis of 2-(4-chloro-2-methoxy-6-methylphenyl)-N-(1-methylazetidin-3-yl)-2H-pyrazolo[3,4-d]pyrimidin-6-amine (62-4)

Compound **62-3** (131.0 mg, 1.07 mmol) was dissolved in IPA (6 mL), followed by sequential addition of Compound **1-7** (150.0 mg, 0.49 mmol) and DIPEA (984.0 mg, 7.61 mmol), and the mixture was stirred at 80°C for 1 hour. The reaction mixture was cooled to room temperature and concentrated under reduced pressure. The residue was purified by column chromatography (DCM: MeOH = 10:1) to obtain Compound **62-4** (259.0 mg) with a yield of 67.5%.

LC-MS (m/z): 359.0 [M+H]⁺.

### Step 4: Synthesis of 5-chloro-3-methyl-2-(6-((1-methylazetidin-3-yl)amino)-2H-pyrazolo[3,4-d]pyrimidin-2-yl)phenol (Target Compound 62)

Compound **62-4** (259.0 mg, 0.72 mmol) and anhydrous DCM (5 mL) were added to a flask, followed by addition of a solution of BBr₃ in DCM (3.7 mL, 2.0 M) in an ice-water bath and under a nitrogen atmosphere, and the mixture was stirred for 1 hour. The reaction mixture was quenched with an appropriate amount of MeOH in an ice-water bath and concentrated under reduced pressure. The residue was purified by reverse-phase preparative chromatography (ACN: 0.1% aqueous formic acid = 85%) to obtain **Target Compound 62** (4.8 mg) with a yield of 1.9%.

LC-MS (m/z): 345.0 [M+H]⁺.

¹H NMR (400 MHz, DMSO-*d*₆) δ 9.16 (s, 1H), 8.50 (s, 1H), 7.86-7.76 (m, 1H), 7.04-6.95 (m, 2H), 4.63-4.49 (m, 1H), 3.84 (t, *J=* 8.0 Hz, 2H), 3.24 (t, *J= 7.2* Hz, 2H), 2.45 (s, 3H), 2.02 (s, 3H).

### Example 17: Synthesis of (S)-5-chloro-2-(6-(((1-ethylpyrrolidin-2-yl)methyl)amino)-2H-pyrazolo[3,4-d]pyrimidin-2-yl)-3-methylphenol (Target Compound 63)

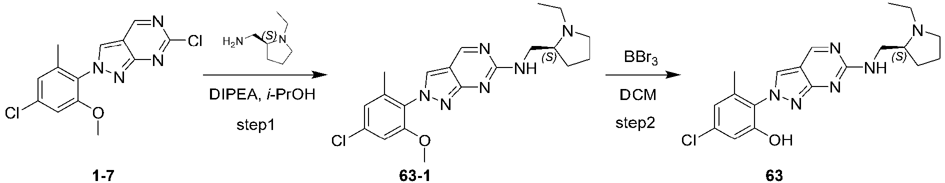

### Step 1: Synthesis of (S)-2-(4-chloro-2-methoxy-6-methylphenyl)-N-((1-ethylpyrrolidin-2-yl)methyl)-2H-pyrazolo[3,4-d]pyrimidin-6-amine (63-1)

Compound **1-7** (100.0 mg, 0.32 mmol), (S)-(1-ethylpyrrolidin-2-yl)methanamine (50.0 mg, 0.39 mmol), and DIPEA (167.2 mg, 1.30 mmol) were dissolved in IPA (3 mL), and the mixture was stirred at 80°C for 3 hours under a nitrogen atmosphere. The reaction mixture was cooled and concentrated. The residue was purified by column chromatography (DCM: MeOH = 85:15) to obtain Compound **63-1** (99.0 mg) with a yield of 76.3%.

LC-MS (m/z): 401.0 [M+H]⁺.

### Step 2: Synthesis of (S)-5-chloro-2-(6-(((1-ethylpyrrolidin-2-yl)methyl)amino)-2H-pyrazolo[3,4-d]pyrimidin-2-yl)-3-methylphenol (Target Compound 63)

Compound **63-1** (99.0 mg, 0.25 mmol) was dissolved in DCM (2 mL), followed by addition of a solution of BBr₃ in DCM (0.5 M, 2 mL) in an ice-water bath, and the mixture was stirred at room temperature for 1.5 hours. The reaction mixture was quenched by dropwise addition of MeOH (2 mL) and concentrated. The residue was purified by column chromatography (DCM: MeOH = 85:15) to obtain **Target Compound 63** (44.8 mg) with a yield of 45.0%.

LC-MS (m/z): 387.0 [M+H]⁺.

¹H NMR (400 MHz, DMSO-*d*₆) δ 9.08 (s, 1H), 8.43 (s, 1H), 7.26 (s, 1H), 6.99-6.90 (m, 2H), 3.63-3.53 (m, 1H), 3.36-3.17 (m, 2H), 3.16-2.93 (m, 2H), 2.59-2.53 (m, 1H), 2.47-2.39 (m, 1H), 2.05-1.86 (m, 4H), 1.82-1.63 (m, 3H), 1.14 *(t, J=* 7.2 Hz, 3H).

### Example 18: Synthesis of 5-chloro-2-(6-(((1R,2R)-2-hydroxycyclohexyl)amino)-2H-pyrazolo[3,4-d]pyrimidin-2-yl)-3-methylphenol (Target Compound 64)

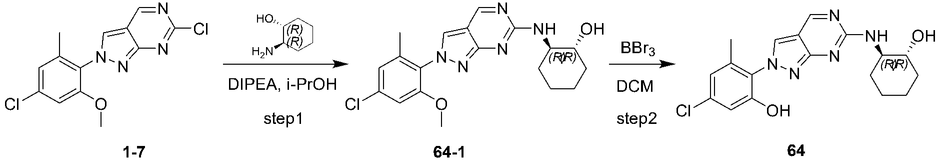

### Step 1: Synthesis of (1R,2R)-2-((2-(4-chloro-2-methoxy-6-methylphenyl)-2H-pyrazolo[3,4-d]pyrimidin-6-yl)amino)cyclohexan-1-ol (64-1)

Compound **1-7** (100.0 mg, 0.32 mmol), (1*R*,2*R*)-2-aminocyclohexan-1-ol (44.7 mg, 0.39 mmol), and DIPEA (167.2 mg, 1.30 mmol) were dissolved in IPA (3 mL), and the mixture was stirred at 80°C for 3 hours under a nitrogen atmosphere. The heating was stopped, and the system was allowed to cool to room temperature. The reaction mixture was concentrated. The residue was purified by column chromatography (DCM: MeOH = 85:15) to obtain Compound **64-1** (70.0 mg) with a yield of 55.8%.

LC-MS (m/z): 388.0 [M+H]⁺.

### Step 2: Synthesis of 5-chloro-2-(6-(((1R,2R)-2-hydroxycyclohexyl)amino)-2H-pyrazolo[3,4-d]pyrimidin-2-yl)-3-methylphenol (Target Compound 64)

Compound **64-1** (70.0 mg, 0.18 mmol) was dissolved in DCM (3 mL), followed by addition of a solution of BBr₃ in DCM (1.5 M, 2 mL) in an ice-water bath, and the mixture was stirred at room temperature for 1 hour. The reaction mixture was quenched by dropwise addition of MeOH (2 mL) and concentrated. The residue was purified by column chromatography (DCM: MeOH = 85:15), and further purified by reverse-phase chromatography (ACN: 0.1% aqueous formic acid = 85%) to obtain **Target Compound 64** (24.5 mg) with a yield of 36.3%.

LC-MS (m/z): 374.0 [M+H]⁺.

¹H NMR (400 MHz, DMSO-*d*₆) δ 10.17 (brs, 1H), 9.05 (s, 1H), 8.38 (s, 1H), 7.05-6.80 (m, 3H), 4.69 (s, 1H), 3.71-3.62 (m, 1H), 3.49-3.38 (m, 1H), 2.10-2.00 (m, 1H), 1.98 (s, 3H), 1.94-1.87 (m, 1H), 1.70-1.60 (m, 2H), 1.33-1.16 (m, 4H).

### Example 19: Synthesis of (S)-3-methyl-2-(6-((1-methylpiperidin-3-yl)amino)-2H-pyrazolo[3,4-d]pyrimidin-2-yl)-5-(trifluoromethyl)phenol (Target Compound 65)

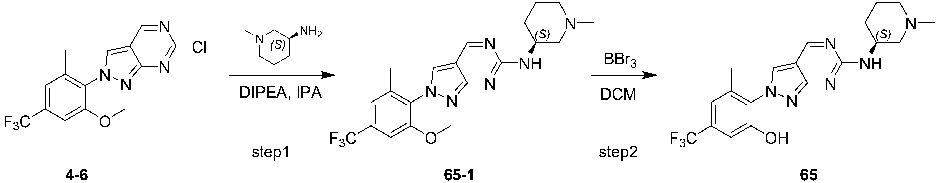

### Step 1: Synthesis of (S)-2-(2-methoxy-6-methyl-4-(trifluoromethyl)phenyl)-N-(1-methylpiperidin-3-yl)-2H-pyrazolo[3,4-d]pyrimidin-6-amine (65-1)

Compound **4-6** (1.00 g, 2.92 mmol) and (S)-1-methylpiperidin-3-amine (433.2 mg, 3.79 mmol) were dissolved in IPA (15 mL), followed by addition of DIPEA (1.02 mL, 2.84 mmol), and the mixture was stirred at 80°C for 3 hours. The reaction mixture was cooled to room temperature and concentrated under reduced pressure. The residue was purified by column chromatography (MeOH: DCM = 10%) to obtain crude Compound **65-1** (1.50 g).

LC-MS (m/z): 421.0 [M+H]⁺.

### Step 2: Synthesis of (S)-3-methyl-2-(6-((1-methylpiperidin-3-yl)amino)-2H-pyrazolo[3,4-d]pyrimidin-2-yl)-5-(trifluoromethyl)phenol (Target Compound 65)

Crude Compound **65-1** (1.50 g) was dissolved in DCM (20 mL), followed by addition of a solution of BBr₃ in DCM (8.92 mL, 2.0 M) in an ice-water bath, and the mixture was stirred at room temperature for 4 hours. The reaction mixture was quenched with MeOH and concentrated under reduced pressure. The residue was purified by column chromatography (MeOH: DCM = 20%) to obtain **Target Compound 65** (856.3 mg) with a yield of 71.9% and e.e.% = 99.23%.

LC-MS (m/z): 407.0 [M+H]⁺.

¹H NMR (600 MHz, DMSO-*d*₆) δ 11.20-10.48 (brs, 1H), 9.09 (s, 1H), 8.47 (s, 1H), 7.24 (s, 1H), 7.18 (s, 1H), 7.15-7.07 (brs, 1H), 4.08-3.91 (m, 1H), 3.03-2.90 (m, 1H), 2.73-2.63 (m, 1H), 2.23 (s, 3H), 2.07 (s, 3H), 2.02-1.79 (m, 3H), 1.75-1.67 (m, 1H), 1.62-1.50 (m, 1H), 1.42-1.30 (m, 1H).

### Example 20: Synthesis of (R)-2-fluoro-5-methyl-6-(6-((1-methylpiperidin-3-yl)amino)-2H-pyrazolo[3,4-d]pyrimidin-2-yl)-3-(trifluoromethyl)phenol (Target Compound 66)

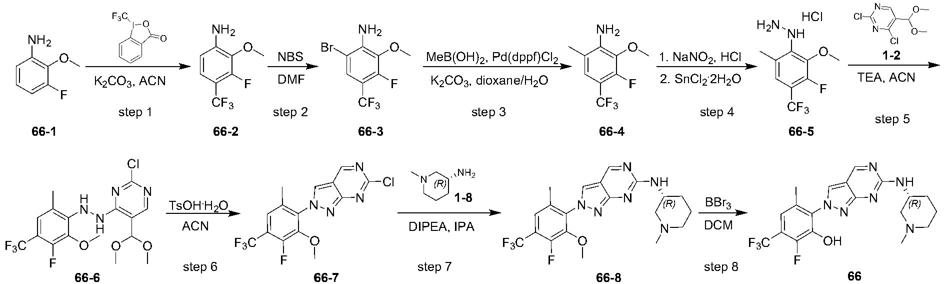

### Step 1: Synthesis of 3-fluoro-2-methoxy-4-(trifluoromethyl)aniline (66-2)

1-Trifluoromethyl-1,2-benziodoxol-3(1*H*)-one (14.00 g, 44.30 mmol) and K₂CO₃ (9.20 g, 66.45 mmol) were dissolved in anhydrous ACN (350 mL), followed by addition of Compound **66-1** (9.40 g, 66.45 mmol), and the mixture was stirred at 75°C for 16 hours. The reaction mixture was concentrated under reduced pressure, dissolved in EA (100 mL), and filtered. The filtrate was washed with HCl solution (2 N, 100 mL × 3) and saturated brine (50 mL × 2), dried over anhydrous Na₂SO₄, filtered, and the filtrate was concentrated under reduced pressure. The residue was purified by column chromatography (EA: PE = 20:80) to obtain Compound **66-2** (4.90 g) with a yield of 52.9%.

LC-MS (m/z): 210.0 [M+H]⁺.

### Step 2: Synthesis of 6-bromo-3-fluoro-2-methoxy-4-(trifluoromethyl)aniline (66-3)

Compound **66-2** (4.70 g, 22.47 mmol) was dissolved in DMF (40 mL), followed by addition of NBS (4.20 g, 23.60 mmol), and the mixture was stirred at room temperature for 1 hour. The reaction mixture was quenched with sodium thiosulfate solution (200 mL), extracted with EA (100 mL × 3), washed with saturated brine (100 mL), dried over anhydrous Na₂SO₄, filtered, and the filtrate was concentrated under reduced pressure. The residue was purified by column chromatography (EA: PE = 20:80) to obtain Compound **66-3** (3.10 g) with a yield of 47.9%.

LC-MS (m/z): 288.0/290.0 [M+H]⁺.

### Step 3: 3-fluoro-2-methoxy-6-methyl-4-(trifluoromethyl)aniline (66-4)

Compound **66-3** (3.10 g, 10.76 mmol), methylboronic acid (1.90 g, 32.29 mmol), Pd(dppf)Cl₂ (393.8 mg, 0.54 mmol), and K₂CO₃ (4.50 g, 32.29 mmol) were dissolved in a mixed solvent of dioxane and water (5:1, 90 mL), and the mixture was stirred at 90°C for 16 hours under a nitrogen atmosphere. The reaction mixture was concentrated, dissolved in EA (100 mL), and filtered. The filtrate was washed with water (50 mL) and saturated brine (50 mL), dried over anhydrous Na₂SO₄, filtered, and concentrated under reduced pressure. The residue was purified by column chromatography (EA: PE = 10:90) to obtain Compound **66-4** (2.10 g) with a yield of 87.4%.

LC-MS (m/z): 224.0 [M+H]⁺.

### Step 4: Synthesis of (3-fluoro-2-methoxy-6-methyl-4-(trifluoromethyl)phenyl)hydrazine hydrochloride (66-5)

Compound **66-4** (270.0 mg, 1.21 mmol) was dissolved in concentrated hydrochloric acid (2 mL) in an ice-salt bath. A solution of NaNO₂ (125.2 mg, 1.81 mmol) in water (1 mL) was slowly added dropwise at 0°C, and the mixture was stirred at the same temperature for 1 hour. A solution of SnCl₂·2H₂O (546.0 mg, 2.42 mmol) in concentrated hydrochloric acid (1 mL) was then slowly added dropwise, and the mixture was stirred at the same temperature for 2 hours. The reaction mixture was filtered. The filter cake was washed with a small amount of water and dried under reduced pressure to obtain crude Compound **66-5** (200.0 mg).

### Step 5: Synthesis of 2-chloro-5-(dimethoxymethyl)-4-(2-(3-fluoro-2-methoxy-6-methyl-4-(trifluoromethyl)phenyl)hydrazinyl)pyrimidine (66-6)

Crude Compound **66-5** (200.0 mg) and Compound 1-2 (182.8 mg, 0.76 mmol) were dissolved in ACN (3 mL), followed by addition of TEA (0.41 mL, 2.91 mmol), and the mixture was stirred at room temperature for 16 hours. The reaction mixture was concentrated under reduced pressure. The residue was purified by column chromatography (EA: PE = 20:80) to obtain Compound **66-6** (150.0 mg) with a two-step yield of 28.9%.

LC-MS (m/z): 425.0 [M+H]⁺.

### Step 6: Synthesis of 6-chloro-2-(3-fluoro-2-methoxy-6-methyl-4-(trifluoromethyl)phenyl)-2H-pyrazolo[3,4-d]pyrimidine (66-7)

Compound **66-6** (150.0 mg, 0.35 mmol) was dissolved in ACN (2 mL), followed by addition of TsOH·H₂O (67.2 mg, 0.35 mmol), and the reaction mixture was stirred at 80°C for 1 hour. The reaction mixture was cooled to room temperature, diluted with EA (10 mL), washed with NaHCO₃ aqueous solution (10 mL) and saturated brine (10 mL), dried over anhydrous Na₂SO₄, filtered, and concentrated under reduced pressure. The residue was purified by column chromatography (EA: PE = 30:70) to obtain Compound **66-7** (70.0 mg) with a yield of 55.0%.

LC-MS (m/z): 361.0 [M+H]⁺.

### Step 7: Synthesis of (R)-2-(3-fluoro-2-methoxy-6-methyl-4-(trifluoromethyl)phenyl)-N-(1-methylpiperidin-3-yl)-2H-pyrazolo[3,4-d]pyrimidin-6-amine (66-8)

Compound **66-7** (70.0 mg, 0.19 mmol) and (R)-1-methylpiperidin-3-amine (28.8 mg, 0.25 mmol) were dissolved in IPA (2 mL), followed by addition of DIPEA (50.2 mg, 0.39 mmol), and the mixture was stirred at 80°C for 3 hours. The reaction mixture was cooled to room temperature and concentrated under reduced pressure. The residue was purified by column chromatography (MeOH: DCM = 10:90) to obtain crude Compound **66-8** (85.0 mg).

LC-MS (m/z): 439.0 [M+H]⁺.

### Step 8: Synthesis of (R)-2-fluoro-5-methyl-6-(6-((1-methylpiperidin-3-yl)amino)-2H-pyrazolo[3,4-d]pyrimidin-2-yl)-3-(trifluoromethyl)phenol (Target Compound 66)

Crude Compound **66-8** (85.0 mg) was dissolved in DCM (2 mL), followed by addition of a solution of BBr₃ in DCM (0.49 mL, 2.0 M) in an ice-water bath, and the mixture was stirred at room temperature for 1 hour. The reaction mixture was quenched with MeOH in an ice-water bath and concentrated under reduced pressure. The residue was purified by reverse-phase preparative chromatography (ACN: 10 mmol/L NH₄HCO₃ aqueous solution = 85%) to obtain **Target Compound 66** (21.7 mg) with a yield of 26.3%.

LC-MS (m/z): 425.0 [M+H]⁺.

¹H NMR (600 MHz, DMSO-*d*₆) δ 9.07 (s, 1H), 8.45 (s, 1H), 7.11 (s, 1H), 6.94 (d, J= 6.0 Hz, 1H), 4.05-3.96 (m, 1H), 3.01-2.95 (m, 1H), 2.76-2.65 (m, 1H), 2.26 (s, 3H), 2.08-1.95 (m, 5H), 1.88-1.81 (m, 1H), 1.76-1.68 (m, 1H), 1.61-1.51 (m, 1H), 1.41-1.29 (m, 1H).

### Example 21: Inhibitory effect of compounds of the present disclosure on IL-1β release in PMA-differentiated THP-1 cells

THP-1 cells were purchased from Wuhan Pricella Biotechnology Co., Ltd. (Cat. No. CL-0233). The complete culture medium used was RPMI 1640 medium containing 10% heat-inactivated fetal bovine serum (FBS), 1% penicillin-streptomycin (Pen-Strep), and 0.05 mM β-mercaptoethanol. Cells were cultured according to the instructions provided by the supplier and maintained in the logarithmic phase prior to experimentation. The cell density was adjusted to 1 × 10⁵ cells/mL. Cells were differentiated by treatment with phorbol 12-myristate 13-acetate (PMA) at a final concentration of 100 ng/mL for 16 hours, followed by stimulation with lipopolysaccharide (LPS) at a concentration of 100 ng/mL for 3 hours. The compounds of the present disclosure were dissolved in dimethyl sulfoxide (DMSO), diluted 3-fold with culture medium to the desired concentrations, and added to a 96-well plate. After 1 hour of compound treatment, nigericin sodium was added to the culture plate at a final concentration of 10 µM, followed by incubation for 1 hour. Cell-free supernatants were collected, and IL-1β levels were measured according to the instructions using an ELISA kit (Invitrogen). Vehicle-treated samples were used as controls. EC₅₀ values were calculated using the four-parameter logistic model [log(agonist) vs. response - Variable slope] in GraphPad software. The results are shown in Table 1.

**Table 1: Inhibitory activity of compounds on IL-1β release in THP-1 cells**

| Compound | IC₅₀ (nM) | | Compound | IC₅₀ (nM) | |
|---|---|---|---|---|---|
| **1** | >10000 | + | **12** | 144.0 | ++ |
| **2** | 3.5 | ++++ | **48** | 12.5 | +++ |
| **3** | 1.7 | ++++ | **60** | 2227.4 | + |
| **4** | 7.8 | ++++ | **61** | 15.1 | +++ |
| **5** | 2.7 | ++++ | **62** | 1231.3 | + |
| **6** | 9.8 | ++++ | **63** | 373.8 | + |
| **7** | 4.7 | ++++ | **64** | 1674.4 | + |
| **8** | 50.1 | +++ | **65** | >10000 | + |
| **9** | 24.4 | +++ | **66** | >1000 | + |
| **10** | 34.0 | +++ | **67** | 350.0 | + |

| | | | | | |
|---|---|---|---|---|---|
| Note: 0 nM < ++++ ≤ 10 nM; 10 nM < +++ ≤ 100 nM; 100 nM < ++ ≤ 300 nM; 300 nM < +. | | | | | |

The experimental results demonstrated that the compounds of the present disclosure significantly inhibited pyroptosis in PMA-differentiated human THP-1 cells and reduced IL-1β expression in these cells.

### Example 22: Inhibitory effect of compounds of the present disclosure on IL-1β release in PBMCs

Human peripheral blood mononuclear cells (PBMCs) were purchased from MileCell Biotechnology (Cat. No. PB010C). The complete culture medium used was RPMI 1640 medium containing FBS (10%) and Pen-Strep (1%). PBMCs were seeded into a 96-well plate at a density of 1 × 10⁵ cells/well and cultured overnight in an incubator, followed by stimulation with LPS at a final concentration of 100 ng/mL for 3 hours. The derivatives of the present disclosure were dissolved in DMSO, diluted 3-fold with culture medium to the desired concentrations, and added to a 96-well plate. After 1 hour of incubation, adenosine triphosphate (ATP) was added to the plate at a final concentration of 5 mM, followed by incubation for another 1 hour. Cell-free supernatants were collected, and IL-1β levels were measured according to the instructions using an ELISA kit (Invitrogen). Vehicle-treated samples were used as controls. IC₅₀ values were calculated using the four-parameter logistic model [log(agonist) vs. response - Variable slope] in GraphPad software. The results are shown in Table 2.

**Table 2: Inhibitory activity of compounds on IL-1β release in PBMCs**

| Compound | IC₅₀ (nM) |
|---|---|
| **2** | 5.9 |
| **3** | 2.4 |
| **4** | 14.8 |
| **5** | 6.9 |
| **6** | 12.2 |

### Example 23: Inhibitory effect of compounds on hERG ion channels

The inhibitory effects of compounds on human hERG ion channels stably expressed in HEK293 cells were evaluated using conventional patch-clamp techniques. Test compounds were prepared at a concentration of 10 µM. Each cell served as its own control. All compounds were perfused using a gravity-driven perfusion system. After stabilization of the hERG current, the current magnitudes before and after compound addition were compared for each cell. The percentage inhibition of the hERG current by each compound was then calculated. The results are shown in Table 3.

**Table 3: Percentage inhibition of the hERG current by compounds**

| Compound | hERG% at 10 µM |
|---|---|
| **2** | 9.3% |
| **4** | 27.7% |

### Example 24: Metabolic stability evaluation of test compounds

### 1. Preparation of experimental materials

### 1.1 Incubation buffer

A certain amount of anhydrous sodium dihydrogen phosphate was weighed into a centrifuge tube, followed by addition of an appropriate amount of ultrapure water with vortex mixing to prepare a 0.1 mol/L sodium dihydrogen phosphate solution for later use. A certain amount of anhydrous disodium hydrogen phosphate was weighed into a centrifuge tube, followed by addition of an appropriate amount of ultrapure water with vortex mixing to prepare a 0.1 mol/L disodium hydrogen phosphate solution for later use. The sodium dihydrogen phosphate solution (0.1 mol/L) and the disodium hydrogen phosphate solution (0.1 mol/L) were mixed at a ratio of 19:81 (v:v) in a reagent bottle, and mixed thoroughly to obtain a 100 mmol/L phosphate buffer (PB) (concentration calculated based on phosphate ion concentration), which was stored at 2 to 8°C for later use.

### 1.2 Initiator (mixture of NADPH and UDPGA)

An appropriate amount of NADPH was precisely weighed into a 1.5 mL centrifuge tube, followed by addition of an appropriate amount of PB with vortex mixing to prepare a 40 mmol/L NADPH solution, which was placed on ice for later use. An appropriate amount of UDPGA was precisely weighed into a 1.5 mL centrifuge tube, followed by addition of an appropriate amount of PB with vortex mixing to prepare a 40 mmol/L UDPGA solution, which was placed on ice for later use. Equal volumes of the NADPH and UDPGA solutions were pipetted into the same centrifuge tube with vortex mixing to prepare a mixture of NADPH and UDPGA (containing 20 mmol/L NADPH and 20 mmol/L UDPGA), which was placed on ice for later use.

### 1.3 Positive control substrate working solution (mixture of testosterone and 7-hydroxycoumarin)

An appropriate amount of testosterone reference standard was precisely weighed and dissolved in DMSO with vortex mixing to prepare a 20 mmol/L testosterone stock solution, which was stored in a -20°C freezer. An appropriate amount of 7-hydroxycoumarin reference standard was precisely weighed and dissolved in DMSO with vortex mixing to prepare a 20 mmol/L 7-hydroxycoumarin stock solution, which was stored in a -20°C freezer. Equal volumes of the testosterone and 7-hydroxycoumarin stock solutions were pipetted into the same 1.5 mL centrifuge tube and diluted with 50% methanol-water to prepare a 20 µmol/L mixed substrate working solution, which was placed on ice for later use.

### 1.4 Test substrate working solution

An appropriate amount of test compound reference standard was precisely weighed and dissolved in DMSO with vortex mixing to prepare a 10 mmol/L test compound stock solution, which was stored in a -20°C freezer for later use. An appropriate amount of the test compound stock solution was pipetted into a 1.5 mL centrifuge tube and diluted with 50% methanol-water to prepare a 20 µmol/L substrate working solution, which was placed on ice for later use.

### 2 Experimental methods

The study consisted of three groups: a positive control group (PC), a negative control group (NC), and a treatment group. The test compounds were incubated with human, rat, or mouse liver microsomes in the presence of NADPH and UDPGA for a specified period (a negative control lacking NADPH and UDPGA and a positive control were included). The reaction was terminated by adding a stop solution. The remaining amount of test compound in the sample was quantified by LC-MS/MS. Metabolic stability of the test compounds in liver microsomes was evaluated by comparing compound concentrations at different reaction times were compared with those measured at T₀. Generally, the final protein concentration of liver microsomes in the reaction system was 1.0 mg/mL. All incubations were performed in a water bath at 37°C.

### 2.1 Experimental groups

Treatment group: The test compound was incubated with liver microsomes in the presence of NADPH and UDPGA for 60 minutes.

Negative control group (NC): The test compound was incubated with liver microsomes for 60 minutes in the absence of any cofactors.

Positive control group (PC): The probe substrates of CYP3A4 and UGT, testosterone and 7-hydroxycoumarin, were incubated with liver microsomes in the presence of NADPH and UDPGA for 60 minutes.

### 2.2 Experimental procedure

(1) An appropriate amount of liver microsomes from the species required for the experiment was thawed on ice and gently shaken to mix.
(2) Treatment group and negative control group (NC): An appropriate amount of PB was mixed with an appropriate amount of the test substrate working solution. An appropriate amount of liver microsomes was then pipetted into a 1.5 mL centrifuge tube and mixed by pipetting 20 to 30 times to ensure homogeneity, with the final concentration of liver microsomes being 1 mg/mL (if required, the concentration of liver microsomes may be adjusted to 0.5 mg/mL).
(3) Positive control group (PC): An appropriate amount of PB was mixed with an appropriate amount of the positive control substrate working solution. An appropriate amount of liver microsomes was then pipetted into a 1.5 mL centrifuge tube and mixed by pipetting 20 to 30 times to ensure homogeneity, with the final concentration of liver microsomes being 1 mg/mL.
(4) Treatment group: 90 µL of the solution prepared in Step (2) was aliquoted and pre-incubated in a water bath at 37°C for 5 minutes. 10 µL of 20 mmol/L initiator was then added to initiate the reaction. At each designated incubation time point, an appropriate amount of pre-cooled methanol solution containing internal standard was added to terminate the reaction (n = 2).
(5) Negative control group (NC): 90 µL of the solution prepared in Step (2) was aliquoted and pre-incubated in a water bath at 37°C for 5 minutes. 10 µL of PB was then added. At each designated incubation time point, an appropriate amount of pre-cooled methanol solution containing internal standard was added to terminate the reaction (n ≥ 2).
(6) Positive control group (PC): 90 µL of the solution prepared in Step (3) was aliquoted and pre-incubated in a water bath at 37°C for 5 minutes. 10 µL of 20 mmol/L initiator was then added to initiate the reaction. At each designated incubation time point, an appropriate amount of pre-cooled methanol solution containing internal standard was added to terminate the reaction (n = 2).
(7) The samples prepared as described above were vortexed at 2500 rpm for 1 minute and centrifuged at 17000 g and 4°C for 10 minutes. The supernatants were collected for LC-MS/MS analysis.

### 3.3 Data processing and analysis

Samples from the stability assay were quantified using a standard curve to obtain the concentration of the test compound at each time point, and the percentage of parent compound remaining relative to the amount present before incubation (0 min) was calculated. Alternatively, the percentage of parent compound remaining relative to the amount present before incubation (0 min) may be calculated using the ratio of the peak area of the test compound to that of the internal standard. The calculation was performed according to the following equation: Parent remaining (% of 0 min) = parent amount at T₆₀ / parent amount at T₀ × 100%; T₆₀: 60-min incubation time point; T₀: 0-min incubation time point. The results are shown in Table 4.

**Table 4: In vitro metabolic stability of compounds in liver microsomes**

| Compound | 60-min remaining (%) | | |
|---|---|---|---|
| | Human | Rat | Mouse |
| **2** | 86.56 | 86.09 | 82.57 |
| **4** | 93.10 | 97.99 | 93.99 |
| **6** | 88.14 | 88.14 | 95.62 |
| **7** | 95.34 | 94.68 | 92.13 |

### Example 25: Evaluation of in vivo pharmacokinetics of selected compounds in rats

Male SD rats were fasted overnight (with free access to drinking water) and divided into an intravenous administration (IV) group and an intragastric administration (PO) group. For the IV group, blood samples (approximately 0.3 mL of blood per sample) were collected from the orbital venous plexus at 2 min, 10 min, 30 min, 1 h, 2 h, 4 h, 6 h, 8 h, and 24 h following administration. The samples were anticoagulated with disodium ethylenediaminetetraacetate (EDTA-2Na), placed on ice immediately after collection, and centrifuged at 4°C for 10 minutes within 1 hour to separate the plasma, which was stored at - 20°C for analysis. For the PO group, blood samples (0.3 mL) were collected from the orbital venous plexus at 5 min, 15 min, 30 min, 1 h, 2 h, 4 h, 6 h, 8 h, and 24 h following administration. Sample processing was identical to that used for the IV group. Plasma concentrations of the parent compounds were determined by LC-MS/MS. Pharmacokinetic parameters were calculated using Phoenix WinNonlin 8.1 based on plasma concentration-time data collected at different time points. The results are shown in Table 5.

**Table 5: Results of in vivo pharmacokinetic studies of selected compounds in rats**

| Compound | Route of administration | Dose (mg/kg) | Cₘₐₓ (ng/mL) | AUC (ng·h/mL) | t_{1/2} (h) | F (%) |
|---|---|---|---|---|---|---|
| **2** | PO | 5 | 1934.7 | 8804.2 | 2.95 | 99.8 |
| **4** | PO | 5 | 5442.0 | 38296.3 | 4.05 | 74.8 |

The experimental results demonstrated that the compounds of the present disclosure exhibited favorable pharmacokinetic properties in SD rats, including good oral bioavailability, oral exposure, maximum plasma concentration, and half-life.

### Example 26: Inhibitory effect of Compound 2 on cytokine production in a mouse inflammation model

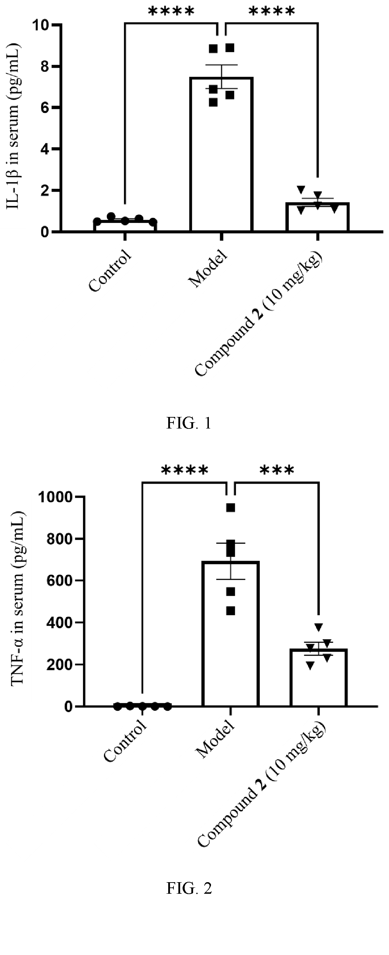

Female C57BL/6 mice aged 6 to 8 weeks were randomly divided into three groups of 5 mice each, namely a Control group, a Model group, and a Compound 2 treatment group. The Compound 2 treatment group received Compound 2 (10 mg/kg) by oral gavage once daily for three consecutive days, while the Control group and the Model group received an equivalent volume of vehicle daily. One hour after intragastric administration on Day 3, mice in the Model group and the Compound 2 treatment group were administered LPS (5 mg/kg) via intraperitoneal injection, while mice in the Control group were administered an equivalent volume of sodium chloride injection via intraperitoneal injection. Two hours after intraperitoneal injection of LPS, blood samples were collected from mice in each group, allowed to stand at room temperature for 1 hour, and centrifuged at 4°C and 5000 rpm for 10 minutes. Serum samples were collected and analyzed for cytokines IL-1β, TNF-α, and IL-6 by ELISA. The experimental results are shown in FIG. 1, FIG. 2, and FIG. 3, wherein *P < 0.05, ***P* < 0.01, ****P* < 0.005, *****P* < 0.001.

Experimental conclusion: The compounds of the present disclosure significantly reduced LPS-induced serum levels of cytokines IL-1β, TNF-α, and IL-6 in mice.

### Example 27: Inhibitory effect of Compound 4 hydrochloride on cytokine production in a mouse inflammation model

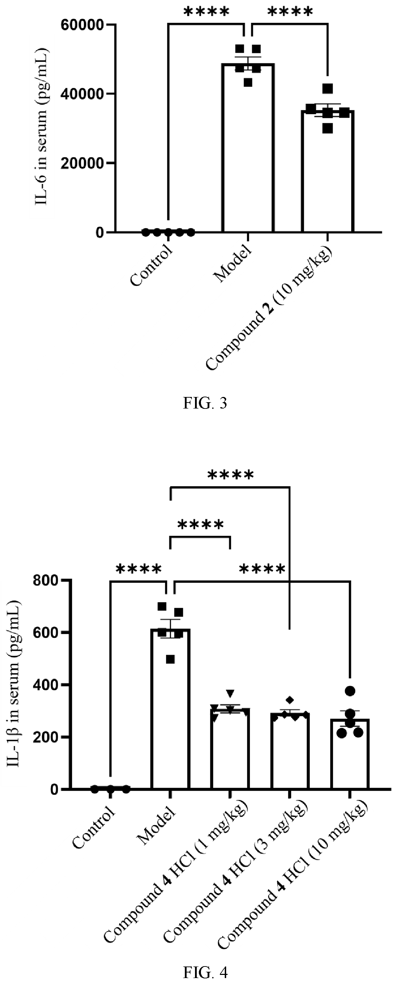

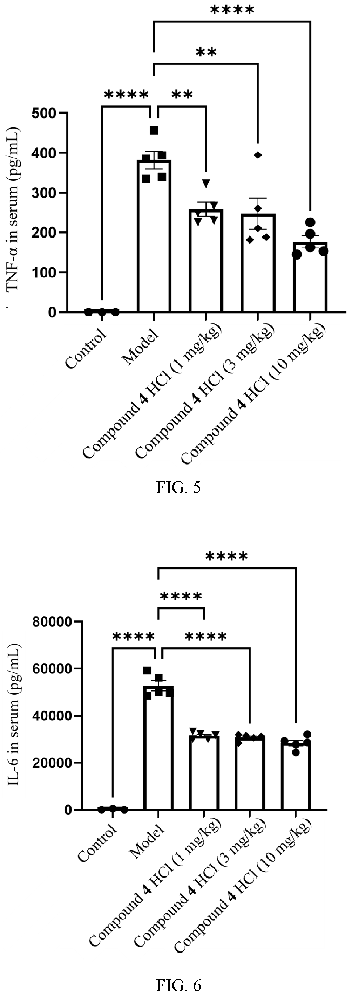

Female C57BL/6 mice aged 6 to 8 weeks were randomly divided into three groups of 5 mice each, namely a Control group, a Model group, and a Compound 4 hydrochloride treatment group (hereinafter referred to as the treatment group). The treatment group received Compound **4** hydrochloride by oral gavage once daily for three consecutive days at doses of 10 mg/kg, 3 mg/kg, and 1 mg/kg, while the Control group and the Model group received an equivalent volume of vehicle daily. One hour after intragastric administration on Day 3, mice in the Model group and the treatment group were administered LPS (5 mg/kg) via intraperitoneal injection, while mice in the Control group were administered an equivalent volume of sodium chloride injection via intraperitoneal injection. Four hours after intraperitoneal injection, blood samples were collected from mice in each group, allowed to stand at room temperature for 1 hour, and centrifuged at 4°C and 5000 rpm for 10 minutes. Serum samples were collected and analyzed for cytokines IL-1β, TNF-α, and IL-6 by ELISA. The experimental results are shown in FIG. 4, FIG. 5, and FIG. 6, wherein *P < 0.05, ***P < 0.01, ***P* < 0.005, *****P* < 0.001.

### Example 28: Toxicity evaluation of Compound 4 hydrochloride administered by oral gavage to SD rats for 28 consecutive days

Eight male and eight female SD rats were randomly divided into groups as follows: normal control group (2 rats per sex), low-dose group (3 rats per sex per group), and high-dose group (3 rats per sex per group). The low-dose and high-dose groups received Compound 4 hydrochloride solutions at concentrations of 3 and 10 mg/mL (calculated as the free base) by oral gavage, respectively. The administration volume was 10 mL/kg, corresponding to doses of 30 and 100 mg/kg, respectively. The normal control group received an equivalent volume of vehicle (water). During the experiment, the rats had free access to drinking water and were administered continuously for 28 days. On the day following the final administration, blood samples were collected for measurement of 23 routine hematological and biochemical parameters. Gross necropsy examinations were performed on the heart, liver, spleen, lungs, kidneys, brain, gastrointestinal tract, thymus, testes (or ovaries), and other organs.

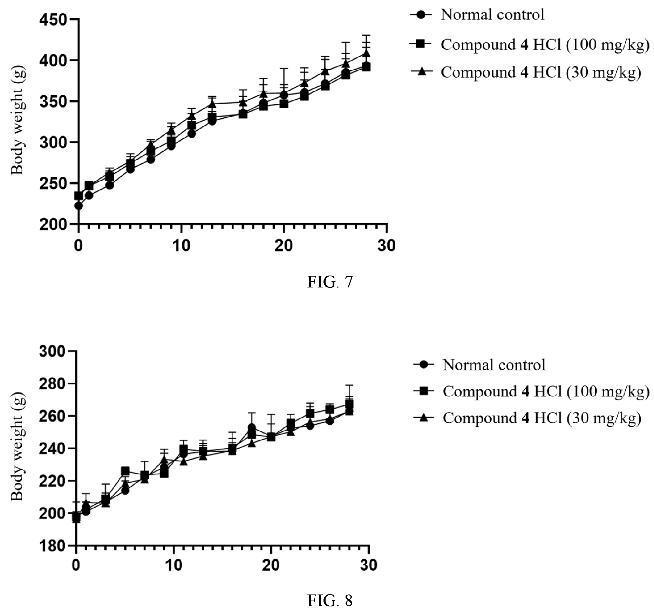

The experimental results demonstrated that no obvious toxic reactions were observed in any treatment group following administration, and there was no significant effect on body weight. The experimental results are shown in FIG. 7 and FIG. 8. Gross pathological examination revealed no significant pathological changes in the surface color, shape, size, or texture of the organs from animals in each treatment group. Hematological and biochemical parameters showed no significant abnormalities compared with the normal group. The compounds of the present disclosure exhibited an excellent safety profile.

### Example 29: Inhibitory effect of Compound 4 hydrochloride on cytochrome P450 isoforms CYP1A2, CYP2B6, CYP2C8, CYP2C9, CYP2C19, CYP2D6, CYP3A4-M, and CYP3A4-T

### 1. Preparation of phosphate buffer (PB, 100 mM, pH 7.4)

Disodium hydrogen phosphate was dissolved in ultrapure water by sonication to prepare a 100 mM solution (designated as Solution A). Potassium dihydrogen phosphate was dissolved in ultrapure water by sonication to prepare a 100 mM solution (designated as Solution B). Solution B was slowly added to Solution A under stirring until the pH reached 7.4. The phosphate buffer was stored at 4°C for later use. Ultrapure water was produced using a Sartorius ultrapure water system.

### 2. Preparation of positive control solution

The positive control was prepared as a 10 mM DMSO stock solution. The volume ratio of the organic solvent introduced into the assay system along with the test compound was 1.0%. The working concentration of the positive inhibitor and the final concentration in the reaction system are shown in Table 6 below.

**Table 6**

| Enzyme | Positive control | Working concentration (µM) | Final concentration (µM) |
|---|---|---|---|
| CYP1A2 | α-Naphthoflavone | 300 | 3 |
| CYP2B6 | Sertraline HCl | 5000 | 50 |
| CYP2C8 | Quercetin | 10000 | 100 |
| CYP2C9 | Sulfaphenazole | 1000 | 10 |
| CYP2C 19 | (+/-)-N-3-Benzylnirvanol | 5000 | 50 |
| CYP2D6 | Quinidine | 500 | 5 |
| CYP3A4 | Ketoconazole | 500 | 5 |

### 3. Preparation of Cocktail substrate stock solution

Substrate stock solutions were prepared as shown in Table 7 below and stored in a - 20°C freezer. The stock solutions were thawed at room temperature before use.

**Table 7**

| Enzyme | Substrate | Substrate concentration (mM) | Working concentration (µM) | Final concentration (µM) |
|---|---|---|---|---|
| CYP1A2 | Phenacetin | 50 (MeOH) | 300 | 30 |
| CYP2B6 | Bupropion HCl | 200 (MeOH) | 50 | 5 |
| CYP2C8 | Amodiaquine HCl | 20 (MeOH) | 50 | 5 |
| CYP2C9 | Diclofenac | 20 (MeOH) | 100 | 10 |
| CYP2C19 | S-Mephenytoin | 20 (MeOH) | 300 | 30 |
| CYP2D6 | Dextromethorphan HBr | 20 (MeOH) | 50 | 5 |
| CYP3A4-M | Testosterone | 20 (DMSO) | 50 | 5 |
| CYP3A4-T | Midazolam | 20 (MeOH) | 800 | 80 |

### 4. Preparation of Compound 4 hydrochloride working solution

Compound 4 hydrochloride was prepared as a 5 mM DMSO stock solution. The volume ratio of the organic solvent introduced into the assay system along with the test compound was 1.0%. The working concentration of Compound 4 hydrochloride and the final concentration in the reaction system are shown in Table 8 below.

**Table 8**

| Stock concentration | Stock volume (µL) | 1:1 DMSO:MeOH added (µL) | Working concentration (mM) | Final concentration (µM) |
|---|---|---|---|---|
| 5 mM | 60.0 | 240.0 | 1.00 | 10.0 |

### 5. Preparation of human liver microsome mixture

Human liver microsomes were purchased from BIOIVT (Cat. No. 452117, Lot No. SEU). The stock concentration was 20 mg/mL. The working concentration of the human liver microsomes and the final concentration in the reaction system are shown in Table 9 below.

**Table 9**

| Enzyme | Volume of human liver microsomes (µL) | Volume of PB (mL) | Working concentration (mg/mL) | Final concentration of liver microsomes (mg/mL) |
|---|---|---|---|---|
| CYP1A2 | 181.0 | 14.118 | 0.2532 | 0.2 |
| CYP2B6 | 181.0 | 14.118 | 0.2532 | 0.2 |
| CYP2C8 | 181.0 | 14.118 | 0.2532 | 0.2 |
| CYP2C9 | 181.0 | 14.118 | 0.2532 | 0.2 |
| CYP2C19 | 60.0 | 1.836 | 0.6329 | 0.5 |
| CYP2D6 | 181.0 | 14.118 | 0.2532 | 0.2 |
| CYP3A4-M | 181.0 | 14.118 | 0.2532 | 0.2 |
| CYP3A4-T | 181.0 | 14.118 | 0.2532 | 0.2 |

### 6. Preparation of NADPH cofactor solution

Prior to the experiment, 13.14 mg of NADPH (Roche, Cat. No. 10107824001, Lot No. 71466624) was weighed and dissolved in 1.545 mL of PB to prepare a 10 mM working solution. The final concentration of NADPH in the assay system was 1 mM.

7. All incubations were performed in a 1.5 mL centrifuge tube. First, 2 µL of the Compound 4 hydrochloride working solution or positive control working solution was added to the corresponding centrifuge tube. For the inhibitor-free group, 2 µL of DMSO:MeOH (1:1) solvent was added. Subsequently, 20 µL of the mixed substrate working solution and 158 µL of the liver microsome working solution were added. The mixture was pre-incubated in a water bath at 37°C for 5 minutes. The reaction was initiated by adding 20 µL of NADPH cofactor. The mixture was then further incubated at 37°C for the designated periods (see Table 10). All samples were prepared in duplicate.

After incubation, the reaction was terminated by adding 400 µL of ice-cold stop solution (methanol containing 1 ng/mL LMTA). The samples were vortexed and centrifuged at 17000 g and 4°C for 10 minutes. 180 µL of the supernatant was transferred into a 96-well plate for LC-MS/MS analysis.

Relative activity was calculated using the following equation: Relative activity (NC) = amount of metabolite produced in the treatment group or positive control group / average amount of metabolite produced in the negative control group; Inhibition (%) = (1 - NC) × 100%. The experimental results are shown in Table 10.

**Table 10: Inhibitory effect of Compound 4 hydrochloride on CYP450 isoforms**

| Enzyme | Incubation period (min) | Inhibition (%) at 10 µM |
|---|---|---|
| CYP1A2 | 15 | 11.5 |
| CYP2B6 | 45 | 21.8 |
| CYP2C8 | 15 | 7.1 |
| CYP2C9 | 15 | -0.4 |
| CYP2C19 | 45 | 9.7 |
| CYP2D6 | 20 | -1.6 |
| CYP3A4-M | 15 | -1.1 |
| CYP3A4-T | 5 | 24.8 |

The experimental results demonstrated that the compounds of the present disclosure exhibited no significant direct inhibitory effects on any of the seven major CYP450 isoforms evaluated in human liver microsomes.

## Claims

1. A pyrazolopyrimidine compound, which is a compound represented by the following general formula (I), an isomer thereof, or a pharmaceutically acceptable salt thereof: 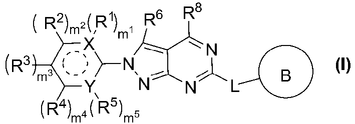
wherein -̅ -̅ -̅ is selected from the group consisting of a single bond and a double bond;
m¹, m², m³, m⁴, and m⁵ are each independently selected from the group consisting of 1 and 2;
X and Y are each independently selected from the group consisting of C and N;
R¹ and R⁵ are each independently selected from the group consisting of absent, hydrogen, halogen, hydroxyl, cyano, amino, C₁₋₆ alkyl, C₁₋₆ alkylamino, C₁₋₆ alkoxy, C₂₋₆ alkenyl, C₂₋₆ alkynyl, C₃₋₈ cycloalkyl, 3- to 6-membered heterocyclyl, and 5- to 10-membered heteroaryl; the C₁₋₆ alkyl, C₁₋₆ alkylamino, C₁₋₆ alkoxy, C₂₋₆ alkenyl, C₂₋₆ alkynyl, C₃₋₈ cycloalkyl, 3- to 6-membered heterocyclyl, or 5- to 10-membered heteroaryl may be optionally further substituted by one or more substituents selected from the group consisting of C₁₋₆ alkyl and halogen;
R², R³, and R⁴ are each independently selected from the group consisting of hydrogen, halogen, hydroxyl, cyano, amino, C₁₋₆ alkyl, C₁₋₆ alkylamino, C₁₋₆ alkoxy, C₂₋₆ alkenyl, C₂₋₆ alkynyl, C₃₋₈ cycloalkyl, 3- to 6-membered heterocyclyl, and 5- to 10-membered heteroaryl; the C₁₋₆ alkyl, C₁₋₆ alkylamino, C₁₋₆ alkoxy, C₂₋₆ alkenyl, C₂₋₆ alkynyl, C₃₋₈ cycloalkyl, 3- to 6-membered heterocyclyl, or 5- to 10-membered heteroaryl may be optionally further substituted by one or more substituents selected from the group consisting of C₁₋₆ alkyl and halogen;
alternatively, R³ and R⁴, together with the carbon atom to which they are attached, form a C₃₋₈ cycloalkyl group, a 3- to 6-membered heterocyclyl group, or a 5- to 10-membered heteroaryl group;
alternatively, R³ and R², together with the carbon atom to which they are attached, form a C₃₋₈ cycloalkyl group, a 3- to 6-membered heterocyclyl group, or a 5- to 10-membered heteroaryl group;
at least one of R¹, R², R³, R⁴, or R⁵ is hydroxyl;
R⁶ is selected from the group consisting of hydrogen, halogen, and C₁₋₆ alkyl; the C₁₋₆ alkyl may be optionally further substituted by one or more substituents selected from the group consisting of C₁₋₆ alkyl and halogen;
L is a chemical bond, NR⁷, OR⁷, or C(R⁷)₂; R⁷ is selected from the group consisting of hydrogen, C₁₋₆ alkyl, and absent;
R⁸ is selected from the group consisting of hydrogen, hydroxyl, cyano, amino, C₁₋₆ alkyl, C₁₋₆ alkoxy, C₂₋₆ alkynyl, C₃₋₈ cycloalkyl, and 5- to 10-membered heteroaryl; the C₁₋₆ alkyl, C₁₋₆ alkoxy, C₂₋₆ alkynyl, C₃₋₈ cycloalkyl, or 5- to 10-membered heteroaryl may be optionally further substituted by one or more substituents selected from the group consisting of C₁₋₆ alkyl and halogen;
ring B is selected from the group consisting of C₃₋₈ cycloalkyl, 3- to 9-membered heterocyclyl, C₆₋₁₀ aryl, and 5- to 10-membered heteroaryl; the C₃₋₈ cycloalkyl, 3- to 9-membered heterocyclyl, C₆₋₁₀ aryl, or 5- to 10-membered heteroaryl may be optionally further substituted by one or more substituents selected from the group consisting of hydroxyl, halogen, C₁₋₆ alkyl, C₁₋₆ haloalkyl, C₁₋₆ hydroxyalkyl, -COOR⁹, -CH₂COOR⁹, and C₁₋₆ alkylaminoalkyl;
R⁹ is selected from the group consisting of hydrogen and C₁₋₆ alkyl;
wherein the 3- to 6-membered heterocyclyl, 3- to 9-membered heterocyclyl, or 5- to 10-membered heteroaryl contains at least one heteroatom selected from the group consisting of N, O, and S.

2. The pyrazolopyrimidine compound according to claim 1, wherein the pyrazolopyrimidine compound is a compound represented by the following general formula (IA), an isomer thereof, or a pharmaceutically acceptable salt thereof: 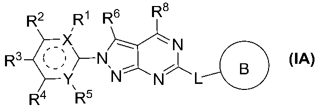
wherein X and Y are each independently selected from the group consisting of C and N;
R¹, R², R³, R⁴, and R⁵ are each independently selected from the group consisting of hydrogen, halogen, substituted or unsubstituted C₁₋₆ alkyl, substituted or unsubstituted C₁₋₆ haloalkyl, substituted or unsubstituted C₁₋₆ alkylamino, substituted or unsubstituted C₁₋₆ alkoxy, substituted or unsubstituted C₁₋₆ haloalkoxy, substituted or unsubstituted C₂₋₆ alkenyl, substituted or unsubstituted C₂₋₆ haloalkenyl, substituted or unsubstituted C₂₋₆ alkynyl, substituted or unsubstituted C₃₋₈ cycloalkyl, substituted or unsubstituted 3- to 6-membered heterocyclyl, substituted or unsubstituted 5- to 10-membered heteroaryl, hydroxyl, cyano, and amino, and at least one of R¹, R², R³, R⁴, or R⁵ is hydroxyl; when the C₁₋₆ alkyl, C₁₋₆ haloalkyl, C₁₋₆ alkylamino, C₁₋₆ alkoxy, C₁₋₆ haloalkoxy, C₂₋₆ alkenyl, C₂₋₆ haloalkenyl, C₂₋₆ alkynyl, C₃₋₈ cycloalkyl, 3- to 6-membered heterocyclyl, or 5- to 10-membered heteroaryl contains at least one substituent, the substituent is selected from the group consisting of C₁₋₆ alkyl and halogen, or any combination thereof; or
R³ and an adjacent R⁴, together with the carbon atom to which they are attached, form a C₃₋₈ cycloalkyl group, a 3- to 6-membered heterocyclyl group, or a 5- to 10-membered heteroaryl group; R³ and an adjacent R², together with the carbon atom to which they are attached, form a C₃₋₈ cycloalkyl group, a 3- to 6-membered heterocyclyl group, or a 5- to 10-membered heteroaryl group;
R⁶ is selected from the group consisting of hydrogen, substituted or unsubstituted C₁₋₆ alkyl, and halogen; when the C₁₋₆ alkyl contains at least one substituent, the substituent is selected from the group consisting of C₁₋₆ alkyl and halogen, or any combination thereof;
L is a direct bond, NR⁷, OR⁷, or CR⁷; R⁷ is selected from the group consisting of H, C₁₋₆ alkyl, and absent;
R⁸ is selected from the group consisting of hydrogen, substituted or unsubstituted C₁₋₆ alkyl, substituted or unsubstituted C₁₋₆ haloalkyl, substituted or unsubstituted C₁₋₆ alkoxy, substituted or unsubstituted C₁₋₆ haloalkoxy, substituted or unsubstituted C₂₋₆ alkynyl, substituted or unsubstituted C₃₋₈ cycloalkyl, substituted or unsubstituted 5- to 10-membered heteroaryl, hydroxyl, cyano, and amino; when the C₁₋₆ alkyl, C₁₋₆ haloalkyl, C₁₋₆ alkoxy, C₁₋₆ haloalkoxy, C₂₋₆ alkynyl, C₃₋₈ cycloalkyl, or 5- to 10-membered heteroaryl contains at least one substituent, the substituent is selected from the group consisting of C₁₋₆ alkyl and halogen, or any combination thereof;
ring B is selected from the group consisting of substituted or unsubstituted C₃₋₈ cycloalkyl, substituted or unsubstituted 3- to 9-membered heterocyclyl, substituted or unsubstituted C₆₋₁₀ aryl, and substituted or unsubstituted 5- to 10-membered heteroaryl; when the C₃₋₈ cycloalkyl, 3- to 9-membered heterocyclyl, C₆₋₁₀ aryl, or 5- to 10-membered heteroaryl contains at least one substituent, the substituent is selected from the group consisting of C₁₋₆ alkyl, hydroxyl, halogen, C₁₋₆ haloalkyl, C₁₋₆ hydroxyalkyl, -COOR⁹, and C₁₋₆ alkylaminoalkyl, or any combination thereof;
R⁹ is selected from the group consisting of hydrogen and C₁₋₆ alkyl;
the heterocyclyl or heteroaryl contains at least one heteroatom selected from the group consisting of N, O, and S.

3. The pyrazolopyrimidine compound according to claim 1, wherein the pyrazolopyrimidine compound is a compound represented by the following general formula (IB), an isomer thereof, or a pharmaceutically acceptable salt thereof: 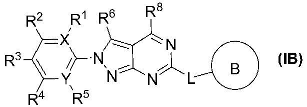
wherein ring B is selected from the group consisting of C₃₋₈ cycloalkyl, 3- to 9-membered heterocyclyl, C₆₋₁₀ aryl, and 5- to 10-membered heteroaryl; the C₃₋₈ cycloalkyl, 3- to 9-membered heterocyclyl, C₆₋₁₀ aryl, or 5- to 10-membered heteroaryl may be optionally further substituted by one or more substituents selected from the group consisting of hydroxyl, halogen, C₁₋₆ alkyl, C₁₋₆ haloalkyl, C₁₋₆ hydroxyalkyl, -CH₂COOR⁹, and C₁₋₆ alkylaminoalkyl;
X, Y, L, R¹, R², R³, R⁴, R⁵, R⁶, R⁸, and R⁹ are as defined in claim 1.

4. The pyrazolopyrimidine compound according to any one of claims 1 to 3, wherein the pyrazolopyrimidine compound is a compound represented by the following general formula (II), an isomer thereof, or a pharmaceutically acceptable salt thereof: 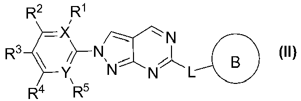
wherein X and Y are each independently selected from the group consisting of C and N;
R¹ and R⁵ are each independently selected from the group consisting of absent, hydrogen, halogen, hydroxyl, cyano, C₁₋₆ alkyl, C₁₋₆ alkylamino, C₁₋₆ alkoxy, C₂₋₆ alkynyl, C₃₋₈ cycloalkyl, 3- to 6-membered heterocyclyl, and 5- to 10-membered heteroaryl; the C₁₋₆ alkyl, C₁₋₆ alkylamino, C₁₋₆ alkoxy, C₂₋₆ alkynyl, C₃₋₈ cycloalkyl, 3- to 6-membered heterocyclyl, or 5- to 10-membered heteroaryl may be optionally further substituted by one or more substituents selected from the group consisting of C₁₋₆ alkyl and halogen;
R², R³, and R⁴ are each independently selected from the group consisting of hydrogen, halogen, hydroxyl, cyano, C₁₋₆ alkyl, C₁₋₆ alkylamino, C₁₋₆ alkoxy, C₂₋₆ alkynyl, C₃₋₈ cycloalkyl, 3- to 6-membered heterocyclyl, and 5- to 10-membered heteroaryl; the C₁₋₆ alkyl, C₁₋₆ alkylamino, C₁₋₆ alkoxy, C₂₋₆ alkynyl, C₃₋₈ cycloalkyl, 3- to 6-membered heterocyclyl, or 5- to 10-membered heteroaryl may be optionally further substituted by one or more substituents selected from the group consisting of C₁₋₆ alkyl and halogen;
alternatively, R³ and R⁴, together with the carbon atom to which they are attached, form a C₃₋₈ cycloalkyl group, a 3- to 6-membered heterocyclyl group, or a 5- to 10-membered heteroaryl group;
alternatively, R³ and R², together with the carbon atom to which they are attached, form a C₃₋₈ cycloalkyl group, a 3- to 6-membered heterocyclyl group, or a 5- to 10-membered heteroaryl group;
at least one of R¹, R², R³, R⁴, or R⁵ is hydroxyl;
L is selected from the group consisting of NR⁷, OR⁷, and C(R⁷)₂; R₇ is selected from the group consisting of hydrogen, methyl, and absent;
ring B is selected from the group consisting of C₃₋₈ cycloalkyl, 3- to 9-membered heterocyclyl, C₆₋₁₀ aryl, and 5- to 10-membered heteroaryl; the C₃₋₈ cycloalkyl, 3- to 9-membered heterocyclyl, C₆₋₁₀ aryl, or 5- to 10-membered heteroaryl may be optionally further substituted by one or more substituents selected from the group consisting of hydroxyl, halogen, C₁₋₆ alkyl, C₁₋₆ haloalkyl, C₁₋₆ hydroxyalkyl, -CH₂COOEt, and C₁₋₆ alkylaminoalkyl;
wherein the 3- to 6-membered heterocyclyl, 3- to 9-membered heterocyclyl, or 5- to 10-membered heteroaryl contains at least one heteroatom selected from the group consisting of N, O, and S.

5. The pyrazolopyrimidine compound according to any one of claims 1 to 3, wherein the pyrazolopyrimidine compound is a compound represented by the following general formula (IIA), an isomer thereof, or a pharmaceutically acceptable salt thereof: 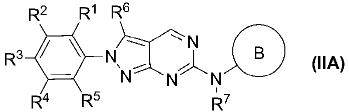
wherein R¹, R², R³, R⁴, and R⁵ are each independently selected from the group consisting of hydrogen, halogen, substituted or unsubstituted C₁₋₆ alkyl, substituted or unsubstituted C₁₋₆ haloalkyl, substituted or unsubstituted C₁₋₆ alkylamino, substituted or unsubstituted C₁₋₆ alkoxy, substituted or unsubstituted C₁₋₆ haloalkoxy, substituted or unsubstituted C₂₋₆ alkynyl, substituted or unsubstituted C₃₋₈ cycloalkyl, substituted or unsubstituted 3- to 6-membered heterocyclyl, substituted or unsubstituted 5- to 10-membered heteroaryl, hydroxyl, and cyano, and at least one of R¹, R², R³, R⁴, or R⁵ is hydroxyl; when the C₁₋₆ alkyl, C₁₋₆ haloalkyl, C₁₋₆ alkoxy, C₁₋₆ haloalkoxy, C₂₋₆ alkynyl, C₃₋₈ cycloalkyl, 3- to 6-membered heterocyclyl, or 5- to 10-membered heteroaryl contains at least one substituent, the substituent is selected from the group consisting of C₁₋₆ alkyl and halogen, or any combination thereof; or
R³ and an adjacent R⁴, together with the carbon atom to which they are attached, form a C₃₋₈ cycloalkyl group, a 3- to 6-membered heterocyclyl group, or a 5- to 10-membered heteroaryl group; R³ and an adjacent R², together with the carbon atom to which they are attached, form a C₃₋₈ cycloalkyl group, a 3- to 6-membered heterocyclyl group, or a 5- to 10-membered heteroaryl group;
R⁶ is selected from the group consisting of hydrogen, C₁₋₆ alkyl, and halogen;
R⁷ is selected from the group consisting of hydrogen and C₁₋₆ alkyl;
ring B is selected from the group consisting of substituted or unsubstituted C₃₋₈ cycloalkyl, substituted or unsubstituted 3- to 8-membered heterocyclyl, and substituted or unsubstituted 5-to 10-membered heteroaryl; when the C₃₋₈ cycloalkyl, 3- to 8-membered heterocyclyl, or 5- to 10-membered heteroaryl contains at least one substituent, the substituent is selected from the group consisting of C₁₋₆ alkyl, hydroxyl, halogen, C₁₋₆ haloalkyl, C₁₋₆ hydroxyalkyl, - CH₂COOEt, and C₁₋₆ alkylaminoalkyl, or any combination thereof;
the heterocyclyl or heteroaryl contains at least one heteroatom selected from the group consisting of N, O, and S.

6. The pyrazolopyrimidine compound according to any one of claims 1 to 4, wherein the pyrazolopyrimidine compound is a compound represented by the following general formula (II-1), an isomer thereof, or a pharmaceutically acceptable salt thereof: 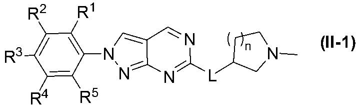
wherein n is selected from the group consisting of 0, 1, 2, and 3;
R¹, R², R³, R⁴, and R⁵ are each independently selected from the group consisting of hydrogen, halogen, hydroxyl, cyano, C₁₋₆ alkyl, C₁₋₆ alkylamino, C₁₋₆ alkoxy, C₂₋₆ alkynyl, C₃₋₈ cycloalkyl, 3- to 6-membered heterocyclyl, and 5- to 10-membered heteroaryl; the C₁₋₆ alkyl, C₁₋₆ alkylamino, C₁₋₆ alkoxy, C₂₋₆ alkynyl, C₃₋₈ cycloalkyl, 3- to 6-membered heterocyclyl, or 5- to 10-membered heteroaryl may be optionally further substituted by one or more substituents selected from the group consisting of C₁₋₆ alkyl and halogen;
alternatively, R³ and R⁴, together with the carbon atom to which they are attached, form a C₃₋₈ cycloalkyl group, a 3- to 6-membered heterocyclyl group, or a 5- to 10-membered heteroaryl group;
alternatively, R³ and R², together with the carbon atom to which they are attached, form a C₃₋₈ cycloalkyl group, a 3- to 6-membered heterocyclyl group, or a 5- to 10-membered heteroaryl group;
at least one of R¹, R², R³, R⁴, or R⁵ is hydroxyl;
L is as defined in claim 4.

7. The pyrazolopyrimidine compound according to any one of claims 1 to 4 and 6, wherein the pyrazolopyrimidine compound is a compound represented by the following general formula **(IIA-1),** an isomer thereof, or a pharmaceutically acceptable salt thereof: 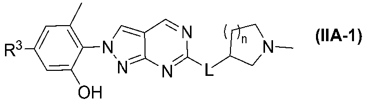
wherein n is selected from the group consisting of 1 and 2;
L is selected from the group consisting of O, NH, and N(CH₃);
R³ is selected from the group consisting of hydrogen, halogen, hydroxyl, cyano, C₁₋₆ alkyl, C₁₋₆ alkylamino, C₁₋₆ alkoxy, C₂₋₆ alkynyl, C₃₋₈ cycloalkyl, 3- to 6-membered heterocyclyl, and 5- to 10-membered heteroaryl; the C₁₋₆ alkyl, C₁₋₆ alkylamino, C₁₋₆ alkoxy, C₂₋₆ alkynyl, C₃₋₈ cycloalkyl, 3- to 6-membered heterocyclyl, or 5- to 10-membered heteroaryl may be optionally further substituted by one or more substituents selected from the group consisting of halogen and C₁₋₆ alkyl.

8. The pyrazolopyrimidine compound according to any one of claims 1 to 7, wherein the pyrazolopyrimidine compound is a compound represented by the following general formula **(IIA-1-1),** an isomer thereof, or a pharmaceutically acceptable salt thereof: 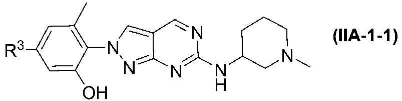 wherein R³ is as defined in claim 7.

9. The pyrazolopyrimidine compound according to claim 8, wherein R³ is selected from the group consisting of hydrogen, C₁₋₆ haloalkyl, and halogen.

10. The pyrazolopyrimidine compound according to any one of claims 1 to 8, wherein the pyrazolopyrimidine compound is a compound represented by the following general formula (IIA-1-2), an isomer thereof, or a pharmaceutically acceptable salt thereof: 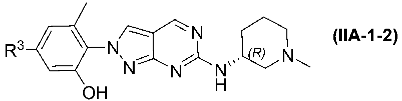 wherein R³ is as defined in claim 7.

11. The pyrazolopyrimidine compound according to any one of claims 1 to 8 and 10, wherein R³ is selected from the group consisting of hydrogen, halogen, cyano, methyl, cyclopropyl, trifluoromethyl, difluoromethyl, trifluoromethoxy, dimethylamino,

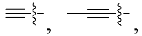

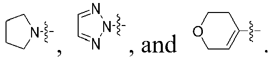

12. A pyrazolopyrimidine compound, which is a compound having the following structure, an isomer thereof, or a pharmaceutically acceptable salt thereof:

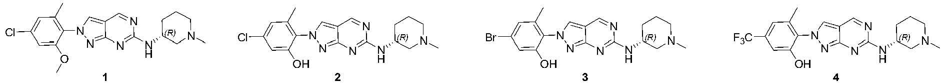

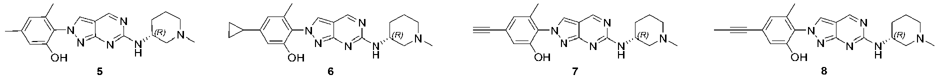

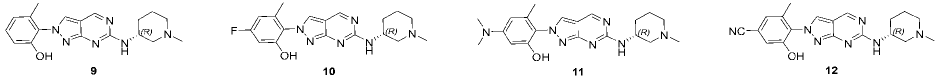

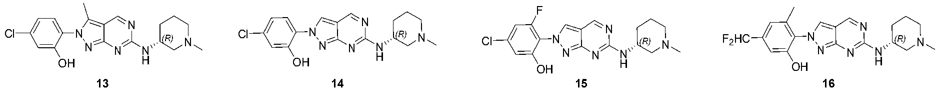

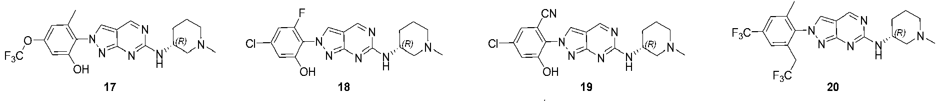

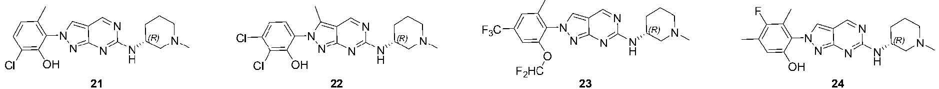

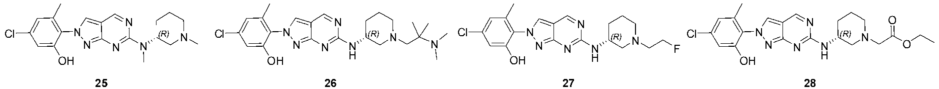

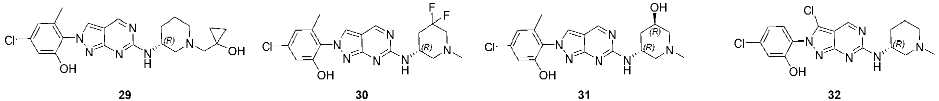

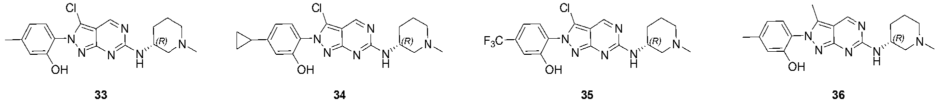

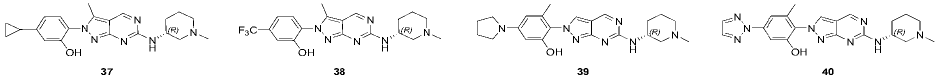

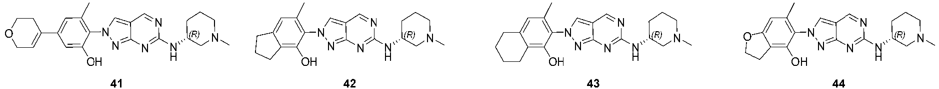

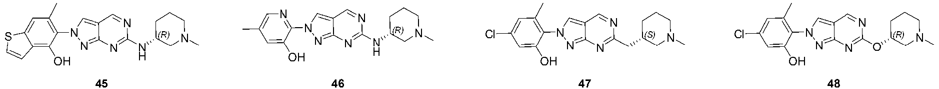

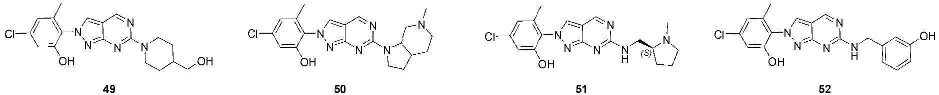

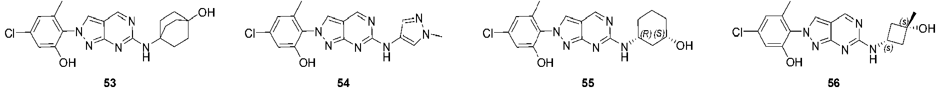

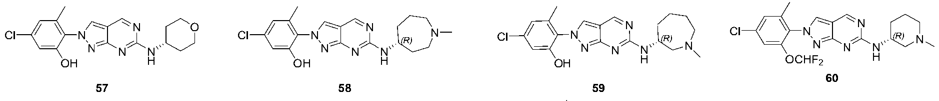

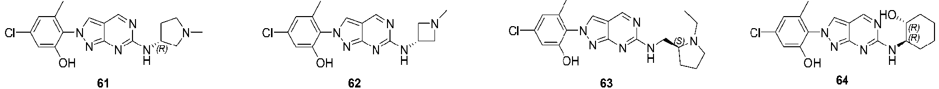

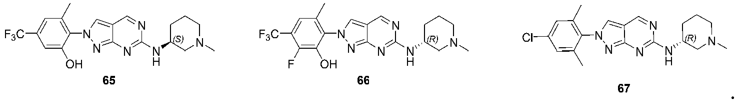

13. A pharmaceutical composition, comprising the compound as defined in any one of claims 1 to 12, an isomer thereof, or a pharmaceutically acceptable salt thereof, and at least one pharmaceutically acceptable carrier or excipient.

14. Use of the compound as defined in any one of claims 1 to 12, an isomer thereof, or a pharmaceutically acceptable salt thereof, or the pharmaceutical composition as defined in claim 13 in the manufacture of a medicament for preventing or treating a disease associated with NLRP3.

15. Use of the compound as defined in any one of claims 1 to 12, an isomer thereof, or a pharmaceutically acceptable salt thereof, or the pharmaceutical composition as defined in claim 13 in preventing or treating a disease associated with NLRP3.

16. A method of treating and/or preventing a disease, comprising administering to a subject in need thereof a therapeutically effective amount of the compound as defined in any one of claims 1 to 12, an isomer thereof, or a pharmaceutically acceptable salt thereof, or the pharmaceutical composition as defined in claim 13; wherein the disease to be treated and/or prevented is a disease or disorder associated with NLRP3.

17. The use according to claim 14 or 15, or the method according to claim 16, wherein the disease associated with NLRP3 is cancer, an inflammatory disease, or a disease accompanied by an inflammatory response;
the cancer comprises, but is not limited to, myeloproliferative neoplasm, myeloid leukemia, lung cancer, nasopharyngeal carcinoma, laryngeal cancer, esophageal cancer, cholangiocarcinoma, oral cancer, head and neck cancer, mesothelioma, adrenocortical carcinoma, kidney cancer, liver cancer, gastric cancer, colon cancer, rectal cancer, bone cancer, brain cancer, breast cancer, melanoma, pancreatic cancer, skin cancer, lymphoma, bladder cancer, small intestine cancer, soft tissue sarcoma, endometrial cancer, cervical cancer, osteosarcoma, prostate cancer, and testicular cancer;
the inflammatory disease or disease accompanied by an inflammatory response comprises, but is not limited to:
1) auto inflammatory diseases, such as cryopyrin-associated periodic syndrome (CAPS), familial Mediterranean fever, Schnitzler syndrome, and mevalonate kinase deficiency (MKD);
2) chronic pain, comprising neuropathic pain and non-neuropathic pain;
3) dermatological disorders, such as contact hypersensitivity, bullous pemphigoid, sunburn, contact dermatitis, seborrheic dermatitis, hidradenitis suppurativa (HS), diabetic (foot) ulcer, lichen planus, scleroderma, pemphigus, epidermolysis bullosa, urticaria, acne, and alopecia;
4) respiratory disorders, such as chronic obstructive pulmonary disease (COPD), asthma, bronchitis, rhinitis, sinusitis, idiopathic pulmonary fibrosis (IPF), cystic fibrosis, sarcoidosis, adult respiratory distress syndrome, and pneumonia;
5) joint disorders, such as arthritis;
6) muscular disorders, such as polymyositis and myasthenia gravis;
7) cardiovascular disorders, such as hypertension, ischemia, reperfusion injury, vasculitis, and pericarditis;
8) hematological disorders, such as sickle cell disease;
9) central nervous system disorders, such as Parkinson's disease, Alzheimer's disease, Huntington's disease, brain injury, multiple sclerosis, and amyotrophic lateral sclerosis;
10) metabolic diseases, such as type 2 diabetes (T2D), atherosclerosis, obesity, and gout;
11) liver diseases, such as non-alcoholic fatty liver disease (NAFLD), non-alcoholic steatohepatitis (NASH), alcoholic fatty liver disease (AFLD), and alcoholic steatohepatitis (ASH);
12) kidney diseases, such as acute kidney disease, hyperoxaluria, chronic kidney disease, nephrocalcinosis, glomerulonephritis, and diabetic nephropathy;
13) gastrointestinal disorders, such as inflammatory bowel disease and pancreatitis;
14) ocular disorders, such as uveitis and allergic conjunctivitis;
15) graft-versus-host disease;
16) burns, sunburn, and mechanical injury.

18. The use or method according to claim 17, wherein the neuropathic pain comprises central neuropathic pain and peripheral neuropathic pain;
the central neuropathic pain comprises, but is not limited to, pain associated with spinal cord injury, post-stroke pain, pain associated with multiple sclerosis, pain associated with syringomyelia, pain associated with ischemic myelopathy, pain associated with compressive myelopathy, pain associated with radiation-induced myelopathy, pain associated with Parkinson's disease, phantom limb pain, and pain associated with myelitis;
the peripheral neuropathic pain comprises, but is not limited to, postherpetic neuralgia, HIV-associated neuropathy, diabetic peripheral neuropathy, chronic post-traumatic or post-surgical pain, chemotherapy- or radiotherapy-induced neuropathy, trigeminal neuralgia, glossopharyngeal neuralgia, residual limb pain, toxin-induced neuropathy, sciatica, and radicular pain;
the non-neuropathic pain comprises, but is not limited to, osteoarthritis pain, chronic low back pain, chronic visceral pain, cancer pain, and fibromyalgia.
